# FASCICULE DE BREVET EUROPEEN

(11) **EP 2 691 377 B1**
(45) Date de publication et mention de la délivrance du brevet: **07.03.2018**
(21) Numéro de dépôt: 12713125.8
(22) Date de dépôt: 30.03.2012
(51) Int. Cl.: C07D 241/42, A61K 31/498

(54) **DÉRIVÉS AMINO-QUINOXALINES POUR LE TRAITEMENT DE MALADIES NEURODÉGÉNÉRATIVES**
AMINOCHINOXALINDERIVATE ZUR BEHANDLUNG VON NEURODEGENERATIVEN ERKRANKUNGEN
AMINOQUINOXALINE DERIVATIVES FOR TREATMENT OF NEURODEGENERATIVE DISEASES

(30) Priorité: 30.03.2011 FR 1152645
(43) Date de publication de la demande: 05.02.2014
(73) Titulaire: Centre National de la Recherche Scientifique (C.N.R.S.), 75016 Paris (FR); Université Paris-Sud 11, 91400 Orsay (FR)
(72) Inventeur: FERRIE, Laurent, 91120 Palaiseau (FR); FIGADERE, Bruno, 91530 Saint Cheron (FR); LE DOUARON, Gaël, 91370 Verriere Le Buisson (FR); RAISMAN-VOZARI, Rita, 75013 Paris (FR); SCHMIDT, Fanny, 92290 Chatenay Malabry (FR)
(74) Mandataire: Lavoix
(86) Numéro de dépôt international: PCT/EP2012/055903
(87) Numéro de publication internationale: WO 2012/131080

(56) Documents cités:
- EP-A1- 1 479 384
- WO-A1-2010/007179
- WO-A1-2011/044229
- WO-A2-2004/030635
- WO-A2-2010/151799
- US-A- 5 480 883
- US-A1- 2004 102 360
- DATABASE CHEMABS [Online] CHEMICAL ABSTRACTS SERVICE, COLUMBUS, OHIO, US; 1988, Poradowska, Henryka: "The nitration of aminoquinoxalines", XP002658456, extrait de STN Database accession no. 1988:492044
- RAYMONDE NASIELSKI-HINKENS ET AL: "Fragmentation of 1,4,5,8-tetraazaphenanthrenes upon electron impact: A new example of structural integrity of gas phase heteroaromatic cations", ORGANIC MASS SPECTROMETRY, vol. 20, no. 12, 1 décembre 1985 (1985-12-01), pages 733-737, XP055006452, ISSN: 0030-493X, DOI: 10.1002/oms.1210201207
- KASAI HIROSHI ET AL: "Synthesis of 2-amino-3, 8-dimethylimidazo (4,5-f)quinoxaline (Me-IQx), a potent mutagen isolated from fried beef", CHEMISTRY LETTERS, CHEMICAL SOCIETY OF JAPAN, JP, no. 5, 1 janvier 1981 (1981-01-01), pages 675-678, XP002955028, ISSN: 0366-7022, DOI: 10.1246/CL.1981.675
- NASIELSKI, GARCIA Y ABELEDO, NASIELSKI-HINKENS: "A liposoluble cationic complex of Ru(II) based on 1,4,5,8-tetra-azaphenanthrene", BULLETIN DES SOCIÉTÉS CHIMIQUES BELGES, vol. 97, no. 10, 1988, pages 743-750, XP002658457, DOI: 10.1002/bscb.19880971003
- ZHANG ET AL: "Quinoxalinylurea derivatives as a novel class of JSP-1 inhibitors", BIOORGANIC & MEDICINAL CHEMISTRY LETTERS, PERGAMON, ELSEVIER SCIENCE, GB, vol. 17, no. 8, 30 mars 2007 (2007-03-30), pages 2118-2122, XP022009217, ISSN: 0960-894X, DOI: 10.1016/J.BMCL.2007.01.094
- NASIELSKI J ET AL: "1,4,5,8-Tetraazaphenanthrene complexes of copper(I) and silver(I)", BULLETIN DES SOCIETES CHIMIQUES BELGES, LOUVAIN [U.A.] : CENTERICK, BE, vol. 97, no. 11-12, 1 janvier 1988 (1988-01-01), pages 983-992, XP008151516, ISSN: 0037-9646, DOI: 10.1002/BSCB.19880971124 [extrait le 2010-09-01]
- ELMES ROBERT B P ET AL: "Quaternarized pdppz: synthesis, DNA-binding and biological studies of a novel dppz derivative that causes cellular death upon light irradiation", CHEMICAL COMMUNICATIONS - CHEMCOM; [6015D], ROYAL SOCIETY OF CHEMISTRY, GB, vol. 47, no. 2, 1 janvier 2011 (2011-01-01), pages 686-688, XP008151514, ISSN: 1359-7345, DOI: 10.1039/C0CC04303F
- JOHN W. BARTON, MICHAEL C. GOODLAND, KEN J. GOULD, JOHN F.W. MCOMIE, W.RODERICK MOUND, SADIQ A. SALEH: "Biphenylenes-xxxi: Condensation of benzocyclobutene-1,2-dione with aliphatic and heterocyclic 1,2-diamines and the synthesis of cis-2-cyano-3-(2'-cyanovinyl)-1,4-diazabip henylene", TETRAHEDRON, vol. 35, 1979, pages 241-247, XP002675533,

## Description

La présente invention concerne des dérivés d'amino-quinoxalinepour leur utilisation en tant que médicament destiné au traitement ou à la prévention de la maladie de Parkinson.

Avec l'allongement de l'espérance de vie, de plus en plus de personnes sont atteintes de maladies neurodégénératives telles que la maladie d'Alzheimer ou la maladie de Parkinson.

Une maladie neurodégénérative est une maladie qui affecte le fonctionnement du système nerveux, et en particulier du cerveau, de façon progressive, la maladie pouvant évoluer plus ou moins rapidement (quelques semaines à plusieurs années), et souvent de façon irréversible. Ainsi, le fonctionnement des cellules nerveuses, en particulier les neurones, se trouve détérioré, ce qui peut conduire à leur mort cellulaire. Selon la région du système nerveux atteint par la maladie, différentes fonctions pourront être affectées comme la motricité, le langage, la mémoire, la perception ou encore la cognition. Parmi les maladies neurodégénératives les plus fréquentes, on peut citer en particulier la maladie d'Alzheimer et la maladie de Parkinson.

La maladie d'Alzheimer, qui touche environ 24 millions de personnes dans le monde entier, est une maladie du tissu cérébral qui entraîne la perte progressive et irréversible des fonctions mentales. Le premier symptôme est la perte du souvenir des événements récents (amnésie), puis les déficits cognitifs s'étendent aux domaines du langage (aphasie), de l'organisation des mouvements (apraxie), de la reconnaissance visuelle (agnosie) et des fonctions exécutives (telles que la prise de décision et la planification).

La maladie de Parkinson, quant à elle, affecte le système nerveux central et provoque des troubles moteurs d'évolution progressive, notamment des tremblements du corps.

Actuellement, les médicaments prescrits pour ces deux maladies permettent uniquement de retarder l'évolution de la maladie. Aucun ne permet de guérir la maladie, ni même d'arrêter son évolution, d'où le besoin de trouver de nouvelles molécules plus actives pour le traitement de ces maladies neurodégénératives.

Les inventeurs de la présente invention ont déjà mis en évidence le potentiel de composés chimères possédant un noyau quinoxaline substitué par une chaîne hydrophobe aliphatique dans le traitement de maladies neurodégénératives (WO 2010/007179).

Toutefois, les inventeurs ont découvert de manière surprenante que les dérivés de quinoxaline conservaient leur activité et étaient toujours capables de passer la barrière hémato-encéphalique en absence de substitution avec une chaîne hydrophobe qui semblait pourtant essentielle au passage de la barrière hémato-encéphalique.

De tels composés dérivés d'amino-quinoxaline sont décrits notamment dans WO 2011/044229; WO 2010/151799; WO 2004/030635; EP 1 479 384; US 2004/0102360 ; US 5 480 883 ; Barton et al. Tetrahedron 1979, 35, 241-247 ; Kasai et al. Chemistry Letters 1981, 675-678 ; Nasielski-Hinkens et Kaisin Organic Mass Spectrometry 1985, 20(12), 733-737 ; Nasielski et al. Bull. Soc. Chim. Belg. 1988, 97(10), 743-750; Nasielski et al. Bull. Soc. Chim. Belg. 1988, 97, 983-992; Poradowska H. Zeszyty Naukowe Uniwersytetu Jagiellonskiego, Prace Chemiczne 1987, 30, 97-115 ; Zhang et al. Bioorg. Med. Chem. Lett. 2007, 17, 2118-2122 ; et Elmes et al. Chem. Commun. 2011, 47, 686-688, sans qu'une utilisation dans le traitement ou à la prévention de la maladie de Parkinson soit mentionnée ou suggérée.

La présente invention a donc pour objet un composé de formule (I) suivante : 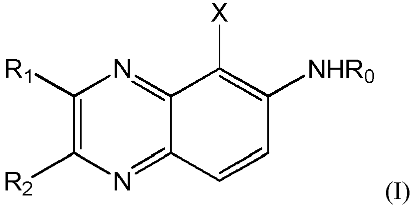 dans laquelle :
- X représente un atome d'hydrogène, un atome d'halogène tel qu'un brome ou un chlore, un groupe NO₂ ou NH₂,
- R₀ représente H ou -CH₂-C≡CH, et notamment H, et
- R₁ et R₂ représentent chacun, indépendamment l'un de l'autre, un atome d'hydrogène ; une chaîne hydrocarbonée saturée ou insaturée, linéaire ou ramifiée, comportant de 1 à 10, de préférence 1 à 6 atomes de carbone ; ou un groupe aryle éventuellement substitué,
ainsi que ses sels pharmaceutiquement acceptables, ses stéréoisomères ou mélanges de stéréoisomères en toutes proportions, en particulier un mélange d'énantiomères, et notamment un mélange racémique,
pour son utilisation en tant que médicament destiné au traitement ou à la prévention de la maladie de Parkinson.

Par « halogène », on entend, au sens de la présente invention, un atome de brome, chlore, iode ou fluor, et notamment un atome de brome.

Par groupement « (C₁-C₆)-alkyle », on entend, au sens de la présente invention, une chaîne hydrocarbonée saturée, linéaire ou ramifiée, comportant de 1 à 6 atomes de carbone, en particulier les groupes méthyle, éthyle, n-propyle, isopropyle, n-butyle, iso-butyle, sec-butyle, tert-butyle, n-pentyle, n-hexyle.

Par groupement « (C₁-C₆)-alcoxy », on entend, au sens de la présente invention, un groupe (C₁-C₆)-alkyle tel que défini ci-dessus lié au reste de la molécule par l'intermédiaire d'un atome d'oxygène et en particulier les groupes méthoxy, éthoxy, n-propoxy, isopropoxy, n-butoxy, iso-butoxy, sec-butoxy, tert-butoxy, n-pentoxy, n-hexoxy.

Par groupement « aryle », on entend, au sens de la présente invention, un groupement hydrocarboné aromatique, comportant de préférence de 6 à 10 atomes de carbone et comprenant un ou plusieurs cycles accolés, comme par exemple un groupement phényle ou naphtyle. Avantageusement, il s'agit du phényle.

Lorsque le groupement aryle est substitué, il pourra avantageusement être substitué par un ou plusieurs groupes choisis parmi un atome d'halogène, un groupe (C₁-C₆)alkyle, (C₁-C₆)alcoxy, aryle, N₃, NO₂, NH₂, ou -NH-((C₁-C₆)alkyle) ; de préférence choisis parmi un atome d'halogène, un groupe (C₁-C₆)alkyle, (C₁-C₆)alcoxy ou aryle.

Par « insaturée », on entend, au sens de la présente invention, que la chaîne hydrocarbonée peut comportée une ou plusieurs insaturation(s).

Par « insaturation », on entend, au sens de la présente invention, une double ou une triple liaison.

Dans la présente invention, on entend par « pharmaceutiquement acceptable » ce qui est utile dans la préparation d'une composition pharmaceutique qui est généralement sûre, non toxique et ni biologiquement ni autrement non souhaitable et qui est acceptable pour une utilisation vétérinaire de même que pharmaceutique humaine.

Par « groupe N-protecteur », on entend, au sens de la présente invention, tout substituant qui protège le groupe NH₂ contre les réactions indésirables tel que les groupes N-protecteur décrits dans Greene « Protective Groups In Organic synthesis » (John Wiley & Sons, New York (1981)) et Harrison et al. « Compendium of Synthetic Organic Methods » Vols. 1 à 8 (J. Wiley & sons, 1971 à 1996). Les groupes N-protecteurs comprennent, fonction amine protégée incluse, les carbamates, les amides, les dérivés N-alkylés, les dérivés amino acétale, les dérivés N-benzylés, les dérivés imine, les dérivés énamine, les dérivés mono- ou di-alkylaminopropargylamines et les dérivés N-hétéroatome. En particulier, le groupe N-protecteur comprend le formyle, l'acétyle, le benzoyle, le pivaloyle, le phénylsulfonyle, le benzyle (Bn), le t-butyloxycarbonyle (Boc), le benzyloxycarbonyle (Cbz), le p-méthoxybenzyloxycarbonyle, le p-nitrobenzyl-oxycarbonyle, le trichloroéthoxycarbonyle (TROC), l'allyloxycarbonyle (Alloc), le 9-fluorénylméthyloxycarbonyle (Fmoc), le trifluoro-acétyle, le diméthylaminopropargyle, les carbamates de benzyle (substitués ou non) et similaires.

Par « sels pharmaceutiquement acceptables » d'un composé, on entend désigner dans la présente invention des sels qui sont pharmaceutiquement acceptables, comme défini ici, et qui possèdent l'activité pharmacologique souhaitée du composé parent. De tels sels comprennent :
(1) les hydrates et les solvates,
(2) les sels d'addition d'acide formés avec des acides inorganiques tels que l'acide chlorhydrique, l'acide bromhydrique, l'acide sulfurique, l'acide nitrique, l'acide phosphorique et similaires ; ou formés avec des acides organiques tels que l'acide acétique, l'acide benzènesulfonique, l'acide benzoïque, l'acide camphresulfonique, l'acide citrique, l'acide éthane-sulfonique, l'acide fumarique, l'acide glucoheptonique, l'acide gluconique, l'acide glutamique, l'acide glycolique, l'acide hydroxynaphtoïque, l'acide 2-hydroxyéthanesulfonique, l'acide lactique, l'acide maléique, l'acide malique, l'acide mandélique, l'acide méthanesulfonique, l'acide muconique, l'acide 2-naphtalènesulfonique, l'acide propionique, l'acide salicylique, l'acide succinique, l'acide dibenzoyl-L-tartrique, l'acide tartrique, l'acide p-toluènesulfonique, l'acide triméthylacétique, l'acide trifluoroacétique et similaires ; et
(3) les sels formés lorsqu'un proton acide présent dans le composé parent soit est remplacé par un ion métallique, par exemple un ion de métal alcalin (Na⁺, K⁺ ou Li⁺ par exemple), un ion de métal alcalino-terreux (comme Ca²⁺ ou Mg²⁺) ou un ion d'aluminium ; soit se coordonne avec une base organique ou inorganique. Les bases organiques acceptables comprennent la diéthanolamine, l'éthanolamine, N-méthylglucamine, la triéthanolamine, la trométhamine et similaires. Les bases inorganiques acceptables comprennent l'hydroxyde d'aluminium, l'hydroxyde de calcium, l'hydroxyde de potassium, le carbonate de sodium et l'hydroxyde de sodium.

Par « stéréoisomère », on entend, au sens de la présente invention, un isomère géométrique ou un isomère optique.

Les isomères géométriques résultent de la position différente des substituants sur une double liaison qui peut avoir alors une configuration Z ou E.

Les isomères optiques résultent notamment de la position différente dans l'espace des substituants sur un atome de carbone comprenant 4 substituants différents. Cet atome de carbone constitue alors un centre chiral ou asymétrique. Les isomères optiques comprennent les diastéréoisomères et les énantiomères. Les isomères optiques qui sont des images l'un de l'autre dans un miroir mais non superposables sont désignés par « énantiomères ». Les isomères optiques qui ne sont pas des images superposables l'un de l'autre dans un miroir sont désignés par « diastéréoisomères ».

Un mélange contenant des quantités égales de deux formes énantiomères individuelles de chiralité opposée est désigné par « mélange racémique ».

Avantageusement, X représentera un atome d'hydrogène, un atome de brome, un groupe NH₂ ou NO₂. Plus particulièrement, X pourra représenter un atome d'hydrogène.

Selon un autre mode de réalisation, X représentera un atome d'halogène ou un groupe NH₂ ou NO₂ ; notamment un atome de brome ou un groupe NH₂ ou NO₂.

Le terme « chaîne hydrocarbonée saturée ou insaturée, linéaire ou ramifiée, comportant de 1 à 10, de préférence 1 à 6 atomes de carbone » représentera plus particulièrement une chaîne hydrocarbonée saturée, linéaire ou ramifiée, comportant de 1 à 10, de préférence 1 à 6 atomes de carbone dans la définitinon des groupes R₁ et R₂, et plus particulièrement un groupe (C₁-C₆)alkyle tel que défini ci-dessus.

Avantageusement, R₁ et R₂ représentent chacun, indépendamment l'un de l'autre, un atome d'hydrogène ; un groupe (C₁-C₆)alkyle ; ou un groupe aryle éventuellement substitué, notamment par un ou plusieurs groupes choisis parmi un atome d'halogène, un groupe (C₁-C₆)alkyle, (C₁-C₆)alcoxy, aryle, N₃, NO₂, NH₂, ou -NH-((C₁-C₆)alkyle), de préférence choisis parmi un atome d'halogène, un groupe (C₁-C₆)alkyle, (C₁-C₆)alcoxy ou aryle.

En particulier, R₁ et R₂ pourront représenter chacun, indépendamment l'un de l'autre, un atome d'hydrogène ; un groupe (C₁-C₆)alkyle ; ou un groupe phényle éventuellement substitué, notamment par un ou plusieurs groupes choisis parmi un atome d'halogène, un groupe (C₁-C₆)alkyle, (C₁-C₆)alcoxy, aryle, N₃, NO₂, NH₂, ou -NH-((C₁-C₆)alkyle), de préférence choisis parmi un atome d'halogène, un groupe (C₁-C₆)alkyle, (C₁-C₆)alcoxy ou aryle.

Selon un mode de réalisation particulier, R₁ représentera un groupe aryle, et notamment phényle, éventuellement substitué, notamment par un ou plusieurs groupes choisis parmi un atome d'halogène, un groupe (C₁-C₆)alkyle, (C₁-C₆)alcoxy, aryle, N₃, NO₂, NH₂, ou -NH-((C₁-C₆)alkyle) ; de préférence choisis parmi un atome d'halogène, un groupe (C₁-C₆)alkyle, (C₁-C₆)alcoxy ou aryle.

Selon un autre mode de réalisation particulier de l'invention, R₂ représentera une chaîne hydrocarbonée saturée ou insaturée, linéaire ou ramifiée, comportant de 1 à 10, de préférence 1 à 6 atomes de carbone ; de préférence un groupe (C₁-C₆)alkyle.

Selon encore un autre mode de réalisation particulier de l'invention :
- R₁ représentera une chaîne hydrocarbonée saturée ou insaturée, linéaire ou ramifiée, comportant de 1 à 10, de préférence 1 à 6 atomes de carbone, telle qu'un groupe (C₁-C₆)alkyle ; ou un groupe aryle éventuellement substitué, notamment par un ou plusieurs groupes choisis parmi un atome d'halogène, un groupe (C₁-C₆)alkyle, (C₁-C₆)alcoxy, aryle, N₃, NO₂, NH₂, ou -NH-((C₁-C₆)alkyle), et
- R₂ représentera une chaîne hydrocarbonée saturée ou insaturée, linéaire ou ramifiée, comportant de 1 à 10, de préférence 1 à 6 atomes de carbone, telle qu'un groupe (C₁-C₆)alkyle.

En particulier, les radicaux R₁ et R₂ pourront être définis comme suit :
- R₁ représentera un groupe (C₁-C₆)alkyle ou un groupe aryle éventuellement substitué, notamment par un ou plusieurs groupes choisis parmi un atome d'halogène, un groupe (C₁-C₆)alkyle, (C₁-C₆)alcoxy, aryle, N₃, NO₂, NH₂, ou -NH-((C₁-C₆)alkyle) ; avantageusement un groupe aryle éventuellement substitué, notamment par un ou plusieurs groupes choisis parmi un atome d'halogène, un groupe (C₁-C₆)alkyle, (C₁-C₆)alcoxy, aryle, N₃, NO₂, NH₂, ou -NH-((C₁-C₆)alkyle), et
- R₂ représentera un groupe (C₁-C₆)alkyle.

Dans les définitions de R₁ et R₂ ci-dessus, le groupe (C₁-C₆)alkyle pourra être plus particulièrement un groupe méthyle, *n*-butyle, *n*-hexyle, *s*-butyle ou *t*-butyle ; et le groupe aryle éventuellement substitué pourra être plus particulièrement un groupe phényle *p*-méthoxyphényle, *m*-méthoxyphényle, 3,4-diméthoxyphényle, 3,4,5-triméthoxyphényle, 2-naphthyle, *p*-fluorophényle, *p*-méthylphényle, *p*-chlorophényle, 3,4-dichlorophényle, ou biphényle.

Les composé de formule (I) pourront notamment être choisis parmi les composés suivants :

| | | | |
|---|---|---|---|
| **4ba** | 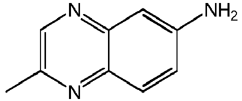 | **4bd** | 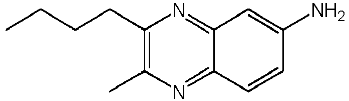 |
| **4be** | 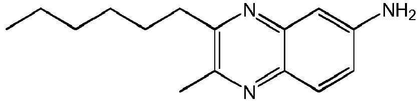 | **4bf** | 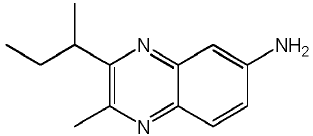 |
| **4bg** | 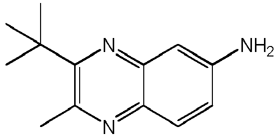 | **4bc** | 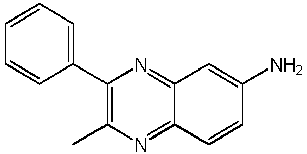 |
| **4bb** | 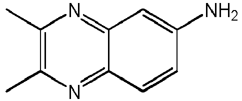 | **4aa** | 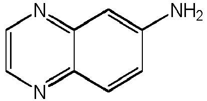 |
| **4ad** | 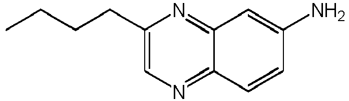 | **4dd** | 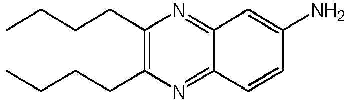 |
| **4ae** | 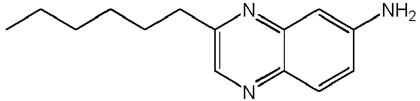 | **4af** | 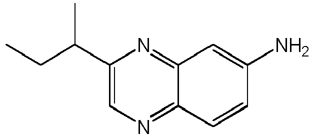 |
| **4ag** | 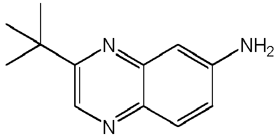 | **4ac** | 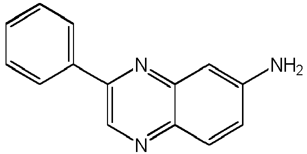 |
| **4ed** | 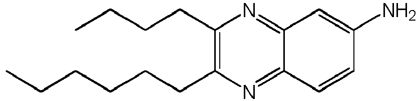 | **4de** | 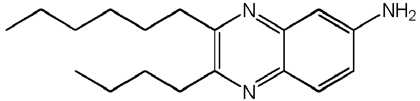 |
| **4cd** | 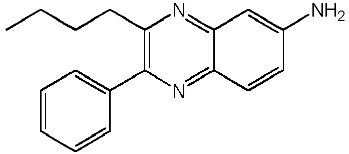 | **4bh** | 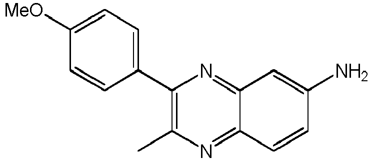 |
| **4bi** | 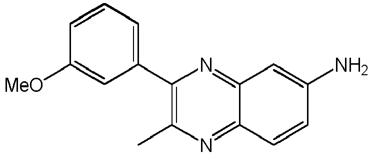 | **4bj** | 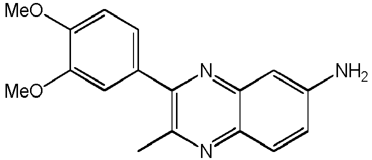 |
| **4bk** | 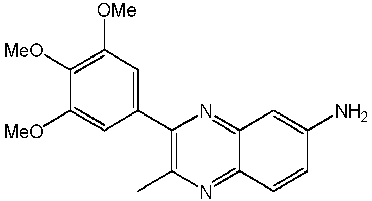 | **4bl** | 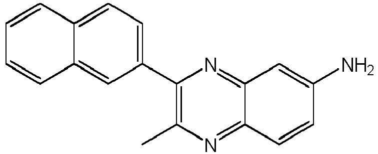 |
| **4bm** | 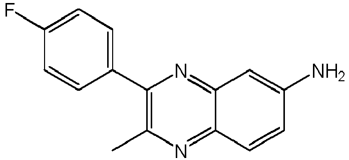 | **4bo** | 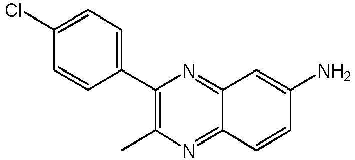 |
| **4bp** | 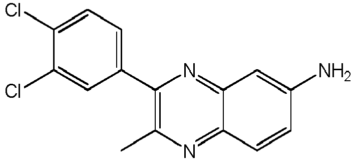 | **4bq** | 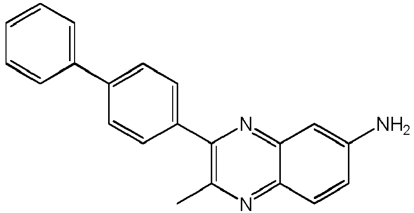 |
| **4bn** | 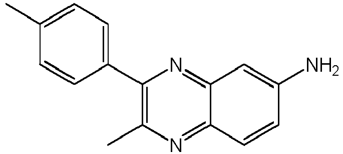 | **4ca** | 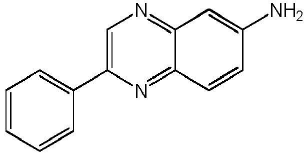 |
| **4ch** | 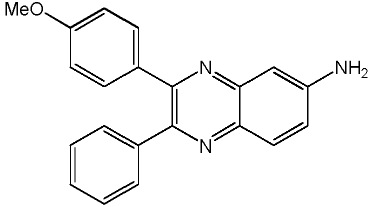 | **4cb** | 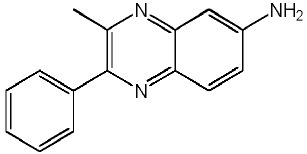 |
| **5aa** | 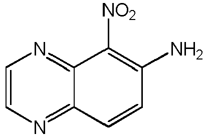 | **5b b** | 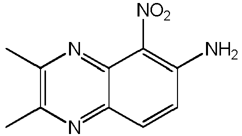 |
| **5ba** | 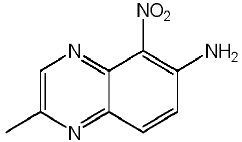 | **5bc** | 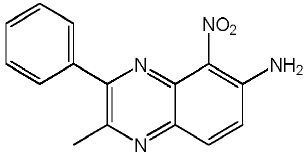 |
| **6aa** | 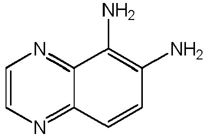 | **6b b** | 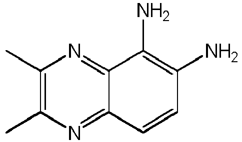 |
| **6ba** | 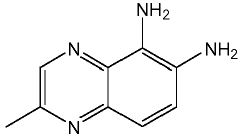 | **6bc** | 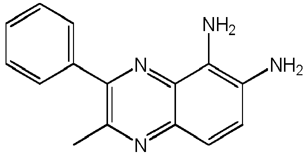 |
| **7aa** | 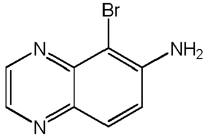 | **7bc** | 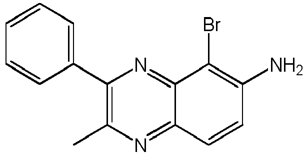 |
| **8bc** | 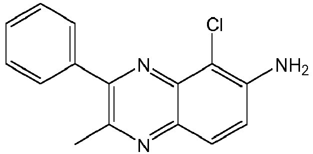 | **9bc** | 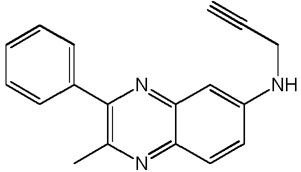 |
| **9bh** | 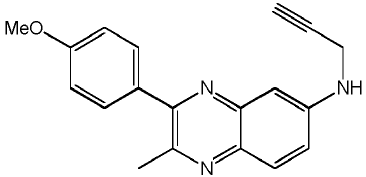 | **9bj** | 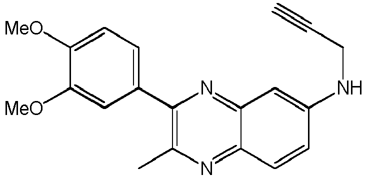 |
| **9bk** | 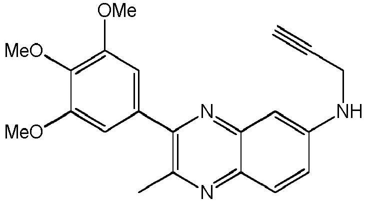 | **9bl** | 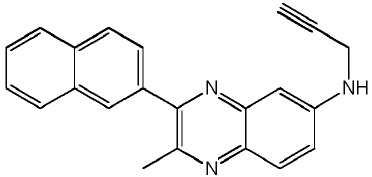 |
| **9bm** | 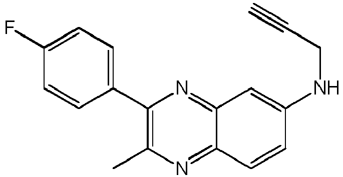 | **9b n** | 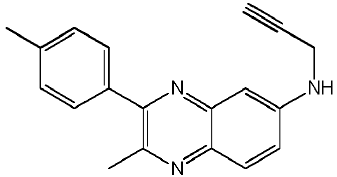 |
| **9bo** | 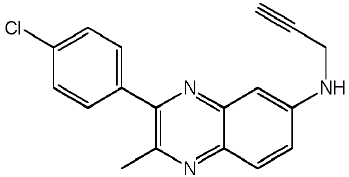 | **9b p** | 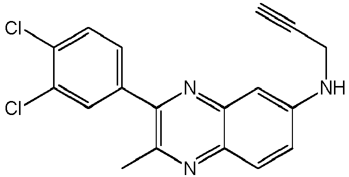 |
| **9ba** | 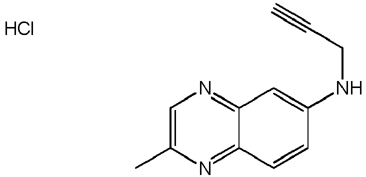 | | |

La présente invention a ainsi pour objet un composé de formule (I) tel que défini ci-dessus pour son utilisation dans le traitement ou la prévention de la maladie de Parkinson.

La présente demande décrit également l'utilisation d'un composé de formule (I) tel que défini ci-dessus pour la fabrication d'un médicament destiné au traitement ou à la prévention de la maladie de Parkinson.

La présente demande décrit également une composition pharmaceutique comprenant au moins un composé de formule (I) tel que défini ci-dessus et un véhicule pharmaceutiquement acceptable.

Ces compositions pharmaceutiques peuvent être formulées pour une administration parentérale (par exemple sous-cutanée, intra-péritonéale, intramusculaire, intraveineuse, intracranienne, intrathécale, etc.), orale, sublinguale, transdermique, locale ou rectale, destinée aux mammifères, y compris l'homme. La posologie varie selon le traitement et selon l'affection en cause.

Dans ces compositions pharmaceutiques, l'ingrédient actif peut être administré sous formes unitaires d'administration, en mélange avec des supports pharmaceutiques classiques, aux animaux ou aux êtres humains.

Les formes unitaires d'administration par voie orale appropriées comprennent les comprimés, les gélules, les poudres, les granules et les solutions ou suspensions orales, et les formes d'administration parentérale, notamment intra-péritonéale.

Lorsque l'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir peut contenir l'ingrédient actif conjointement avec un édulcorant, un antiseptique, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, de même qu'avec des correcteurs de goût ou des édulcorants.

Pour une administration parentérale, on utilise des suspensions aqueuses, des solutions salines isotoniques ou des solutions stériles et injectables qui contiennent des agents de dispersion et/ou des agents mouillants pharmacologiquement compatibles.

Le principe actif peut être formulé également sous forme de microcapsules, éventuellement avec un ou plusieurs supports additifs.

Les composés de l'invention peuvent être utilisés à des doses comprises entre 0,01 mg et 1000 mg par jour, donnés en une seule dose une fois par jour ou administrés en plusieurs doses tout au long de la journée, par exemple deux fois par jour en doses égales. La dose administrée par jour est avantageusement comprise entre 5 mg et 500 mg, encore plus avantageusement entre 10 mg et 200 mg. Il peut être nécessaire d'utiliser des doses sortant de ces gammes ce dont l'homme du métier pourra se rendre compte lui-même.

Selon un mode de réalisation particulier, la composition pharmaceutique telle que définie ci-dessus pourra comprendre en outre un autre principe actif, utile notamment dans le traitement ou la prévention de maladies neurodégénératives, et avantageusement choisi parmi des inhibiteurs d'acétylcholinestérase tels que le donépézil, la galanthamine, la rivastigmine, la mémantine et la tacrine ; des inhibiteurs de monoamines oxydase tels que la sélégiline ou rasagiline ; des inhibiteurs de catécholamines O-méthyltransférase tels que l'entacapone ; des inhibiteurs glutamatergiques tels que l'amantadine et le baclofène ; des agonistes cholinergiques tels que la sabcoméline ; des agonistes dopaminergiques tels que le pergolide, le cabergoline, le ropirinole et le pramipexole ; des analogues ou précurseurs de neuromédiateurs tels que la L-3,4-dihydroxyphénylalanine ; et des anticholinergiques tels que le trihexyphénidyl et la tropatépine.

La présente demande décrit également un composé de formule (I') suivante : 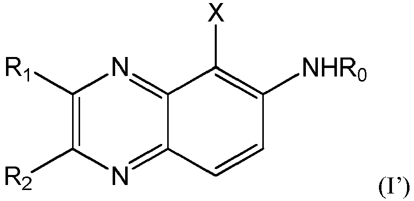 dans laquelle :
- X représente un atome d'hydrogène, un atome d'halogène tel qu'un brome ou un chlore, un groupe NO₂ ou NH₂,
- R₀ représente H ou -CH₂-C≡CH, et notamment H, et
- R₁ et R₂ représentent chacun, indépendamment l'un de l'autre, un atome d'hydrogène ; une chaîne hydrocarbonée saturée ou insaturée, linéaire ou ramifiée, comportant de 1 à 10, de préférence 1 à 6 atomes de carbone ; ou un groupe aryle éventuellement substitué,
ainsi que ses sels pharmaceutiquement acceptables, ses stéréoisomères ou mélanges de stéréoisomères en toutes proportions, en particulier un mélange d'énantiomères, et notamment un mélange racémique,
à l'exclusion des composés pour lesquels X et R₀ représentent chacun un atome d'hydrogène, et R₁ et R₂ représentent chacun, indépendamment l'un de l'autre, un atome d'hydrogène ; une chaîne hydrocarbonée saturée ou insaturée, linéaire ou ramifiée, comportant de 1 à 6 atomes de carbone ; ou un groupe aryle non substitué, et leurs sels pharmaceutiquement acceptables.

Les composés exclus correspondent à des composés décrits dans WO 2010/007179 comme intermédiaires de synthèse.

Selon un mode de réalisation particulier, le composé de formule (I') ne sera pas un composé pour lequel X et R₀ représentent chacun un atome d'hydrogène et R₁ et R₂ représentent chacun, indépendamment l'un de l'autre, un atome d'hydrogène ou un groupe aryle éventuellement substitué, ou un de ses sels pharmaceutiquement acceptables.

De préférence, le composé de formule (I') ne sera pas la 6-amino-5-bromoquinoxaline, la 5-nitro-6-amino-quinoxaline, la 3-méthyl-5-nitro-6-amino-quinoxaline, la 2-méthyl-5-nitro-6-amino-quinoxaline, la 5,6-diaminoquinoxaline, la 5,6-diamino-2-méthyl-quinoxaline, la 5,6-diamino-2-décyl-quinoxaline et la 5,6-diamino-3-décyl-quinoxaline. De tels composés sont décrits notamment dans WO 2011/044229 ; Nasielski-Hinkens et Kaisin Organic Mass Spectrometry 1985, 20(12), 733-737 ; et Nasielski et al. Bull. Soc. Chim. Belg. 1988, 97(10), 743-750.

Selon un premier mode de réalisation particulier, X et R₀ représentent chacun un atome d'hydrogène et au moins l'un des groupes R₁ et R₂ représente un groupe aryle substitué, notamment par un ou plusieurs groupes choisis parmi un atome d'halogène, un groupe (C₁-C₆)alkyle, (C₁-C₆)alcoxy, aryle, N₃, NO₂, NH₂, ou -NH-((C₁-C₆)alkyle), de préférence choisis parmi un atome d'halogène, un groupe (C₁-C₆)alkyle, (C₁-C₆)alcoxy ou aryle.

Ce groupe aryle substitué pourra être un groupe *p*-méthoxyphényle, m-méthoxyphényle, 3,4-diméthoxyphényle, 3,4,5-triméthoxyphényle, *p*-fluorophényle, *p*-méthylphényle, *p*-chlorophényle, 3,4-dichlorophényle, ou biphényle.

L'autre groupe R₁ ou R₂ pourra représenter alors un atome d'hydrogène ; un groupe (C₁-C₆)alkyle ; ou un groupe aryle, tel que phényle, éventuellement substitué, notamment par un ou plusieurs groupes choisis parmi un atome d'halogène, un groupe (C₁-C₆)alkyle, (C₁-C₆)alcoxy, aryle, N₃, NO₂, NH₂, ou -NH-((C₁-C₆)alkyle), de préférence choisis parmi un atome d'halogène, un groupe (C₁-C₆)alkyle, (C₁-C₆)alcoxy ou aryle.

Avantageusement, X et R₀ représentent chacun un atome d'hydrogène, l'un des groupes R₁ et R₂ représente un groupe aryle, tel que phényle, substitué, notamment par un ou plusieurs groupes choisis parmi un atome d'halogène, un groupe (C₁-C₆)alkyle, (C₁-C₆)alcoxy, aryle, N₃, NO₂, NH₂, ou -NH-((C₁-C₆)alkyle), et l'autre groupe R₁ ou R₂ représente une chaîne hydrocarbonée saturée ou insaturée, linéaire ou ramifiée, comportant de 1 à 10, de préférence 1 à 6 atomes de carbone, telle que un groupe (C₁-C₆)alkyle.

Dans le cadre de ce premier mode de réalisation particulier, le groupe (C₁-C₆)alkyle pourra être plus particulièrement un groupe méthyle, *n*-butyle, *n*-hexyle, *s*-butyle ou *t*-butyle ; et le groupe aryle éventuellement substitué pourra être plus particulièrement un groupe phényle *p*-méthoxyphényle, *m*-méthoxyphényle, 3,4-diméthoxyphényle, 3,4,5-triméthoxyphényle, 2-naphthyle, *p*-fluorophényle, *p*-méthylphényle, *p*-chlorophényle, 3,4-dichlorophényle, ou biphényle.

Avantageusement, R₁ représentera un groupe aryle, et notamment phényle, éventuellement substitué, en particulier par un ou plusieurs groupes choisis parmi un atome d'halogène, un groupe (C₁-C₆)alkyle, (C₁-C₆)alcoxy, aryle, N₃, NO₂, NH₂, ou -NH-((C₁-C₆)alkyle), de préférence choisis parmi un atome d'halogène, un groupe (C₁-C₆)alkyle, (C₁-C₆)alcoxy ou aryle. Ce groupe pourra notamment être choisi parmi les groupes phényle *p*-méthoxyphényle, *m*-méthoxyphényle, 3,4-diméthoxyphényle, 3,4,5-triméthoxyphényle, 2-naphthyle, *p*-fluorophényle, *p*-méthylphényle, *p*-chlorophényle, 3,4-dichlorophényle, et biphényle.

Selon un autre mode de réalisation de l'invention, X représente un atome d'halogène, un groupe NO₂ ou NH₂ ; et notamment représente un atome de brome, un groupe NO₂ ou NH₂, et/ou R₀ représente -CH₂-C≡CH.

R₁ et R₂ représenteront alors avantageusement chacun, indépendamment l'un de l'autre, un atome d'hydrogène ; un groupe (C₁-C₆)alkyle ; ou un groupe aryle éventuellement substitué, notamment par un ou plusieurs groupes choisis parmi un atome d'halogène, un groupe (C₁-C₆)alkyle, (C₁-C₆)alcoxy, aryle, N₃, NO₂, NH₂, ou -NH-((C₁-C₆)alkyle), de préférence choisis parmi un atome d'halogène, un groupe (C₁-C₆)alkyle, (C₁-C₆)alcoxy ou aryle.

En particulier, R₁ et R₂ pourront représenter chacun, indépendamment l'un de l'autre, un atome d'hydrogène ; un groupe (C₁-C₆)alkyle ; ou un groupe phényle éventuellement substitué, notamment par un ou plusieurs groupes choisis parmi un atome d'halogène, un groupe (C₁-C₆)alkyle, (C₁-C₆)alcoxy, aryle, N₃, NO₂, NH₂, ou -NH-((C₁-C₆)alkyle), de préférence choisis parmi un atome d'halogène, un groupe (C₁-C₆)alkyle, (C₁-C₆)alcoxy ou aryle.

Selon un mode de réalisation particulier, R₁ représentera un groupe aryle, et notamment phényle, éventuellement substitué, en particulier par un ou plusieurs groupes choisis parmi un atome d'halogène, un groupe (C₁-C₆)alkyle, (C₁-C₆)alcoxy, aryle, N₃, NO₂, NH₂, ou -NH-((C₁-C₆)alkyle), de préférence choisis parmi un atome d'halogène, un groupe (C₁-C₆)alkyle, (C₁-C₆)alcoxy ou aryle.

Selon un autre mode de réalisation particulier, R₂ représentera une chaîne hydrocarbonée saturée ou insaturée, linéaire ou ramifiée, comportant de 1 à 10, de préférence 1 à 6 atomes de carbone ; de préférence un groupe (C₁-C₆)alkyle.

Selon encore un autre mode de réalisation particulier :
- R₁ représentera une chaîne hydrocarbonée saturée ou insaturée, linéaire ou ramifiée, comportant de 1 à 10, de préférence 1 à 6 atomes de carbone, tel qu'un groupe (C₁-C₆)alkyle ; ou un groupe aryle éventuellement substitué, notamment par un ou plusieurs groupes choisis parmi un atome d'halogène, un groupe (C₁-C₆)alkyle, (C₁-C₆)alcoxy, aryle, N₃, NO₂, NH₂, ou -NH-((C₁-C₆)alkyle), et
- R₂ représentera une chaîne hydrocarbonée saturée ou insaturée, linéaire ou ramifiée, comportant de 1 à 10, de préférence 1 à 6 atomes de carbone, tel qu'un groupe (C₁-C₆)alkyle.

En particulier, les radicaux R₁ et R₂ pourront être définis comme suit :
- R₁ représentera un groupe (C₁-C₆)alkyle ou un groupe aryle éventuellement substitué, notamment par un ou plusieurs groupes choisis parmi un atome d'halogène, un groupe (C₁-C₆)alkyle, (C₁-C₆)alcoxy, aryle, N₃, NO₂, NH₂, ou -NH-((C₁-C₆)alkyle) ; avantageusement un groupe aryle éventuellement substitué, notamment par un ou plusieurs groupes choisis parmi un atome d'halogène, un groupe (C₁-C₆)alkyle, (C₁-C₆)alcoxy, aryle, N₃, NO₂, NH₂, ou -NH-((C₁-C₆)alkyle), et
- R₂ représentera un groupe (C₁-C₆)alkyle.

Dans les définitions de R₁ et R₂ ci-dessus, le groupe (C₁-C₆)alkyle pourra être plus particulièrement un groupe méthyle, *n*-butyle, *n*-hexyle, *s*-butyle ou *t*-butyle ; et le groupe aryle éventuellement substitué pourra être plus particulièrement un groupe phényle *p*-méthoxyphényle, *m*-méthoxyphényle, 3,4-diméthoxyphényle, 3,4,5-triméthoxyphényle, 2-naphthyle, *p*-fluorophényle, *p*-méthylphényle, *p*-chlorophényle, 3,4-dichlorophényle, ou biphényle.

Les composé de formule (I') pourront notamment être choisis parmi les composés suivants :

| | | | |
|---|---|---|---|
| **4bi** | 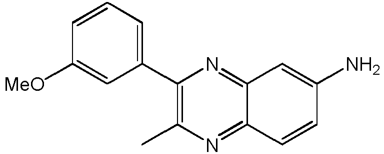 | **4bh** | 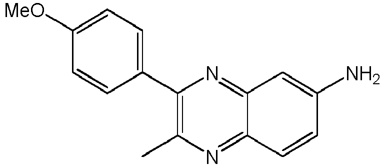 |
| **4bk** | 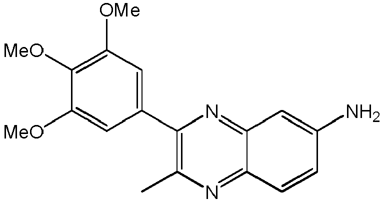 | **4bj** | 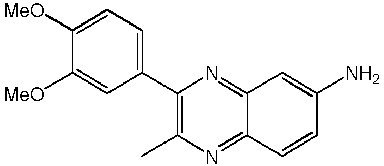 |
| **4bm** | 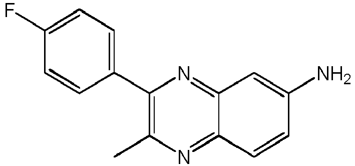 | **4bo** | 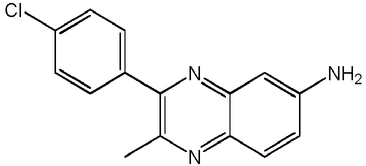 |
| **4bp** | 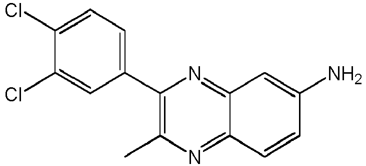 | **4bq** | 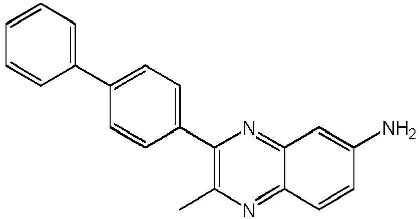 |
| **4bn** | 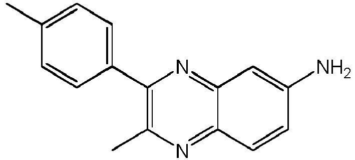 | **4ch** | 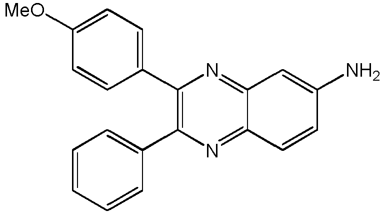 |
| **5aa** | 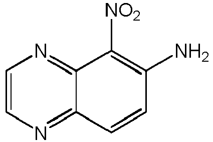 | **5bb** | 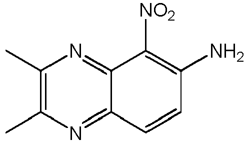 |
| **5ba** | 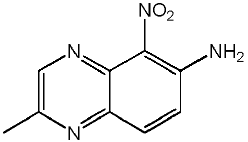 | **5bc** | 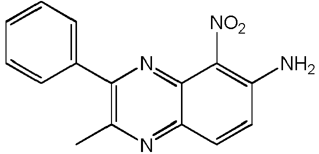 |
| **6aa** | 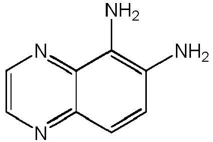 | **6bb** | 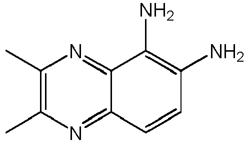 |
| **6ba** | 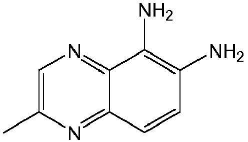 | **6bc** | 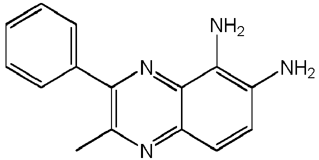 |
| **7aa** | 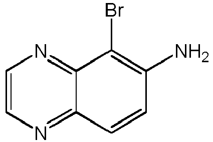 | **7bc** | 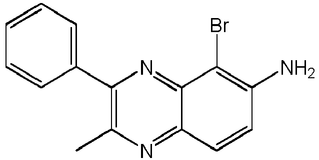 |
| **8bc** | 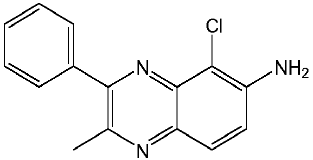 | **9bc** | 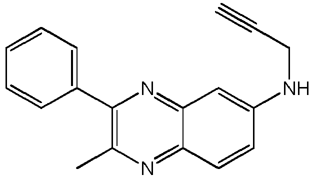 |
| **9bh** | 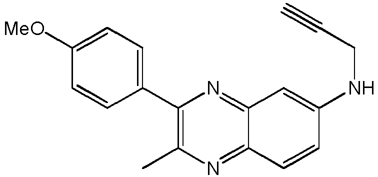 | **9bj** | 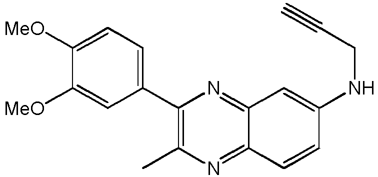 |
| **9bk** | 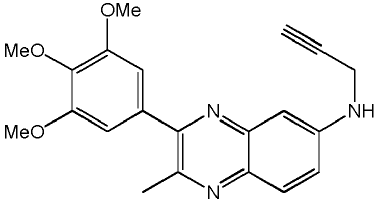 | **9bl** | 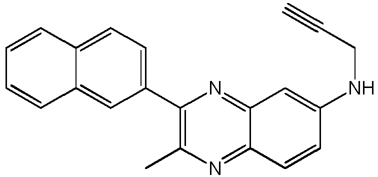 |
| **9bm** | 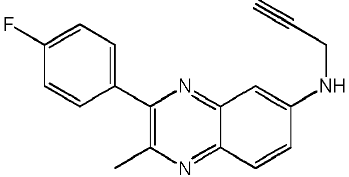 | **9bn** | 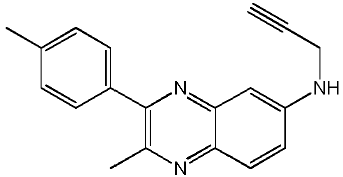 |
| **9bo** | 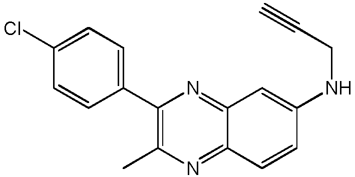 | **9bp** | 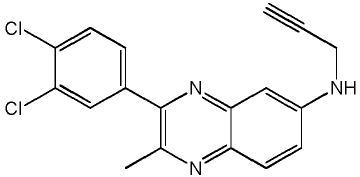 |
| **9ba** | 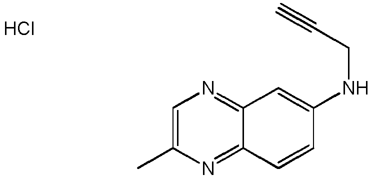 | | |

La présente demande décrit également un procédé (1) de préparation d'un composé de formule (I) ci-dessus pour lequel X = R₀ = H et au moins l'un des groupes R₁ et R₂ représente un groupe aryle substitué, comprenant les étapes successives suivantes :
(a1) couplage entre la 4-nitrophénylène-1,2-diamine et un composé de formule (II) suivante : 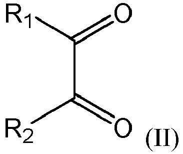 pour laquelle R₁ et R₂ sont tels que définis précédemment, l'un au moins des groupes R₁ et R₂ représentant un groupe aryle substitué,
   pour donner un composé de formule (III) suivante : 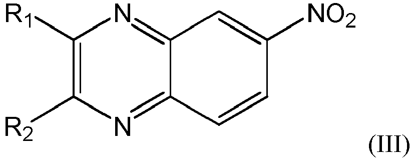 pour laquelle R₁ et R₂ sont tels que définis précédemment, l'un au moins des groupes R₁ et R₂ représentant un groupe aryle substitué, et
(b1) réduction de la fonction nitro du composé (III) obtenu à l'étape (a1) précédente pour donner un composé de formule (I) pour lequel X = R₀ = H et au moins l'un des groupes R₁ et R₂ représente un groupe aryle substitué.

De préférence, X représente un atome d'hydrogène, l'un des groupes R₁ et R₂ représente un groupe aryle, tel que phényle, substitué, notamment par un ou plusieurs groupes choisis parmi un atome d'halogène, un groupe (C₁-C₆)alkyle, (C₁-C₆)alcoxy, aryle, N₃, NO₂, NH₂, ou -NH-((C₁-C₆)alkyle), et l'autre groupe R₁ ou R₂ représente une chaîne hydrocarbonée saturée ou insaturée, linéaire ou ramifiée, comportant de 1 à 10, de préférence 1 à 6 atomes de carbone, telle que un groupe (C₁-C₆)alkyle.

### Etape (a1) :

Les produits de départ utilisés pour cette étape sont généralement disponibles dans le commerce ou peuvent être facilement préparés par des méthodes bien connues de l'homme du métier.

Cette réaction sera avantageusement réalisée dans l'eau comme solvant, notamment au reflux.

### Etape(b1) :

Le réducteur utilisé à l'étape (b1) pour réduire la fonction nitro en fonction amino sera avantageusement SnCl₂. Cette réaction pourra être réalisée dans l'éthanol, de préférence absolu, et avantageusement au reflux de celui-ci.

Cette étape de réduction pourra également être réalisée sous atmosphère d'hydrogène, en présence de palladium sur charbon.

Le composé ainsi obtenu pourra être séparé du milieu réactionnel par des méthodes bien connues de l'homme du métier, comme par exemple par extraction, évaporation du solvant ou encore par précipitation et filtration.

Le composé pourra être par ailleurs purifié si nécessaire par des techniques bien connues de l'homme du métier, comme par recristallisation si le composé est cristallin, par distillation, par chromatographie sur colonne sur gel de silice ou encore par chromatographie liquide haute performance (HPLC).

La présente demande décrit également un procédé (2) de préparation d'un composé de formule (I) ci-dessus pour lequel X = R₀ = H et au moins l'un des groupes R₁ et R₂ représente un groupe aryle substitué, comprenant les étapes successives suivantes :
(a2) réaction d'un composé de formule R₁Li pour lequel R₁ est tel que défini précédemment mais ne peut représenter un atome d'hydrogène, avec un composé de formule (Ia) suivante : 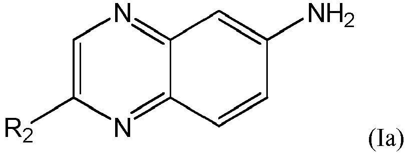 pour lequel R₂ est tel que défini précédemment,
   à une température comprise entre -100 et 25°C, notamment à environ -78°C, pour donner un composé de formule (IV) suivante : 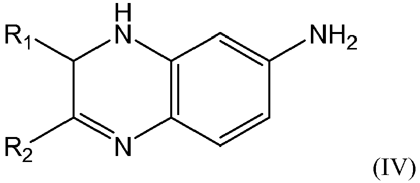 pour lequel R₁ et R₂ sont tels que définis précédemment avec R₁ ≠ H, l'un au moins des groupes R₁ et R₂ représentant un groupe aryle substitué,
(b2) éventuellement, lorsque R₂ = H dans la formule (IV) précédente, réaction du composé de formule (IV) obtenu à l'étape (a2) précédente avec un composé de formule R₂Li pour lequel R₂ est tel que défini précédemment mais ne peut représenter un atome d'hydrogène,
   à une température comprise entre -78 et 25°C, notamment à environ 0°C,
   pour donner un composé de formule (V) suivante : 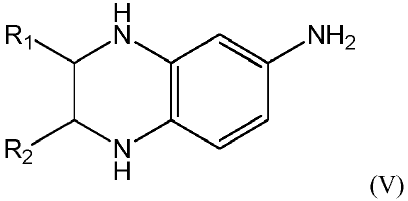 pour lesquels R₁ et R₂ sont tels que définis précédemment avec R₁ et R₂ ≠ H, l'un au moins des groupes R₁ et R₂ représentant un groupe aryle substitué, et
(c2) oxydation du composé de formule (IV) ou (V) obtenu respectivement à l'étape (a2) ou (b2) précédente pour donner un composé de formule (I) pour lequel R₁ et R₂ sont tels que définis précédemment, l'un au moins des groupes R₁ et R₂ représentant un groupe aryle substitué.

De préférence, X représente un atome d'hydrogène, l'un des groupes R₁ et R₂ représente un groupe aryle, tel que phényle, substitué, notamment par un ou plusieurs groupes choisis parmi un atome d'halogène, un groupe (C₁-C₆)alkyle, (C₁-C₆)alcoxy, aryle, N₃, NO₂, NH₂, ou -NH-((C₁-C₆)alkyle), et l'autre groupe R₁ ou R₂ représente une chaîne hydrocarbonée saturée ou insaturée, linéaire ou ramifiée, comportant de 1 à 10, de préférence 1 à 6 atomes de carbone, telle que un groupe (C₁-C₆)alkyle.

### Etape (a2) :

Le composé R₁Li utilisé pour cette étape est soit commercial, soit facilement accessible par des méthodes de synthèse bien connues de l'homme du métier.

Les composés de formule (Ia), quant à eux, peuvent être préparés selon le procédé (1) décrit ci-dessus ou par toute autre méthode connue de l'homme du métier.

Cette réaction sera avantageusement réalisée dans le tétrahydrofurane comme solvant.

### Etape(b2) :

Le composé R₂Li utilisé pour cette étape est soit commercial, soit facilement accessible par des méthodes de synthèse bien connues de l'homme du métier.

Cette réaction sera avantageusement réalisée dans le tétrahydrofurane comme solvant.

### Etape (c2) :

Cette étape pourra être réalisée en présence d'un oxydant, tel que MnO₂, permettant de réaromatiser le noyau quinoxaline.

La réaction pourra être réalisée dans le chloroforme utilisé comme solvant, notamment au reflux.

Le composé ainsi obtenu pourra être séparé du milieu réactionnel par des méthodes bien connues de l'homme du métier, comme par exemple par extraction, évaporation du solvant ou encore par précipitation et filtration.

Le composé pourra être par ailleurs purifié si nécessaire par des techniques bien connues de l'homme du métier, comme par recristallisation si le composé est cristallin, par distillation, par chromatographie sur colonne sur gel de silice ou encore par chromatographie liquide haute performance (HPLC).

La présente demande décrit également un procédé (3) de préparation d'un composé de formule (I) ci-dessus pour lequel X ≠ H, comprenant les étapes successives suivantes :
(a3) nitration ou halogénation d'un composé de formule (Ib) suivante : 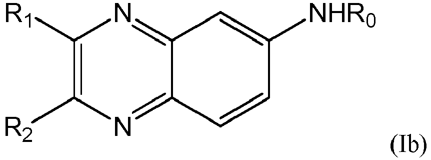 pour lequel R₀, R₁ et R₂ sont tels que définis précédemment, pour donner un composé de formule (Ic) suivante : 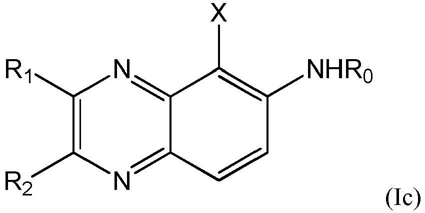 pour lequel R₀, R₁ et R₂ sont tels que définis précédemment et X représente un atome d'halogène ou un groupe NO₂, et
(b3) éventuellement, lorsque X = NO₂ dans la formule (Ic) précédente, réduction du composé de formule (Ic) obtenu à l'étape (a3) précédente pour donner un composé de formule (Id) suivante : 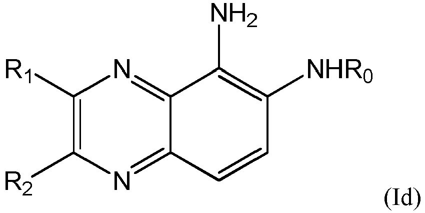 pour lequel R₀, R₁ et R₂ sont tels que définis précédemment.

### Etape (a3) :

Les composés de formule (Ib) peuvent être préparés notamment selon le procédé (1) ou (2) décrit ci-dessus lorsque R₀ = H ou selon le procédé (4) ci-dessous lorsque R₀ = CH₂-C≡CH.

La réaction de nitration pourra être réalisée en présence de KNO₃ dans l'acide sulfurique, notamment à une température inférieure à 0°C, par exemple à environ -10°C.

L'étape d'halogénation pourra être effectuée avec tout gent d'halogénation connu de l'homme du métier. Dans le cas d'une bromation, le dibrome pourra être utilisé, notamment dans l'acide acétique, par exemple à température ambiante. Dans le cas d'une chloration, le *N*-chlorosuccinimide pourra être utilisé, notamment dans le dichlorométhane comme solvant, par exemple à température ambiante.

### Etape(b3) :

Le réducteur utilisé à l'étape (b3) pour réduire la fonction nitro en fonction amino sera avantageusement SnCl₂. Cette réaction pourra être réalisée dans l'éthanol, de préférence absolu, et avantageusement au reflux de celui-ci.

Cette étape de réduction pourra également être réalisée sous atmosphère d'hydrogène, en présence de palladium sur charbon.

Le composé (Ic) ou (Id) ainsi obtenu pourra être séparé du milieu réactionnel par des méthodes bien connues de l'homme du métier, comme par exemple par extraction, évaporation du solvant ou encore par précipitation et filtration.

Le composé pourra être par ailleurs purifié si nécessaire par des techniques bien connues de l'homme du métier, comme par recristallisation si le composé est cristallin, par distillation, par chromatographie sur colonne sur gel de silice ou encore par chromatographie liquide haute performance (HPLC).

Il est à noter que les formules (Ia) à (Id) décrites précédemment représentent des sous-formules particulières de la formule (I) et doivent donc être considérées comme représentant des composés de formule (I).

La présente demande décrit également un procédé (4) de préparation d'un composé de formule (I) ci-dessus pour lequel R₀ = CH₂-C≡CH, comprenant la réaction d'un composé de formule (Ie) suivante : 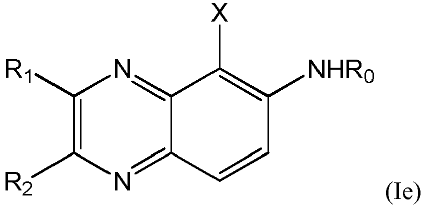 dans laquelle :
- X représente un atome d'hydrogène, un atome d'halogène tel qu'un brome ou un chlore, ou un groupe NO₂,
- R₀ représente H, et
- R₁ et R₂ représentent chacun, indépendamment l'un de l'autre, un atome d'hydrogène ; une chaîne hydrocarbonée saturée ou insaturée, linéaire ou ramifiée, comportant de 1 à 10, de préférence 1 à 6 atomes de carbone ; ou un groupe aryle éventuellement substitué,
avec un halogénure de propargyle, de préférence avec le bromure de propargyle.

La formule (Ie) représente donc une sous-formule particulière de la formule (I) selon l'invention (pour laquelle principalement R₀ = H) et doit donc être considérée comme représentant des composés de formule (I).

Les composés de formule (Ie) peuvent être préparés notamment selon les procédés (1), (2) et (3) décrits précédemment.

Des composés de formule (I) pour lesquels X = NH₂ ne peuvent être utilisés dans cette réaction car le motif propargyle pourrait s'additionner également sur cette fonction amino. Il est donc préférable dans ce cas d'effectuer la réaction de substitution par un motif propargyle à partir un composé pour lequel R₀ = H et X = NO₂ et de reduire ensuite cette fonction nitro en fonction amino conformément à l'étape (b3) du procédé (3) décrit ci-dessus.

La réaction avec l'halogénure de propargyle est avantageusement réalisée en milieu basique, notamment en présence d'une base telle que K₂CO₃. Elle pourra être réalisée en présence d'ions iodures avantageusement introduits par ajout d'un sel d'iodure tel que KI.

Cette réaction pourra être réalisée dans un solvant tel que le diméthylformamide, notamment à une température de 100°C.

Des étapes supplémentaires de protection/déprotection peuvent éventuellement être nécessaires pour protéger des fonctionnalités qui seraient sensibles/réactives dans les conditions de réaction ce que saura apprécier l'homme du métier.

Le composé ainsi obtenu pourra être séparé du milieu réactionnel par des méthodes bien connues de l'homme du métier, comme par exemple par extraction, évaporation du solvant ou encore par précipitation et filtration.

Le composé pourra être par ailleurs purifié si nécessaire par des techniques bien connues de l'homme du métier, comme par recristallisation si le composé est cristallin, par distillation, par chromatographie sur colonne sur gel de silice ou encore par chromatographie liquide haute performance (HPLC).

La présente invention sera mieux comprise à la lumière des exemples non limitatifs qui suivent.

### FIGURES

La **figure 1** présente des images de cultures soit non traitées (contrôle, B), soit traitées avec le composé **4bc** à 100 nM (A) ou avec la dbc-AMP à 200µM (C), dont les neurones dopaminergiques sont immunomarqués par reconnaissance de la tyrosine hydroxylase.
La **figure 2** présente un schéma de dissection du cerveau de souris en 3 parties.
La **figure 3** présente un diagramme représentant la moyenne des poids (g) des souris d'un même groupe en fonction du temps (j) durant le traitement *per os* pour des souris non traitées, des souris ayant reçu le véhicule ou des souris ayant reçu le composé **4bc** à raison de 300 mg/kg/jour.
La **figure 4** présente des chromatogrammes (abscisses : temps (min) ; ordonnées : intensité relative (%)) obtenus par HPLC-MS/MS à partir d'extraits de cerveaux de différentes souris de l'étude par voie IP de manière à quantifier le composé **4bc.**
La **figure 5** présente des chromatogrammes (abscisses : temps (min) ; ordonnées : intensité relative (%)) obtenus par HPLC-MS/MS à partir d'extraits de cerveaux de différentes souris de l'étude par voie IP euthanasiées 2 h après le dernier traitement, les extraits ayant été conservés 40 jours à 4°C avant analyse de manière à quantifier le composé **4bc.**
La **figure 6** présente des chromatogrammes (abscisses : temps (min) ; ordonnées : intensité relative (%)) obtenus par HPLC-MS/MS à partir d'extraits de cerveaux de différentes souris de l'étude par voie IP euthanasiées entre 4 et 6 h après le dernier traitement, les extraits ayant été conservés 40 jours à 4°C avant analyse de manière à détecter le composé **4bc.**
La **figure 7** présente des chromatogrammes (abscisses : temps (min) ; ordonnées : intensité relative (%)) obtenus par HPLC-MS/MS à partir d'extraits de cerveaux de différentes souris de l'étude *per os* de manière à vérifier la présence du composé **4bc.**
La **figure 8** présente la cinétique dans le cerveau et le plasma de souris du composé **4bc** après administration par voie IP.
La **figure 9** présente la densité optique moyenne des striatum droit (ST.R) et gauche (ST.L) des différents groupes de souris (NT/NL : non traitées / non lésées ; T/NL : traitées / non lésées ; NT/L : non traitées / lésées ; T/L : traitées / lésées) utilisés dans l'étude *in vivo* de neuroprotection du composé **4bc** administré par voie orale, vis-à-vis d'une toxine - le MPTP - administré par voie intra-nasale.
La **figure 10** présente la quantité moyenne de neurone tyrosine hydroxylase positifs (TH⁺) dans la substance noire des différents groupes de souris (NT/NL : non traitées / non lésées ; T/NL : traitées / non lésées ; NT/L : non traitées / lésées ; T/L : traitées / lésées) utilisés dans l'étude *in vivo* de neuroprotection du composé **4bc** administré par voie orale, vis-à-vis d'une toxine - le MPTP - administré par voie intra-nasale.

### EXEMPLES

Les abréviations suivantes ont été utilisées dans cette partie.
- AMP: Adénosine 3',5'-monophosphate
- dbc-AMP: Dibutyryl adénosine 3',5'-monophosphate cyclique
- DCM: Dichlorométhane
- DMSO: Diméthylsulfoxyde
- équiv.: Equivalent
- ESI: Ionisation par électrospray
- EtOH_{abs}: Ethanol absolu
- HPLC: Chromatographie Liquide Haute Performance
- HPLC-: Chromatographie Liquide Haute Performance couplée à deux
- MS/MS: spectrométries de masse
- IR: Infrarouge
- NCS: *N*-chloro-succinimide
- MPTP: 1-Méthyl-4-phényl-1,2,3,6-tétrahydropyridine
- MRM: Multiple Reaction Monitoring
- MS: Spectre de masse
- RMN: Résonance Magnétique Nucléaire
- t.a.: Température ambiante
- THF: Tétrahydrofurane
- UV: Ultraviolet

Les composés ont été nommés comme suit :
- premier suffixe = R₂
- second suffixe = R₁
avec **a** = H, **b** = Me, **c** = Ph, **d** = *n*-Bu, **e** = *n*-Hex, **f** = *s*-Bu, **g** = *t*-Bu, **h** = *p-*méthoxyphényle, **i** = *m*-méthoxyphényle, **j** = 3,4-diméthoxyphényle, **k** = 3,4,5-triméthoxyphényle, **l** = 2-naphthyle, **m** = *p*-fluorophényle, **n** = *p*-méthylphényle, **o** = *p*-chlorophényle, **p** = 3,4-dichlorophényle, **q** = biphényle.

### I. Synthèse des composés selon l'invention

### I.1. Procédures générales

Une condensation d'Hinsberg entre la 4-nitrophénylène-1,2-diamine **1** et le glyoxal **2aa,** le pyruvaldéhyde **2ba** ou le phénylglyoxal **2ca,** composés tous commerciaux, permet d'obtenir régiosélectivement la 6-nitroquinoxaline **3aa,** la 2-méthyl-6-nitroquinoxaline **3ba** ou la 2-phényl-6-nitroquinoxaline **3ca.** Une réduction de la fonction nitro en fonction amine par le chlorure d'étain (ou l'hydrogène en présence de palladium sur charbon) conduit à la 6-aminoquinoxaline **4aa,** la 2-méthyl-6-aminoquinoxaline **4ba** et la 2-phényl-6-aminoquinoxaline **4ca** (voir schéma 1). 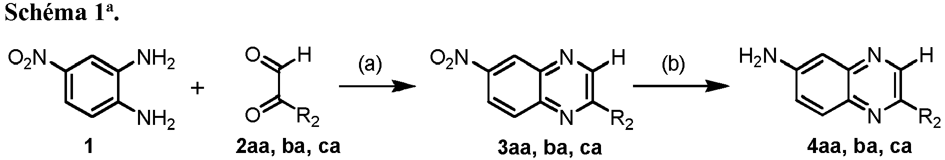 ^{a} Réactifs et conditions : (a) H₂O, reflux. (b) SnCl₂, EtOH_{abs}, reflux, ou H₂, Pd/C, 60°C.

Les substitutions en positions 2 et 3 de la quinoxaline sont introduites par une réaction impliquant des composés organolithiens. En effet, la substitution en position 3 du cycle par un organolithien est sélective à -78°C sur les composés **4aa, 4ba** ou **4ca,** permettant ainsi d'obtenir des quinoxalines diversement substituées en position 3. Une seconde substitution en position 2 du cycle est possible à 0°C lorsque la position 3 est déjà occupée par un substituant et que la position 2 est libre, permettant d'obtenir des quinoxalines disubstituées non-symétriques. Une réaromatisation au dioxyde de manganèse a permis d'obtenir les composés **4** diversement substitués (voir schéma 2). Il est également possible d'obtenir une quinoxaline **4** disubstituée en effectuant une réaction d'Hinsberg décrite précédemment avec une dicétone à la place du cétoaldéhyde (voir schéma 3). 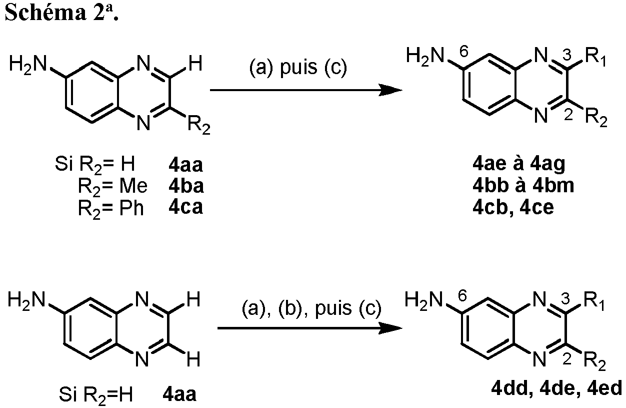 ^{a} Réactifs et conditions : (a) R₁Li, THF, -78°C. (b) R₂Li, THF, -78°C puis 0°C. (b) MnO₂, CHCl₃, reflux. 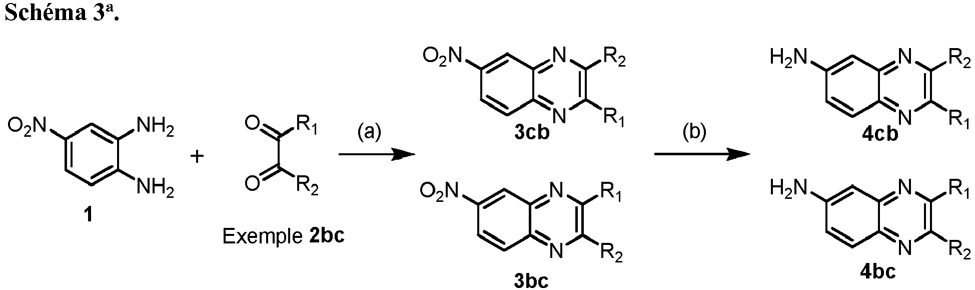 ^{a} Réactifs et conditions : (a) H₂O, reflux. (b) (i) SnCl₂, EtOH_{abs}, reflux, ou H₂, Pd/C, 60°C. (ii) Chromatographie, séparation des isomères.

Les quinoxalines **4** sont sélectivement substitués en position 5 par substitution aromatiques avec des électrophiles tels que l'ion nitronium produit avec du nitrate de potassium dans l'acide sulfurique donnant les composés nitrés **5,** ainsi que des halogénures comme du brome dans l'acide acétique donnant les composés halogénés **7.** A partir des dérivés nitrés **5,** les composés aminés **6** sont obtenus par réduction avec le chlorure d'étain (II) à reflux dans l'éthanol absolu ou encore sous atmosphère d'hydrogène en présence de Pd/C à 60°C (voir schéma 4). 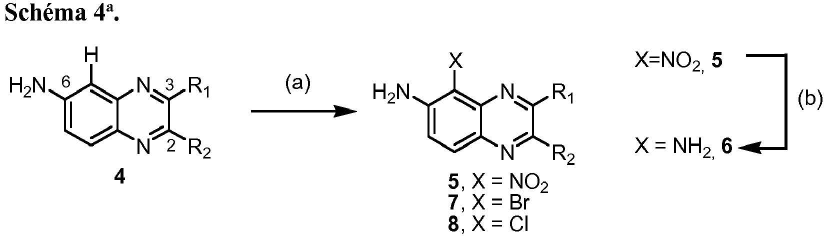 ^{a} Réactifs et conditions : (a) KNO₃, H₂SO₄, -10 °C ou Br₂, AcOH, t.a. ou NCS, DCM, t.a. (b) SnCl₂, EtOH_{abs}, reflux, ou H₂, Pd/C, 60°C.

La fonction amine des 6-amino-quinoxalines peut également être substituée par un groupe propargyle par réaction des quinoxalines avec le bromure de propargyle en condition basique dans le DMF à 100°C (voir schéma 5). 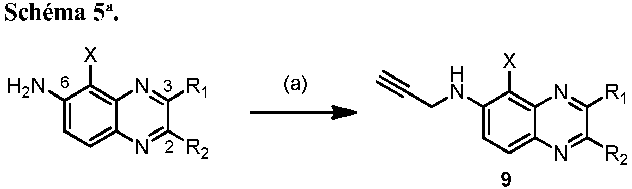 ^{a} Réactifs et conditions : (a) KI, K₂C0₃, DMF, 100°C.

### I.2. Synthèse des composés 3

| | | | | |
|---|---|---|---|---|
| **3aa** | 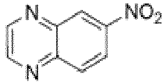 | C₈H₅N₃O₂ | 1 75 g. mol⁻¹ | Solide jaune |
| | 6-nitroquinoxaline | | | |

Une solution aqueuse (30 mL) de 4-nitro-phénylène-1,2-diamine commerciale (1,53 g, 10 mmol, 1 équiv.) et de glyoxal (40 % dans l'eau) commercial (1,8 mL, 10 mmol, 1 équiv.) est chauffée au reflux pendant 4 heures. Après refroidissement, le précipité est récupéré par filtration et lavé à l'eau. Le solide obtenu est séché à l'étuve à 100°C pendant 24 h.
Rendement : 93 % (1,63 g). RMN ¹H (400 MHz, CDCl₃) δ ppm : 8.26 (d, *J* = 9.2 Hz, 1H) ; 8.52 (dd, *J* = 9.2 Hz, *J* = 2.4 Hz, 1H) ; 9.01 (s, 3H). RMN ¹³C (50 MHz, CDCl₃) δ ppm : 123.4, 125.9, 131.3, 141.9, 145.3, 147.0, 147.6, 147.9. IR v cm⁻¹ : 3090, 3055, 1610, 1585, 1545, 1520, 1490, 1445, 1370, 1345, 1295, 1205, 1190, 1130, 1075, 955, 930, 870, 850, 810, 740. MS (ESI) m/z: 176 ([M+H]⁺, 23).

| | | | | |
|---|---|---|---|---|
| **3ba** | 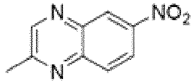 | C₉H₇N₃O₂ | 189 g mol⁻¹ | Solide ocre |
| | 2-méthyl-6-nitroquinoxaline | | | |

Une solution aqueuse (50 mL) de 4-nitro-phénylène-1,2-diamine commerciale (3,06 g, 20 mmol, 1 équiv.) et d'aldéhyde pyruvique (40 % dans l'eau) commercial (3,6 mL, 20 mmol, 1 équiv.) est chauffée au reflux pendant 1,5 heures. Après refroidissement, le précipité est récupéré par filtration et lavé à l'eau. Le solide obtenu est séché à l'étuve à 100°C pendant 24 h.
Rendement : 90 % (3,40 g). RMN ¹H (400 MHz, CDCl₃) δ ppm : 2.82 (s, 3H) ; 8.10 (d, *J* = 8.4 Hz, 1H) ; 8.45 (d, *J* = 8.0 Hz, 1H) ; 8.87 (s, 1H) ; 8.92 (s, 1H). RMN ¹³C (50 MHz, CDCl₃) δ ppm : 22.8, 123.3, 125.5, 130.2, 139.7, 144.5, 147.0, 148.1, 157.3. IR v cm⁻¹ : 3045, 2955, 1615, 1565, 1520, 1490, 1455, 1390, 1340, 1295, 1210, 1185, 1080, 965, 940, 930, 860, 830, 795, 745, 715. MS (ESI) m/z : 190 ([M+H]⁺, 100).

| | | | | |
|---|---|---|---|---|
| **3ca** | 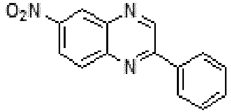 | C₁₄H₉N₃0₂ | 251,2g.mol-1 | Solide orangé |
| | 6-nitro-2-phenylquinoxaline | | | |

Une solution aqueuse (40 mL) de 4-nitro-phénylène-1,2-diamine commerciale (1,530 g, 10 mmol, 1 équiv.) et de phénylglyoxal monohydrate commercial (1,522 g, 10 mmol, 1 équiv.) est chauffée au reflux pendant 1 heures. Après refroidissement, le précipité est récupéré par filtration et lavé à l'eau. Le solide obtenu est séché à l'étuve à 100°C pendant 24 h.
Rendement : 98 % (2,455 g). RMN ¹H (400 MHz, CDCl₃) δ ppm : 7.61 (m, 3H), 8.24-8.29 (m, 3H), 8.55 (dd, *J* = 9.3 et 2.3 Hz, 1H), 9.02 (d, *J* = 2.4 Hz, 1H), 9.49 (s, 1H). RMN ¹³C (100 MHz, CDCl₃) δ ppm : 123.8, 125.6, 127.9 (2C), 129.4 (2C), 131.2, 131.4, 135.6, 140.3, 144.9, 145.5, 147.4, 154.3.

| | | |
|---|---|---|
| 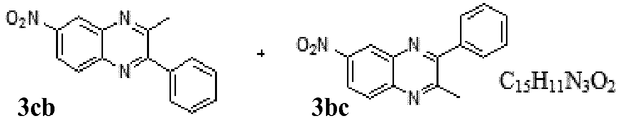 | 265,3 g.mol-1 | Solide orangé |
| 3-methyl-6-nitro-2-phenylquinoxaline 2-methyl-6-nitro-3-phenylquinoxaline | | |

Une solution hydro-alcoolique (20 mL isopropanol/eau 50:50) de 4-nitro-phénylène-1,2-diamine commerciale (0,516 g, 3,4 mmol, 1 équiv.) et de 1-phényl-1,2-propanedione commercial (0,45 mL, 3,4 mmol, 1 équiv.) est chauffée au reflux pendant 1,5 heures. Après ajout de 30 ml d'eau et refroidissement, le précipité est récupéré par filtration et lavé à l'eau. Le solide de couleur orangé obtenu est séché à l'étuve à 100°C pendant 24 h.
Rendement : 93 % (834,4 mg).

### I.3. Synthèse des composés 4

| | | | | |
|---|---|---|---|---|
| **4ba** | 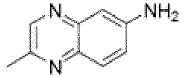 | C₉H₉N₃ | 159 g. mol⁻¹ | Solide rouge marron |
| | 2-méthyl-6-aminoquinoxaline | λabs= 280 nm | λem= 509 nm | |

A une solution de composé 3-méthyl-6-nitroquinoxaline **3ba** (20 mmol, 1 équiv.) dans l'éthanol (100 ml), 318 mg de Pd/C (10% m/m) sont ajoutés. La réaction est agitée à 60°C et placée sous hydrogène pendant 4 h. Après refroidissement, la réaction est filtrée sur célite puis rincée à l'éthanol et le filtrat est concentré sous pression réduite.
Rendement : 80 % (2,54 g). RMN ¹H (300 MHz, CDCl₃) δ ppm : 2.62 (s, 3H) ; 4.22 (s, 2H) ; 7.09-7.11 (m, 2H) ; 7.73 (d, *J* = 4.2 Hz, 1H) ; 8.52 (s, 1H). RMN ¹³C (75 MHz, CDCl₃) δ ppm : 149.3, 147.1, 145.7, 142.5, 141.7, 136.8, 129.4, 121.7, 108.1, 121.8. IR v cm⁻¹ : 3330, 3205, 3055, 2920, 1615, 1555, 1500, 1475, 1420, 1365, 1345, 1310, 1230, 1210, 1170, 1130, 1015, 970, 940, 910, 830, 780, 755, 730. MS (ESI) m/z: 160 ([M+H]⁺, 100).

| | | | | |
|---|---|---|---|---|
| **4bd** | 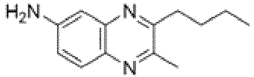 | C₁₃H₁₇N₃ | 215 g. mol⁻¹ | Solide jaune |
| | 3-butyl-2-méthyl-6-aminoquinoxaline | λabs= 280 nm | λem= 495 nm | |

A une solution de 2-méthyl-6-aminoquinoxaline (1 mmol, 1 equiv.) dans le THF (2 mL), placée sous atmosphère inerte d'azote à -78°C, est ajouté lentement du n-BuLi (1 mL, 2,5 mmol, 1,5 équiv.). La solution vire immédiatement au rouge sombre. Le mélange réactionnel est agité à -78°C pendant 2,5 heures, hydrolysé par 3 mL d'une solution aqueuse saturée de NH₄Cl puis extrait trois fois à l'acétate d'éthyle (3 x 20 mL). Les phases organiques réunies sont ensuite lavées par 100 mL d'une solution aqueuse saturée de NaCl, séchées sur MgSO₄, filtrées puis concentrées sous pression réduite. Le résidu obtenu est ensuite repris dans le CHCl₃ (20 mL) puis 430 mg de MnO₂ (5 mmol, 5 équiv.) sont ajoutés. La solution est chauffée au reflux pendant 4 heures. Le mélange réactionnel est ensuite hydrolysé par 2 mL d'eau, filtré sur célite puis lavé par l'acétate d'éthyle (30 mL). La phase organique est séchée avec du MgSO₄ puis évaporée sous pression réduite. Une purification sur gel de silice dans un mélange de cyclohexane et d'acétate d'éthyle en proportions 80 : 20 a permis d'obtenir le composé **4bd.**
Rendement : 75 % (1,61 g). RMN ¹H (300 MHz, CDCl₃) δ ppm : 0.98 (t, *J* = 7.2 Hz, 3H) ; 1.47 (m, 2H) ; 1.77 (m, 2H) ; 2.90 (t, *J* = 7.8 Hz, 2H) ; 4.04 (s, 2H) ; 7.07 (m, 2H) ; 7.75 (d, *J* = 8.1 Hz, 1H). RMN ¹³C (75 MHz, CDCl₃) δ ppm : 13.9, 22.3, 22.8, 30.5, 35.7, 108.1, 120.5, 129.1, 135.7, 142.7, 146.9, 148.9, 156.9. IR v cm⁻¹ : 3320, 3170, 2955, 2925, 2870, 1655, 1620, 1555, 1500, 1460, 1375, 1345, 1315, 1285, 1250, 1160, 1130, 1105, 1075, 1010, 970, 955, 875, 855, 830, 790, 780, 740, 705. MS (ESI) m/z : 216 ([M+H]⁺, 100).

| | | | | |
|---|---|---|---|---|
| **4be** | 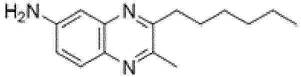 | C₁₅H₂₁N₃ | 243 g. mol⁻¹ | Solide jaune |
| | 3-hexyl-2-méthyl-6-aminoquinoxaline | | | |

Méthode d'obtention similaire à celle utilisée pour la synthèse du composé **4bd.**
Rendement : 65 % (158 mg). RMN ¹H (300 MHz, CDCl₃) δ ppm : 0.89 (t, *J* = 6.6 Hz, 3H) ; 1.21 (m, 4H) ; 1.47 (m, 2H) ; 1.77 (m, 2H) ; 2.68 (s, 3H) ; 2.92 (t, *J* = 8.1 Hz, 2H) ; 4.05 (s, 2H) ; 7.06 (m, 2H) ; 7.77 (d, *J* = 8.4 Hz, 1H). RMN ¹³C (75 MHz, CDCl₃) δ ppm : 14.0, 22.3, 22.6, 28.4, 29.4, 31.6, 108.2, 120.5, 129.2, 135.7, 142.8, 146.9, 148.8, 156.9. IR v cm⁻¹ : 3340, 3225, 2955, 2925, 2855, 2360, 2345, 2145, 2005, 1690, 1620, 1585, 1545, 1500, 1465, 1375, 1345, 1240, 1135, 1080, 1005, 830. MS (ESI) m/z: 244 ([M+H]⁺, 100).

| | | | | |
|---|---|---|---|---|
| **4bf** | 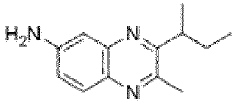 | C₁₃H₁₇N₃ | 215 g. mol⁻¹ | Solide jaune |
| | 3-*sec*-butyl-2-méthyl-6-aminoquinoxaline | | | |

Méthode d'obtention similaire à celle utilisée pour la synthèse du composé **4bd.**
Rendement : 65 % (140 mg). RMN ¹H (300 MHz, CDCl₃) δ ppm : 0.89 (t, *J* = 7.5 Hz, 3H) ; 1.32 (d, *J* = 6.9 Hz, 3H) ; 1.63 (m, 1H) ; 1.92 (m, 1H); 2.69 (s, 3H) ; 3.14 (sex, *J* = 6.9 Hz, 1H) ; 4.02 (s, 2H); 7.09 (m, 2H) ; 7.74 (d, *J* = 8.7 Hz, 1H). RMN ¹³C (75 MHz, CDCl₃) δ ppm : 12.3, 19.3, 22.4, 28.9, 38.9, 108.5, 120.5, 129.0, 135.3, 140.6, 146.8, 148.7, 160.7. IR v cm⁻¹ : 3345, 3220, 2960, 2925, 2870, 2360, 1620, 1555, 1500, 1460, 1375, 1320, 1235, 1180, 1130, 1075, 1050, 1020, 1000, 910, 855, 830, 730. MS (ESI) m/z : 216 ([M+H]⁺, 100).

| | | | | |
|---|---|---|---|---|
| **4bg** | 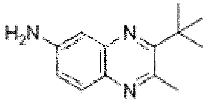 | C₁₃H₁₇N₃ | 215 g. mol⁻¹ | Solide jaune |
| | 3-*tert*-butyl-2-méthyl-6-aminoquinoxaline | | | |

Méthode d'obtention similaire à celle utilisée pour la synthèse du composé **4bd.**
Rendement : 25 % (54 mg). RMN ¹H (300 MHz, CDCl₃) δ ppm : 1.51 (s, 9H); 2.84 (s, 3H) ; 4.22 (s, 2H) ; 7.09 (m, 2H) ; 7.73 (d, *J* = 9.0 Hz, 1H). RMN ¹³C (75 MHz, CDCl₃) δ ppm : 25.7, 29.4, 108.8, 120.7, 128.7, 134.9, 141.5, 146.8, 148.4, 162.2. IR v cm⁻¹ : 3340, 3220, 2965, 2930, 2870, 2360, 1620, 1565, 1545, 1495, 1455, 1410, 1395, 1365, 1325, 1245, 1200, 1130, 1070, 1000, 910, 855, 830, 785, 730. MS (ESI) m/z : 216 ([M+H]⁺, 100).

| | | | | |
|---|---|---|---|---|
| **4bc** | 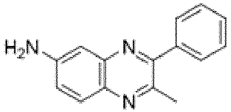 | C₁₅H₁₃N₃ | 235 g. mol⁻¹ | Solide j aune |
| | 2-méthyl-3-phényl-6-aminoquinoxaline | λabs= 283 nm | λem= 505 nm | |

Méthode d'obtention similaire à celle utilisée pour la synthèse du composé **4bd.**
Rendement : 75 % (176 mg). RMN ¹H (300 MHz, CDCl₃) δ ppm : 2.68 (s, 3H) ; 3.88 (s, 2H) ; 7.16 (m, 2H) ; 7.48 (m, 3H) ; 7.61 (m, 2H) ; 7.83 (d, *J* = 8.7 Hz, 1H). RMN ¹³C (75 MHz, CDCl₃) δ ppm : 23.8, 108.4, 121.7, 128.4, 128.7, 128.9, 129.2, 136.2, 139.4, 142.6, 147.3, 148.0, 154.8. IR v cm⁻¹ : 3210, 2965, 2925, 2480, 2215, 1965, 1625, 1560, 1515, 1490, 1420, 1380, 1345, 1325, 1255, 1160, 1005, 970, 905, 830, 775, 725. MS (ESI) m/z : 236 ([M+H]⁺, 100).

| | | | | |
|---|---|---|---|---|
| **4bb** | 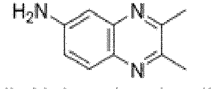 | C₁₀H₁₁N₃ | 173 g. mol⁻¹ | Solide jaune |
| | 2,3-diméthyl-6-aminoquinoxaline | | | |

Méthode d'obtention similaire à celle utilisée pour la synthèse du composé **4bd.**
Rendement : 8 % (176 mg). RMN ¹H (200 MHz, CDCl₃) δ ppm : 2.58 (s, 3H) ; 2.59 (s, 3H) ; 4.11 (s, 2H) ; 6.98 (m, 2H) ; 7.67 (d, *J* = 8.4 Hz, 1H). RMN ¹³C (75 MHz, CDCl₃) δ ppm : 22.52, 22.95, 107.86, 120.45, 129.08, 135.78, 142.68, 147.04, 149.01, 153.21. IR v cm⁻¹ : 680, 728, 793, 829, 913, 949, 996, 1128, 1160, 1243, 1340, 1379, 1445, 1466, 1501, 1560, 1618, 2919, 2947, 2990, 3030, 3208, 3329. MS (ESI) m/z : 174 ([M+H]⁺, 100).

| | | | | |
|---|---|---|---|---|
| **4aa** | 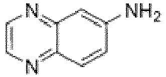 | C₈H₇N₃ | 145 g. mol⁻¹ | Solide jaune |
| | 6-aminoquinoxaline | λabs= 280 nm | λem= 506 nm | |

Méthode d'obtention similaire à celle utilisée pour la synthèse du composé **4ba.**
Rendement : 80 % (2,32 g). RMN ¹H (400 MHz, CDCl₃) δ ppm : 4.10 (s, 2H) ; 7.12 (d, *J* = 2.4 Hz, 1H) ; 7.15 (dd, *J* = 8.8 Hz, *J* = 2.4 Hz, 1H) ; 7.84 (d, *J* = 8.8 Hz, 1H) ; 8.52 (s, 1H) ; 8.62 (s, 1H). RMN ¹³C (50 MHz, CDCl₃) δ ppm : 148.1, 144.9, 140.9, 137.9, 130.3, 122.0, 107.8. IR v cm⁻¹ : 3395, 3315, 3185, 3055, 1645, 1615, 1545, 1500, 1470, 1435, 1370, 1310, 1225, 1210, 1135, 1030, 960, 860, 815, 765. MS (ESI) m/z : 146 ([M+H]⁺, 100).

| | | | | |
|---|---|---|---|---|
| **4ad** | 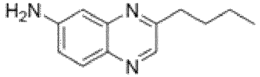 | C₁₂H₁₅N₃ | 201 g. mol⁻¹ | Solide jaune |
| | 3-butyl-2-méthyl-6-aminoquinoxaline | | | |

Méthode d'obtention similaire à celle utilisée pour la synthèse du composé **4bd.**
Rendement : 54 % (108 mg). RMN ¹H (400 MHz, CDCl₃) δ ppm : 0.93 (t, *J* = 7.2Hz, 3H) ; 1.40 (m, 2H) ; 1.74-1.82 (m, 2H) ; 2.89 (t, *J* = 7.5 Hz, 2H) ; 4.17 (s, 2H) ; 7.08 (m, 2H) ; 7.81 (d, *J* = 8.1 Hz, 1H) ; 8.44 (s, 1H). RMN ¹³C (50 MHz, CDCl₃) δ ppm : 13.9, 22.6, 31.7, 36.2, 107.9, 120.7, 130.1, 136.1, 141.8, 144.0, 147.9, 157.7. IR v cm⁻¹ : 3335, 3210, 2955, 2930, 2860, 1620, 1550, 1510, 1465, 1435, 1370, 1275, 1240, 1165, 1130, 1080, 995, 955, 905, 855, 830, 775, 730. MS (ESI) m/z : 202 ([M+H]⁺, 100).

| | | | | |
|---|---|---|---|---|
| **4dd** | 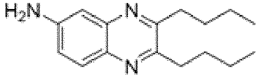 | C₁₆H₂₃N₃ | 257 g. mol⁻¹ | Solide jaune |
| | 2,3-dibutyl-6-aminoquinoxaline | | | |

Produit minoritaire obtenu lors de la synthèse du composé **4ad.**
RMN ¹H (200 MHz, CDCl₃) δ ppm : 0.95 (t, *J* = 7.2 Hz, 6H) ; 1.48 (m, 4H) ; 1.76 (m, 4H) ; 2.92 (m, 4H) ; 4.05 (s, 2H) ; 7.05 (m, 2H) ; 7.75 (d, *J* = 8.8Hz, 1H). RMN ¹³C (50 MHz, CDCl₃) δ ppm : 13.9, 22.9, 31.2, 31.3, 35.2, 34.9, 108.7, 120.5, 129.4, 135.9, 142.6, 146.9, 152.6, 156.6. IR v cm⁻¹ : 730, 830, 862, 962, 1076, 1137, 1173, 1232, 1346, 1375, 1419, 1463, 1499, 1564, 1629, 2870, 2928, 2953, 3197, 3317, 3444. MS (ESI) m/z : 258 ([M+H]⁺, 42).

| | | | | |
|---|---|---|---|---|
| **4ae** | 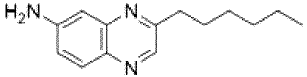 | C₁₄H₁₉N₃ | 229 g. mol⁻¹ | Solide jaune |
| | 3-hexyl-6-aminoquinoxaline | λabs= 280 nm | λem= 502 nm | |

Méthode d'obtention similaire à celle utilisée pour la synthèse du composé **4bd.**
Rendement : 53 % (122 mg). RMN ¹H (400 MHz, CDCl₃) δ ppm : 0.87 (t, *J* = 6.9 Hz, 3H) ; 1.29 (m, 6H) ; 1.77 (m, 4H) ; 2.88 (t, *J* = 7.8 Hz, 2H) ; 4.14 (s, 2H) ; 7.09 (m, 2H) ; 7.80 (d, *J* = 8.7 Hz, 1H) ; 8.42 (s, 1H). RMN ¹³C (50 MHz, CDCl₃) δ ppm : 14.0, 22.5, 29.1, 29.6, 31.6, 36.5, 108.0, 120.7, 130.0, 136.1, 141.8, 144.0, 147.9, 157.7. IR v cm⁻¹ : 3340, 3215, 2955, 2925, 2855, 2360, 1620, 1550, 1510, 1465, 1370, 1340, 1280, 1235, 1185, 1160, 1135, 1080, 975, 905, 830, 775, 730. MS (ESI) m/z : 230 ([M+H]⁺, 100).

| | | | | |
|---|---|---|---|---|
| **4af** | 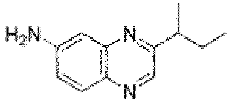 | C₁₂H₁₅N₃ | 201 g. mol⁻¹ | Solide jaune |
| | 3-*sec*-butyl-6-aminoquinoxaline | | | |

Méthode d'obtention similaire à celle utilisée pour la synthèse du composé **4bd.**
Rendement : 40 % (80 mg). RMN ¹H (300 MHz, CDCl₃) δ ppm : 0.90 (t, *J* = 7.5 Hz, 3H) ; 1.37 (d, *J* = 7.2 Hz, 3H) ; 1.73 (m, 1H) ; 1.88 (m, 1H) ; 2.96 (m, 1H) ; 4.12 (s, 2H) ; 7.09 (m, 2H) ; 7.84 (d, *J* = 9.6 Hz, 1H) ; 8.45 (s, 1H). RMN ¹³C (75 MHz, CDCl₃) δ ppm : 12.1, 19.9, 29.6, 42.1, 108.1, 120.7, 130.0, 136.3, 141.0, 144.0, 147.8, 161.4. IR v cm⁻¹ : 3340, 3215, 2960, 2925, 2875, 2360, 1620, 1545, 1510, 1460, 1430, 1370, 1275, 1250, 1230, 1175, 1130, 1275, 1250, 1230, 1175, 1130, 1085, 1050, 1015, 980, 960, 905, 855, 830, 775, 735. MS (ESI) m/z : 202 ([M+H]⁺, 100).

| | | | | |
|---|---|---|---|---|
| **4ag** | 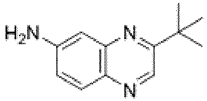 | C₁₂H₁₅N₃ | 201 g. mol⁻¹ | Solide jaune |
| | 3-*tert-*butyl-6-aminoquinoxaline | | | |

Méthode d'obtention similaire à celle utilisée pour la synthèse du composé **4bd.**
Rendement : 26 % (52 mg). RMN ¹H (300 MHz, CDCl₃) δ ppm : 1.44 (s, 9H) ; 4.21 (s, 2H) ; 7.05 (m, 1H) ; 7.78 (d, *J* = 9.3 Hz, 1H) ; 8.67 (s, 1H). RMN ¹³C (75 MHz, CDCl₃) δ ppm : 29.6, 36.9, 108.2, 120.7, 127.9, 135.6, 139.1, 143.2, 147.8, 163.6. IR v cm⁻¹ : 3335, 3215, 2960, 1620, 1545, 1505, 1460, 1430, 1365, 1280, 1245, 1200, 1110, 1020, 975, 955, 905, 855, 830, 775, 730. MS (ESI) m/z : 202 ([M+H]⁺, 100).

| | | | | |
|---|---|---|---|---|
| **4ac** | 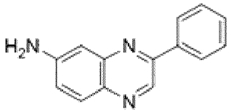 | C₁₄H₁₁N₃ | 221 g. mol⁻¹ | Solide jaune |
| | 3-phényl-6-aminoquinoxaline | | | |

Méthode d'obtention similaire à celle utilisée pour la synthèse du composé **4bd.**
Rendement : 28 % (62 mg). RMN ¹H (300 MHz, CDCl₃) δ ppm : 4.21 (brs, 2H NH), 7.16 (dd, *J* = 8.9 et 2,5 Hz, 1H), 7.21 (d, *J* = 2.5 Hz, 1H), 7.49-7.57 (m, 3H), 7.89 (d, *J* = 8.9 Hz, 1H), 8.14 (dd, *J* = 7.2 et 1,2 Hz, 2H), 9.04 (s, 1H). RMN ¹³C (75 MHz, CDCl₃) δ ppm : 107.5, 120.5, 126.5 (2C), 128.0 (2C), 128.8, 129.1, 135.7, 136.3, 138.5, 143.2, 147.2, 151.1. MS (ESI) m/z : 222,2 ([M+H]⁺, 100. Pureté (HPLC/UV 254 nm) : 100 %.

| | | | | |
|---|---|---|---|---|
| **4ed** | 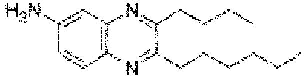 | C₁₈H₂₇N₃ | 285 g. mol⁻¹ | Solide jaune |
| | 3-butyl-2-hexyl-6-aminoquinoxaline | λabs= 280 nm | λem= 494 nm | |

A une solution de 6-aminoquinoxaline **4aa** (1 mmol, 1 équiv.) dans le THF (2 mL), placée sous atmosphère inerte d'azote à -78°C, est ajouté 1 mL (2,5 mmol, 2,5 équiv.) de *n*-BuLi à 2,5 M. La solution vire au rouge sombre. Le mélange est agité pendant 2,5 heures. La solution est ensuite placée à 0°C et 0,8 mL (2 mmol, 2 équiv.) de *n*-HexLi à 2,5 M est immédiatement ajouté goutte à goutte. Le mélange réactionnel est agité à 0°C pendant 2 heures puis hydrolysé par 5 mL d'une solution aqueuse saturée de NH₄Cl et extrait à l'acétate d'éthyle (3 x 20 mL). Les phases organiques réunies sont lavées par 100 mL d'une solution aqueuse saturée de NaCl, séchées avec du MgSO₄, filtrées puis concentrées sous pression réduite. Le résidu obtenu est repris dans le CHCl₃ (20 mL) puis 430 mg de MnO₂ (5 mmol, 5 équiv.) sont ajoutés et le mélange est agité au reflux pendant 4 h. Le mélange réactionnel est hydrolysé par 2 mL d'eau puis filtré sur célite. La phase organique est séchée avec du MgSO₄, filtrée puis concentrée sous pression réduite. Une purification par chromatographie sur gel de silice dans un mélange cyclohexane : acétate d'éthyle en proportions 50:50 a permis d'obtenir le composé **4ed.**
Rendement : 49 % (140 mg). RMN ¹H (300 MHz, CDCl₃) δ ppm : 0.96 (m, 6H) ; 1.25 (m, 8H) ; 1.40 (m, 2H) ; 1.70 (m, 2H) ; 2.90 (m, 4H) ; 4.10 (s, 2H) ; 7.05 (m, 2H) ; 7.74 (d, *J* = 9.6 Hz, 1H). RMN ¹³C (75 MHz, CDCl₃) δ ppm : 13.9, 14.0, 22.5, 29.1, 29.2, 29.3, 29.4, 31.6, 35.1, 35.4, 108.0, 120.5, 129.3, 135.8, 142.5, 146.9, 152.6, 156.6. IR v cm⁻¹ : 3335, 3215, 2955, 2855, 1620, 1500, 1465, 1340, 1235, 1135, 1080, 960, 930, 855, 830, 725. MS (ESI) m/z : 286 ([M+H]⁺, 40).

| | | | | |
|---|---|---|---|---|
| **4de** | 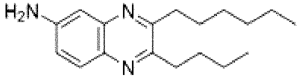 | C₁₈H₂₇N₃ | 285 g. mol⁻¹ | Solide jaune |
| | 2-butyl-3-hexyl-6-aminoquinoxaline | | | |

Méthode d'obtention similaire à celle utilisée pour la synthèse du composé **4ed.**
Rendement : 45 % (128 mg). RMN ¹H (300 MHz, CDCl₃) δ ppm : 0.92 (m, 6H) ; 1.25 (m, 8H) ; 1.40 (m, 2H) ; 1.72 (m, 2H) ; 2.92 (m, 4H) ; 4.06 (s, 2H) ; 7.06 (m, 2H) ; 7.77 (d, *J* = 7.8 Hz, 1H). RMN ¹³C (75 MHz, CDCl₃) δ ppm : 13.9, 14.0, 22.5, 22.8, 29.0, 29.1, 29.4, 31.6, 35.1, 35.4, 108.0, 120.5, 129.2, 135.8, 142.5, 146.9, 152.5, 156.6. IR v cm⁻¹: 3335, 3215, 2955, 2930, 2860, 1625, 1550, 1465, 1345, 1235, 1170, 1135, 1075, 960, 905, 855, 830, 730. MS (ESI) m/z : 286 ([M+H]⁺, 100).

| | | | | |
|---|---|---|---|---|
| **4cd** | 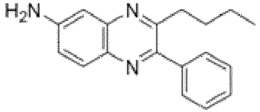 | C₁₈H₁₉N₃ | 277 g. mol⁻¹ | Solide jaune |
| | 3-butyl-2-phényl-6-aminoquinoxaline | | | |

Méthode d'obtention similaire à celle utilisée pour la synthèse du composé **4ed.**
Rendement : 32 % (88 mg). RMN ¹H (300 MHz, CDCl₃) δ ppm : 0.82 (t, *J* = 7.2 Hz, 3H) ; 1.25 (m, 4H) ; 1.67 (t, *J* = 7.8 Hz, 2H) ; 2.95 (t, *J* = 7.8 Hz, 2H) ; 4.15 (s, 2H) ; 7.13 (m, 2H) ; 7.43-7.58 (m, 5H) ; 7.88 (d, *J* = 8.7 Hz, 1H). RMN ¹³C (75 MHz, CDCl₃) δ ppm : 13.7, 22.6, 26.8, 31.3, 35.6, 107.5, 121.1, 128.0, 128.2, 128.3, 128.8, 129.6, 129.7, 130.1, 135.6, 139.5, 143.2, 147.8, 151.1, 156.2. IR v cm⁻¹ : 3335, 3215, 3060, 2955, 2925, 2855, 1620, 1580, 1560, 1495, 1460, 1445, 1420, 1345, 1240, 1135, 1075, 1010, 965, 910, 855, 830, 765, 730. MS (ESI) m/z : 278 ([M+H]⁺, 100).

| | | | | |
|---|---|---|---|---|
| **4bh** | 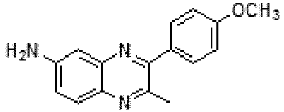 | C₁₆H₁₅N₃O | 265,3 g.mol⁻¹ | Solide jaune |
| | 3-(4-methoxyphenyl)-2-methylquinoxalin-6-amine | | | |

A une solution de 4-bromoanisole (8 mmol, 4 équiv.) dans l'éther anhydre (5 ml), 10 ml de *tert*-butyl lithium (1,6 M dans le pentane, 8 équiv.) sont ajoutés goutte à goutte à - 78°C sous atmosphère inerte d'azote. Après 1 h d'agitation à -78°C, une solution de composé **4ba** (2 mmol, 1 équiv.) dans le THF (5 ml) est ajoutée goutte à goutte. Le mélange réactionnel est agité à -78°C pendant 2 h, puis laissé réchauffé doucement à température ambiante.

Après 24 h, le milieu réactionnel est hydrolysé par une solution aqueuse saturée de NaHCO₃ puis extrait à l'acétate d'éthyle. Les phases organiques réunies sont ensuite lavées par une solution aqueuse saturée de NaCl puis séchées sur Na₂SO₄, filtrées et évaporées sous pression réduite.

Le résidu obtenu est ensuite repris dans le chloroforme puis 0,869 g de MnO₂ (10 mmol, 5 équiv.) sont ajoutés. Le milieu réactionnel est chauffé au reflux pendant 2 h puis laissé à température ambiante pendant 24 h.

Le milieu réactionnel est filtré sur célite puis concentré sous pression réduite. Une purification par chromatographie sur gel de silice dans un mélange DCM : MeOH en proportions 98 : 2 a permis d'obtenir le composé **4bh.**
Rendement : 34 % (168,2 mg). RMN ¹H (400 MHz, CDCl₃) δ ppm : 2.68 (s, 3H) ; 3.85 (s, 3H) ; 4.16 (brs, 2H) ; 7.00 (d, *J* = 8.4 Hz, 2H) ; 7.09 (d, *J* = 9.1 Hz, 1H) ; 7.14 (s, 1H) ; 7.58 (d, *J* = 9.1 Hz, 2H) ; 7.79 (d, *J* = 9.1 Hz, 1H). RMN ¹³C (100 MHz, CDCl₃) δ ppm : 23.8, 55.3, 108.3, 113.8 (2C), 121.4, 129.1, 130.3 (2C), 131.8, 135.9, 142.7, 147.3, 148.1, 154.4, 160.0. MS (ESI) m/z : 266,2 ([M+H]⁺, 100). Pureté (HPLC/UV 254 nm) : 100 %.

| | | | | |
|---|---|---|---|---|
| **4bi** | 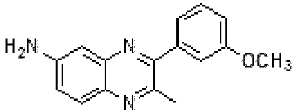 | C₁₆H₁₅N₃O | 265,3 g.mol⁻¹ | Solide jaune |
| | 3-(3-methoxyphenyl)-2-methylquinoxalin-6-amine | | | |

Méthode d'obtention similaire à celle utilisée pour la synthèse du composé **4bh.**
Rendement : 6 % (59,7 mg). RMN ¹H (300 MHz, CDCl₃) δ ppm : 2.70 (s, 3H), 3.88 (s, 3H), 3.90 (brs, 1H, NH) 4.20 (brs, 1H NH), 7.02 (d, *J* = 8.1 Hz, 1H), 7.12-7.23 (m, 4H), 7.42 (td, *J* = 8.1 et 1.5 Hz, 1H), 7.85 (dd, *J* =8.7 et 1.5 Hz, 1H). RMN ¹³C (75 MHz, CDCl₃) δ ppm : 23.7, 55.3, 108.3, 114.3, 114.5, 121.2, 121.7, 129.1, 129.4, 136.2, 140.7, 142.6, 147.4, 148.0, 154.6, 159.5. MS (ESI) m/z: 266,2 ([M+H]⁺, 100). Pureté (HPLC/UV 254 nm) : 100 %.

| | | | | |
|---|---|---|---|---|
| **4bj** | 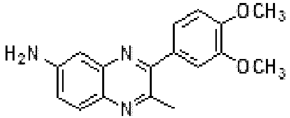 | C₁₇H₁₇N₃O₂ | 295,3 g.mol⁻¹ | Solide jaune |
| | 3-(3,4-dimethoxyphenyl)-2-methylquinoxalin-6-amine | | | |

Méthode d'obtention similaire à celle utilisée pour la synthèse du composé 4bh.
Rendement : 31 % (310,0 mg). RMN ¹H (400 MHz, CDCl₃) δ ppm : 2.74 (s, 3H), 3,94 (brs, 2H NH), 3.95 (s, 3H), 3.96 (s, 3H), 6.99 (d, *J* = 8.8 Hz, 1H), 7.17 (d, *J* = 8.8 Hz, 1H), 7.21 (s, 1H), 7.22 (d, *J* = 6.0 Hz, 1H), 7.27 (s, 1H), 7.87 (d, *J* = 8.8 Hz, 1H). RMN ¹³C (75 MHz, CDCl₃) δ ppm : 23.8, 56.0 (2C), 108.4, 110.9, 112.3, 121.5, 121.7, 129.2, 132.1, 136.1, 142.7, 147.3, 148.2, 148.9, 149.6, 154.5. MS (ESI) m/z : 296,2 ([M+H]⁺, 100). Pureté (HPLC/UV 254 nm) : 92 %.

| | | | | |
|---|---|---|---|---|
| **4bk** | 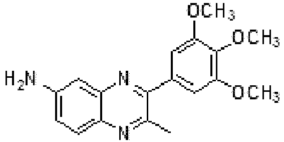 | C₁₈H₁₉N₃O₃ | 323,3 g.mol⁻¹ | Solide jaune |
| | 2-methyl-3-(3,4,5-trimethoxyphenyl)quinoxalin-6-amine | | | |

Méthode d'obtention similaire à celle utilisée pour la synthèse du composé **4bh.**
Rendement : 21 % (317,4 mg). RMN ¹H (400 MHz, CDCl₃) δ ppm : 2.72 (s, 3H), 3.92 (s, 9H), 4.28 (brs, 2H NH), 6.84 (s, 2H), 7.17 (dd, *J* = 8.8 et 2.4 Hz, 1H), 7.18 (s, 1H), 7.83 (d, *J* = 8.1 Hz, 1H). RMN ¹³C (100 MHz, CDCl₃) δ ppm : 23.7, 56.1 (2C), 60.8, 106.2 (2C), 108.0, 121.7, 129.1, 134.9, 136.1, 138.5, 142.4, 147.5, 147.8, 153.1 (2C), 154.4. MS (ESI) m/z : 326,1 ([M+H]⁺, 100), 348,2 ([M+Na]⁺, 100). Pureté (HPLC/UV 254 nm) : 96 %.

| | | | | |
|---|---|---|---|---|
| **4bl** | 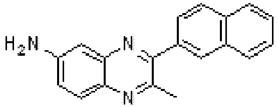 | C₁₉H₁₅N₃ | 285,3 g.mol⁻¹ | Solide marron |
| | 2-methyl-3-(naphthalen-2-yl)quinoxalin-6-amine | | | |

Méthode d'obtention similaire à celle utilisée pour la synthèse du composé **4bh.**
Rendement : 48 % (531,8 mg). RMN ¹H (400 MHz, CDCl₃) δ ppm : 2.74 (s, 3H), 4.14 (brs, 2H NH), 7.16(dd, *J* = 8.9 Hz et 2.5 Hz, 1H), 7.21 (d, *J* = 2.5 Hz, 1H), 7.52-7.57 (m, 2H), 7.74 (dd, *J* = 8.1 et 1.8 Hz, 1H), 7.86 (d, *J* = 8.9 Hz, 1H), 7.91 (d, *J* = 9.5 Hz, 1H), 7.92 (d, *J* = 9.5 Hz, 1H), 7.97 (d, *J* = 8.9 Hz, 1H), 8.10 (s, 1H). RMN ¹³C (100 MHz, CDCl₃) δ ppm : 23.9, 108.4, 121.7, 126.4, 126.7 (2C), 127.7, 128.1, 128.4, 128.5, 129.3, 133.1, 133.2, 136.3, 136.9, 142.8, 147.4, 148.3, 154.8. MS (ESI) m/z : 286,2 ([M+H]⁺, 100). Pureté (HPLC/UV 254 nm) : 88 %.

| | | | | |
|---|---|---|---|---|
| **4bm** | 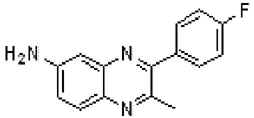 | C₁₅H₁₂FN₃ | 253,2 g.mol⁻¹ | Solide jaune |
| | 3-(4-fluorophenyl)-2-methylquinoxalin-6-amine | | | |

Méthode d'obtention similaire à celle utilisée pour la synthèse du composé **4bh.**
Rendement : 35 % (352,2 mg). RMN ¹H (400 MHz, CDCl₃) δ ppm : 2.68 (s, 3H), 3.90 (brs, 2H NH), 7.16-7.21 (m, 4H), 7.61 (d, *J* = 8.4 Hz, 1H), 7.63 (d, *J* = 8.4 Hz, 1H), 7.85 (d, *J* = 9.2 Hz, 1H). RMN ¹³C (100 MHz, CDCl₃) δ ppm : 23.7, 108.1, 115.5 (d, *J* = 21.5 Hz), 122.0, 129.2, 130.9 (d, *J* = 8.1 Hz), 135.3 (d, *J* = 2.5 Hz), 136.2, 142.6, 147.5, 147.8, 153.6, 163.1 (d, *J* = 250.4 Hz). MS (ESI) m/z : 254,2 ([M+H]⁺, 100). Pureté (HPLC/UV 254 nm) : 92 %.

| | | | | |
|---|---|---|---|---|
| **4bo** | 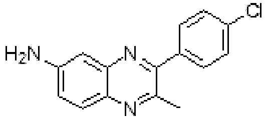 | C₁₅H₁₂ClN₃ | 269,7 g.mol⁻¹ | Solide jaune |
| | 3-(4-chlorophenyl)-2-methylquinoxalin-6-amine | | | |

Méthode d'obtention similaire à celle utilisée pour la synthèse du composé **4bh.**
Rendement : 70 % (1,1274 g). RMN ¹H (300 MHz, CDCl₃) δ ppm : 2.67 (s, 3H), 4.15 (brs, 2H NH), 7.15 (s, 1H), 7.16 (d, *J* = 7.5 Hz, 1H), 7.47 (d, *J* = 7.9 Hz, 2H), 7.58 (d, *J* = 7.9 Hz, 2H), 7.83 7.47 (d, *J* = 9.1 Hz, 1H). RMN ¹³C (75 MHz, CDCl₃) δ ppm : 23.7, 108.2, 122.0, 128.7 (2C), 129.3, 130.4 (2C), 134.9, 136.4, 137.9, 142.7, 147.4, 147.8, 153.5. MS (ESI) m/z : 270.1 (³⁵Cl) ([M+H]⁺, 100), 272.2 (³⁷Cl) ([M+H]⁺, 40).

| | | | | |
|---|---|---|---|---|
| **4bp** | 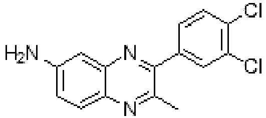 | C₁₅H₁₁Cl₂N₃ | 304,2 g.mol⁻¹ | Solide jaune |
| 3-(3,4-dichlorophenyl)-2-methylquinoxalin-6-amine | | | | |

Méthode d'obtention similaire à celle utilisée pour la synthèse du composé **4bh.**
Rendement : 45 % (813,9 mg). RMN ¹H (300 MHz, CDCl₃) δ ppm : 2.68 (s, 3H), 4.17 (brs, 2H NH), 7.14 (d, *J* = 2.0 Hz, 1H), 7.18 (dd, *J* = 9.1 et 2.3 Hz, 1H), 7.47 (dd, *J* = 8.4 et 2.0 Hz, 1H), 7.58 (d, *J* = 8.4 Hz, 1H), 7.75 (d, *J* = 2.0 Hz, 1H), 7.83 (d, *J* = 8.9 Hz, 1H). RMN ¹³C (75 MHz, CDCl₃) δ ppm : 23.7, 108.1, 122.3, 128.3, 129.3, 130.4, 131.1, 132.8, 133.1, 136.5, 139.4, 142.7, 147.4, 147.6, 152.2. MS (ESI) m/z: 304.1 (³⁵Cl et ³⁵Cl) ([M+H]⁺, 100), 306.1 (³⁵Cl et ³⁷Cl) ([M+H]⁺, 55).

| | | | | |
|---|---|---|---|---|
| **4bq** | 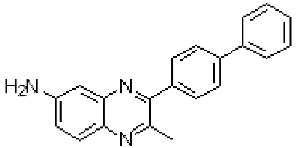 | C₂₁H₁₇N₃ | 311,4 g.mol⁻¹ | Solide marron |
| | 3-(biphenyl)-2-methylquinoxalin-6-amine | | | |

Méthode d'obtention similaire à celle utilisée pour la synthèse du composé **4bh.**
Rendement : 30 % (219,3 mg). RMN ¹H (300 MHz, CDCl₃) δ ppm : 2.77 (s, 3H), 4.23 (brs, 2H NH), 7.12 (dd, *J* = 8.7 et 1.8, 1H), 7.18 (d, *J* = 1.8, 1H), 7.38 (t, *J* = 7.5, 1H), 7.47 (t, *J* = 7.5, 2H), 7.66 (d, *J* = 7.5, 2H), 7.71 (m, 4H), 7.84 (d, *J* = 8.7, 1H). RMN ¹³C (75 MHz, CDCl₃) δ ppm : 23.7, 108.1, 121.7, 127.0 (4C), 127.5, 128.8 (2C), 129.0, 129.3 (2C), 136.0, 138.2, 140.4, 141.4, 142.7, 147.4, 147.9, 154.3. MS (ESI) m/z : 312.2 ([M+H]⁺, 100).

| | | | | |
|---|---|---|---|---|
| **4bn** | 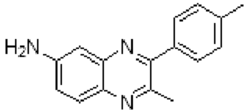 | C₁₆H₁₅N₃ | 249,3 g.mol⁻¹ | Solide jaune |
| | 2-methyl-3-*p*-tolyquinoxalin-6-amine | | | |

Méthode d'obtention similaire à celle utilisée pour la synthèse du composé **4bh.**
Rendement : 56 % (851,7 mg). RMN ¹H (300 MHz, CDCl₃) δ ppm : 2.45 (s, 3H), 2.72 (s, 3H), 4.28 (brs, 2H NH), 7.12 (dd, *J* = 8.9 et 2.1 Hz, 1H), 7.20 (d, *J* = 2.1 Hz, 1H), 7.32 (d, *J* = 7.6 Hz, 2H), 7.56 (d, *J* = 7.6 Hz, 2H), 7.84 (d, *J* = 8.9 Hz, 1H). RMN ¹³C (75 MHz, CDCl₃) δ ppm : 21.2, 23.7, 108.0, 121.5, 128.7 (2C), 128.9 (3C), 135.9, 136.4, 138.5, 142.6, 147.3, 147.9, 154.6. MS (ESI) m/z : 250.2 ([M+H]⁺, 100).

| | | | | |
|---|---|---|---|---|
| **4ca** | 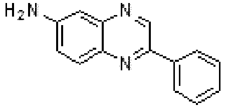 | C₁₄H₁₁N₃ | 221,2 g.mol-1 | Solide jaune |
| | 2-phenylquinoxalin-6-amine | | | |

A une solution de composé **3ca** (9,8 mmol) dans l'éthanol (150 ml), 245 mg de Pd/C (10 % m/m) sont ajoutés. La réaction est agitée à 60°C et placée sous hydrogène pendant 24 h. Après refroidissement, la réaction est filtrée sur célite puis rincée à l'éthanol. Le filtrat est enfin concentré sous pression réduite. Une purification par chromatographie sur gel de silice dans un mélange DCM : MeOH en proportions 98:2 a permis d'obtenir le composé **4ca** (majoritaire) ainsi que le composé **4ac** (minoritaire : 4 % (79,5 mg)).
Rendement : 21 % (453,3 mg). RMN ¹H (400 MHz, CDCl₃) δ ppm : 3.99 (brs, 2H NH), 7.17 (brs, 1H), 7.18 (dd, *J* = 8.9 et 2.6 Hz, 1H), 7.45 (tt, *J* = 7.4 et 1.6 Hz, 1H), 7.52 (td, *J* = 7.4 et 1.6 Hz, 2H), 7.9 (d, *J* = 8.9 Hz, 1H), 8.11 (dd, *J* = 7.3 et 1.6 Hz, 2H), 9.15 (s, 1H). RMN ¹³C (100 MHz, CDCl₃) δ ppm : 107.8, 122.2, 126.8 (2C), 128.9 (2C), 129.2, 130.5, 137.1 (2C), 143.2 (2C), 147.7, 148.1. MS (ESI) m/z: 222,2 ([M+H]⁺, 100). Pureté (HPLC/UV 254 nm) : 100 %.

| | | | | |
|---|---|---|---|---|
| **4ch** | 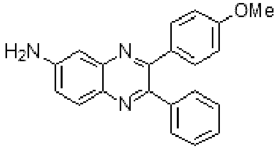 | C₂₁H₁₇N₃O | 327,4 g.mol⁻¹ | Solide jaune |
| | 3-(4-methoxyphenyl)-2-phenylquinoxalin-6-amine | | | |

Méthode d'obtention similaire à celle utilisée pour la synthèse du composé **4bh** mais à partir du composé **4ca.**
Rendement : 2 % (8,3 mg HPLC prep). RMN ¹H (300 MHz, CDCl₃) δ ppm : 3.63 (brs, 2H NH), 3.81 (s, 3H), 6.83 (d, *J* = 8.8 Hz, 2H), 7.16 (dd, *J* = 8.8 et 2.1 Hz, 1H), 7.22 (d, *J* = 2.2 Hz, 1H), 7.32 (m, 3H), 7.44 (d, *J* = 8.8 Hz, 2H), 7.46-7.49 (m, 2H), 7.94 (d, *J* = 8.9 Hz, 1H). RMN ¹³C (75 MHz, CDCl₃) δ ppm : 55.3, 107.9, 113.6 (2C), 121.7, 128.1, 128.2 (2C), 129.7 (2C), 130.2, 131.3 (2C), 131.7, 136.1, 139.7, 143.0, 148.0, 149.6, 152.9, 160.0. MS (ESI) m/z : 328.2 ([M+H]⁺, 100).

| | | | | |
|---|---|---|---|---|
| **4cb** | 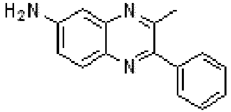 | C₁₅H₁₃N₃ | 235,3 g.mol-1 | Solide jaune |
| | 3-methyl-2-phenylquinoxalin-6-amine | | | |

A une solution du mélange de composés **3cb** et **3bc** (3 mmol) dans l'éthanol (100 ml), 80 mg de Pd/C (10 % m/m) sont ajoutés. La réaction est agitée à 60°C et placée sous hydrogène pendant 4 h. Après refroidissement, la réaction est filtrée sur célite puis rincée à l'éthanol et le filtrat est concentré sous pression réduite. Une purification par chromatographie sur gel de silice dans un mélange DCM : MeOH en proportions 98:2 a permis d'obtenir le composé **4cb** (minoritaire) ainsi que le composé **4bc** (majoritaire). (Ratio RMN (1:3)).
Rendement : 33 % sur RMN (3 mg isolé). RMN ¹H (300 MHz, CDCl₃) δ ppm : 2.70 (s, 3H), 4.18 (brs, 2H NH), 7.12 (s, 1H), 7.13 (dd, *J* = 2.5 et 8.2 Hz, 1H), 7.45-7.43 (m, 3H), 7.62 (dd, *J* = 7.6 et 2.0 Hz, 2H), 7.89 (dd, *J* = 8.2 et 1.1 Hz, 1H). RMN ¹³C (75 MHz, CDCl₃) δ ppm : 24.1, 107.2, 121.1, 128.3 (3C), 128.9 (2C), 130.1, 135.8, 139.3, 143.0, 147.9, 150.8, 152.3.

### I.4. Synthèse des composés 5

| | | | | |
|---|---|---|---|---|
| **5aa** | 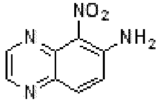 | C₈H₆N₄O₂ | 190,2 g.mol⁻¹ | Solide marron |
| | 5-nitroquinoxalin-6-amine | | | |

A une solution de composé **4aa** (1 équiv.) dans l'acide sulfurique (20 ml, d=1,83), du KNO₃ (1,25 équiv.) est ajouté lentement à une température inférieure à -10°C. La solution est agitée à cette température pendant 1 h, puis placée dans un bain eau-glace et laissée toute la nuit. Le mélange réactionnel est versé sur un mélange eau-glace, neutralisé par ajout de NH₄OH (d=0,9) puis extrait avec de l'AcOEt. La phase organique est lavée avec une solution saturée de NaCl, séchée sur MgSO₄ puis concentrée sous pression réduite. Une purification par chromatographie flash sur colonne de silice a permis d'obtenir le composé **5aa** désiré.
Rendement : 30,5 % (sur 2 étapes, à partir du composé **3aa** sans puririfcation intermédiaire). RMN ¹H (200 MHz, DMSO) δ ppm : 7.31 (s, 2H), 7.42 (d, *J* = 9.4 Hz, 1H), 7.90 (d, *J* = 9.4 Hz, 1H), 7.63 (d, *J* = 2.2 Hz, 1H), 8.76 (d, *J* = 2.0 Hz, 1H). RMN ¹³C (50 MHz, DMSO) δ ppm : 145.46, 144.29, 141.18, 138.06, 135.33, 133.12, 125.72, 123.82.
IR v cm⁻¹ : 3383, 3293, 3200, 3086, 2924, 2851, 1629, 1560, 1544, 1506, 1479, 1463, 1425, 1364, 1342, 1283, 1265, 1214, 1150, 1118, 1053, 1017, 892, 867, 837, 804, 793, 751. MS (ESI) m/z : 191 ([M+H]⁺, 20).

| | | | | |
|---|---|---|---|---|
| **5bb** | 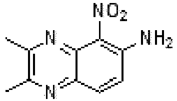 | C₁₀H₁₀N₄O₂ | 218,2 g.mol⁻¹ | Solide marron |
| | 2,3-dimethyl-5-nitroquinoxalin-6-amine | | | |

Méthode d'obtention similaire à celle utilisée pour la synthèse du composé **5aa.**
Rendement : 23,3 %. RMN ¹H (200 MHz, DMSO) δ ppm : 2.55 (s, 3H), 2.56 (s, 3H), 6.96 (s, 2H), 7.27 (d, *J* = 9.2Hz, 1H), 7.76 (d, *J* = 9.4 Hz, 1H). RMN ¹³C (50 MHz, DMSO)
δ ppm : 154.11, 149.34, 142.99, 135.61, 133.00, 132.05, 126.28, 121.75, 22.68, 21.80. IR v cm⁻¹ : 3459, 3336, 3200, 2920, 1630, 1548, 1508, 1481, 1457, 1427, 1381, 1361, 1350, 1285, 1255, 1211, 1168, 1044, 992, 954, 835, 797, 744, 687, 674. MS (ESI) m/z : 219 ([M+H]⁺, 15).

| | | | | |
|---|---|---|---|---|
| **5ba** | 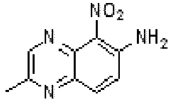 | C₉H₈N₄O₂ | 204,2 g.mol⁻¹ | Solide marron |
| | 2-methyl-5-nitroquinoxalin-6-amine | | | |

Méthode d'obtention similaire à celle utilisée pour la synthèse du composé **5aa.**
Rendement : 27,9 %. RMN ¹H (200 MHz, DMSO) δ ppm : 2.59 (s, 3H), 7.16 (s, 2H), 7.38 (d, *J* = 9.4 Hz, 1H), 7.82 (d, *J* = 9.4 Hz, 1H), 8.69 (s, 1H). RMN ¹³C (50 MHz, DMSO) δ ppm : 154.57, 149.76, 145.98, 143.45, 134.31, 132.53, 125.96, 123.44, 21.11. IR v cm⁻¹ : 3450, 3328, 3178, 1635, 1557, 1544, 1512, 1476, 1420, 1376, 1341, 1269, 1218, 1200, 1148, 1035, 1014, 949, 909, 833, 817, 799, 768. MS (ESI) m/z : 227 ([M+Na]⁺, 75).

| | | | | |
|---|---|---|---|---|
| **5bc** | 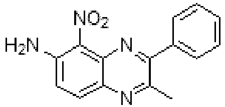 | C₁₅H₁₂N₄O₂ | 280,3 g.mol⁻¹ | Solide jaune |
| | 2-methyl-5-nitro-3-phenylquinoxalin-6-amine | | | |

Méthode d'obtention similaire à celle utilisée pour la synthèse du composé **5aa.**
Rendement : 20 % (121,2 mg). RMN ¹H (300 MHz, CDCl₃) δ ppm : 2.74 (s, 3H), 6.08 (brs, 2H NH), 7.11 (d, *J* = 9.5 Hz, 1H), 7.42-7.52 (m, 3H), 7.75 (d, *J* = 6.6 Hz, 2H), 7.83 (d, *J* = 9.2 Hz, 1H). RMN ¹³C (75 MHz, CDCl₃) δ ppm : 23.7, 122.5, 126.5, 128.4 (2C), 129.3, 129.4 (2C), 133.8, 134.7, 136.9, 138.3, 144.2, 148.9, 154.8.MS (ESI) m/z : 281.1 ([M+H]⁺, 50), 303.1 ([M+Na]⁺, 85), 583.2 ([2M+Na]⁺, 100).

### I.5. Synthèse des composés 6

| | | | | |
|---|---|---|---|---|
| **6aa** | 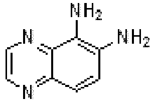 | C₈H₈N₄ | 160,2 g.mol⁻¹ | Solide marron |
| | quinoxaline-5,6-diamine | | | |

A une solution de composé **5aa** (1 équiv.) dans l'EtOH absolu (40 mL) est ajouté du SnCl₂ (5 equiv.). Le milieu réactionnel est placé au reflux pendant 42-68 h sous N₂, puis basifié (pH = 8) par ajout de NaHCO₃. Après filtration sur célite et ajout d'eau au filtrat obtenu, une extraction est réalisée avec de l'AcOEt. La phase organique est lavée à l'eau, séchée sur MgSO₄ puis concentrée sous pression réduite. Une purification par chromatographie flash sur colonne de gel de silice a permis d'obtenir le composé **6aa** désiré.
Rendement : 75,0 %. En partant de la 4-nitro-phénylène-1,2-diamine sans purification intermédiaire, le rendement est de 30,0 % (sur 3 étapes).
RMN ¹H (200 MHz, DMSO) δ ppm : 5.20 (s, 4H), 7.17 d, *J* = 8.8 Hz, 1H), 7.25 (d, *J* = 8.8 Hz, 1H), 8.49 (d, *J* = 1.8 Hz, 1H), 8.57 (d, *J* = 1.8 Hz, 1H). RMN ¹³C (50 MHz, DMSO) δ ppm : 141.83, 140.06, 136.88, 132.97, 132.42, 125.95, 121.79, 116.36. IR v cm⁻¹ : 3379, 3306, 3225, 1610, 1570, 1542, 1505, 1483, 1420, 1367, 1322, 1269, 1233, 1174, 1120, 1073, 1035, 908, 856, 827, 807, 779, 667. MS (ESI) m/z : 161([M+H]⁺, 100).

| | | | | |
|---|---|---|---|---|
| **6bb** | 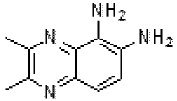 | C₁₀H₁₂N₄ | 188,2 g.mol⁻¹ | Solide marron |
| | 2,3-dimethylquinoxaline-5,6-diamine | | | |

Méthode d'obtention similaire à celle utilisée pour la synthèse du composé **6aa.**
Rendement : 7,9 % (sur 2 étapes, en partant du composé **4bb** sans purification intermédiaire). RMN ¹H (200 MHz, CDCl₃) δ ppm : 2.64 (s, 3H), 2.66 (s, 3H), 3.96 (s, 4H), 7.10 (d, *J* = 8.8 Hz, 7.32 (d, *J* = 8.8 Hz, 1H). RMN ¹³C (50 MHz, CDCl₃) δ ppm : 150.85, 149.64, 131.74, 127.44, 118.16, 23.08, 22.71. IR v cm⁻¹ : 3440, 3355, 3177, 2917, 1651, 1615, 1571, 1501, 1477, 1441, 1408, 1377, 1344, 1291, 1257, 1202, 1143, 999, 935, 820, 785, 733. MS (ESI) m/z : 189([M+H]⁺, 100).

| | | | | |
|---|---|---|---|---|
| **6ba** | 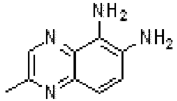 | C₉H₁₀N₄ | 174,2 g.mol⁻¹ | Solide marron |
| | 2-methylquinoxaline-5,6-diamine | | | |

Méthode d'obtention similaire à celle utilisée pour la synthèse du composé **6aa.**
Rendement : 42,6 %. RMN ¹H (200 MHz, DMSO) δ ppm : 2.56 (s, 3H), 5.12 (s, 4H), 7.07 (d, *J* = 8,6 Hz, 1H), 7.19 (d, *J* = 8.8 Hz, 1H), 8.50 (s, 1H). RMN ¹³C (50 MHz, DMSO) δ ppm : 148.06, 142.31, 135.86, 131.86, 130.56, 126.36, 121.47, 115.29, 21.38. IR v cm⁻¹ : 3380, 3280, 3218, 2918, 2852, 1671, 1613, 1571, 1497, 1478, 1420, 1355, 1309, 1296, 1267, 1238, 1172, 1115, 1066, 1014, 953, 903, 816, 784, 708, 683. MS (ESI) m/z : 175 ([M+H]⁺, 100).

| | | | | |
|---|---|---|---|---|
| **6bc** | 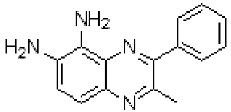 | C₁₅H₁₄N₄ | 250,3 g.mol⁻¹ | Solide jaune |
| | 2-methyl-3-phenylquinoxaline-5,6-diamine | | | |

Méthode d'obtention similaire à celle utilisée pour la synthèse du composé **6aa.**
Rendement : 90 % (10 mg). RMN ¹H (300 MHz, CDCl₃) δ ppm : 2.73 (s, 3H), 3.78-4.30 (2 brs, 4H), 7.23 (d, *J* = 8.6 Hz, 1H), 7.43 (d, *J* = 8.6 Hz, 1H), 7.48-7.55 (m, 3H), 7.69 (dd, *J* = 8.1 et 1.9 Hz, 2H). RMN ¹³C (75 MHz, CDCl₃) δ ppm : 24.0, 118.1, 122.1, 127.9, 128.3 (2C), 128.7, 129.2 (2C), 132.0, 132.4, 136.7, 139.6, 148.6, 152.1. MS (ESI) m/z : 251.2 ([M+H]⁺, 80).

### I.6. Synthèse des composés 7

| | | | | |
|---|---|---|---|---|
| **7aa** | 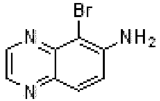 | C₈H₆BrN₃ | 224,1 g.mol⁻¹ | Solide marron |
| | 5-bromoquinoxalin-6-amine | | | |

A une solution de composé **4aa** (14,2 mmol) dans l'acide acétique (15 ml) à une température proche de 15°C est ajoutée lentement une solution de brome (14,4 mmol, 0,74 ml) dans 10 ml d'acide acétique glacial. Un solide rouge-orangé apparaît. Le mélange réactionnel est agité pendant 1 h, hydrolysé puis basifié avec une solution 1N de NaOH. Le mélange réactionnel est ensuite extrait avec de l'acétate d'éthyle (200 mL x 3), séché sur Na₂SO₄ puis concentré sous pression réduite pour donner 2,9918 g d'un solide marron.
Rendement : 94,0 %. RMN ¹H (400 MHz, CDCl₃) δ ppm : 4.80 (s, 2H), 7.24 (d, *J* = 9.2 Hz, 1H), 7.81 (d, *J* = 9.2 Hz, 1H), 8.56 (d, *J* = 1.6 Hz, 1H), 8.76 (d, *J* = 1.6 Hz, 1H). RMN ¹³C (50 MHz, CDCl₃) δ ppm : 145.91, 145.11, 142.08, 141.04, 138.56, 129.30, 121.27, 102.66. IR v cm⁻¹ : 3458, 3305, 3185, 3049, 1619, 1541, 1495, 1460, 1422, 1373, 1326, 1270, 1218, 1181, 1142, 1044, 974, 957, 861, 829, 788. MS (ESI) m/z : 224 ([M+H]⁺, 80), 226 ([M+H]⁺, 75).

| | | | | |
|---|---|---|---|---|
| **7bc** | 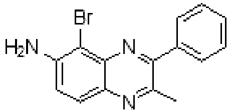 | C₁₅H₁₂BrN₃ | 314,2 g.mol⁻¹ | Solide marron |
| | 5-bromo-2-methyl-3-phenylquinoxalin-6-amine | | | |

Méthode d'obtention similaire à celle utilisée pour la synthèse du composé **7aa.**
Rendement : 90 % (385,2 mg). RMN ¹H (300 MHz, CDCl₃) δ ppm : 2.73 (s, 3H), 4.83 (brs, 2H NH), 7.13 (d, *J* = 8.7 Hz, 1H), 7.46 (m, 3H), 7.73 (m, 3H). RMN ¹³C (75 MHz, CDCl₃) δ ppm : 23.3, 102.9, 120.9, 127.4, 128.2 (2C), 128.8, 129.3 (2C), 135.7, 138.6, 140.0, 145.3, 147.7, 154.5. MS (ESI) m/z : 314 (⁷⁹Br) et 316 (⁸¹Br) ([M+H]⁺, 70), 336 (⁷⁹Br) et 338 (⁸¹Br) ([M+Na]⁺, 80), 651 ([2M+Na]⁺, 100).

### I.7. Synthèse des composés 8

| | | | | |
|---|---|---|---|---|
| **8bc** | 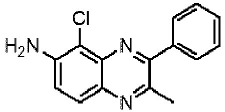 | C₁₅H₁₂ClN₃ | 269,5 g.mol⁻¹ | Solide marron |
| | 5-chloro-2-methyl-3-phenylquinoxalin-6-am ine | | | |

A une solution de composé **4bc** (208 mg, 0,89 mmol, 1 équiv.) dans le dichlorométhane anhydre à température ambiante est ajouté 118 mg de *N*-chlorosuccinimide (1,07 mmol, 1,2 équiv.). Le milieu réactionnel est ensuite mis au reflux du CH₂Cl₂ durant une nuit. Après refroidissement, le milieu réactionnel est hydrolysé par une solution de NaOH à 5 %, puis extrait 3 fois avec du CH₂Cl₂. Les phases organiques sont rassemblées, lavées avec une solution saturée de NaCl, séchées sur Na₂SO₄ puis concentrées sous pression réduite pour donner 279 mg du produit désiré.
Rendement : 99 % (279 mg). RMN ¹H (400 MHz, CDCl₃) δ ppm : 2.73 (s, 3H), 4.60 (brs, 2H NH), 7.19 (d, *J* = 9,2 Hz, 1H), 7.48 (m, 3H), 7.71 (dd, *J* = 8,6 et 1,3 Hz, 2H), 7.45 (d, *J* = 9,2 Hz, 1H). RMN ¹³C (100 MHz, CDCl₃) δ ppm : 23.7, 111.1, 120.8, 127.3, 128.3 (2C), 128.9, 129.3 (2C), 136.4, 139.0, 139.1, 143.6, 148.3, 154.6.

### I.8. Synthèse des composés 9

| | | | | |
|---|---|---|---|---|
| **9bc** | 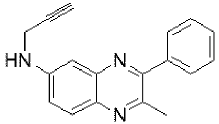 | C₁₈H₁₅N₃ | 273,4 g.mol⁻¹ | Solide jaune |
| 2-methyl-3-phenyl-*N-*(prop-2-ynyl)quinoxalin-6-amine | | | | |

A une solution de composé **4bc** (200 mg, 0,85 mmol, 1 équiv.) dans le diméthylformamide anhydre sont ajoutés 118 mg de K₂CO₃ (0,85 mmol, 1 équiv.), 141 mg de KI (0,85 mmol, 1 équiv.) et 0,19 ml de bromure de propargyle (1,7 mmol, 2 équiv.). Le milieu réactionnel est ensuite chauffé à 100°C pendant 24 h. Après refroidissement, le milieu réactionnel est hydrolysé par une solution saturée de K₂CO₃, puis extrait 3 fois avec de l'acétate d'éthyle. Les phases organiques sont rassemblées, lavées avec une solution saturée de NaCl, séchées sur Na₂SO₄ puis concentrées sous pression réduite. Une purification par chromatographie sur gel de silice dans un mélange cyclohexane : acétate d'éthyle en proportions 7:3 puis 6:4 a permis d'obtenir le composé **9bc** (majoritaire, 90,1 mg) ainsi que le composé dialkylé (minoritaire, 14 mg, non décrit).
Rendement : 40 % (90,1 mg). RMN ¹H (400 MHz, CDCl₃) δ ppm : 2.23 (t, *J* = 2,4 Hz, 1H), 2.67 (s, 3H), 4.01 (brs, 2H), 4,56 (brs, 1H, NH), 7.09 (dd, *J* = 2,6 et 8,8 Hz, 1H), 7.12 (d, *J* = 2,6 Hz, 1H), 7.43-7.50 (m, 3H), 7.61 (dd, *J* = 1,6 et 8,8 Hz, 2H), 7.81 (d, *J* = 8,8 Hz, 1H). RMN ¹³C (100 MHz, CDCl₃) δ ppm: 23.6, 33.3, 71.7, 79.9, 105.2, 121.6, 128.3 (2C), 128.6, 128.8 (2C), 128.9, 136.3, 139.4, 142.8, 147.3, 147.9, 154.6. MS (ESI) m/z : 274.2 ([M+H]⁺, 100). Pureté (HPLC/UV 260 nm) : 100 %.

| | | | | |
|---|---|---|---|---|
| **9bh** | 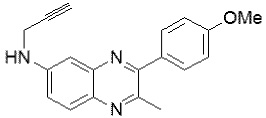 | C₁₉H₁₇N₃O | 303,4 g.mol⁻¹ | Solide jaune |
| | 3-(4-methoxyphenyl)-2-methyl-*N*-(prop-2-ynyl)quinoxalin-6-amine | | | |

Méthode d'obtention similaire à celle utilisée pour la synthèse du composé **9bc.**
Rendement : 71 % (280,0 mg). MS (ESI) m/z : 304.3 ([M+H]⁺, 100). Pureté (HPLC/UV 260 nm) : 100 %.

| | | | | |
|---|---|---|---|---|
| **9bj** | 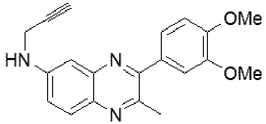 | C₂₀H₁₉N₃O₂ | 333,4 g.mol⁻¹ | Solide jaune |
| | 3-(3,4-dimethoxyphenyl)-2-methyl-*N*-(prop-2-ynyl)quinoxalin-6-amine | | | |

Méthode d'obtention similaire à celle utilisée pour la synthèse du composé **9bc.**
Rendement : 47 % (29,6 mg). MS (ESI) m/z : 334.2 ([M+H]⁺, 100). Pureté (HPLC/UV 260 nm) : 84 %.

| | | | | |
|---|---|---|---|---|
| **9bk** | 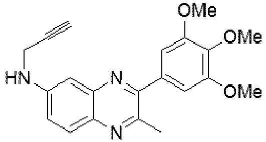 | C₂₁H₂₁N₃O₃ | 363,4 g.mol⁻¹ | Solide jaune |
| | 2-methyl-*N*-(prop-2-ynyl)-3-(3,4,5-trimethoxyphenyl)quinoxalin-6-amine | | | |

Méthode d'obtention similaire à celle utilisée pour la synthèse du composé **9bc.**
Rendement : 42 % (118,7 mg). MS (ESI) m/z : 364.2 ([M+H]⁺, 60) et 749.3 [2M+Na]⁺, 100). Pureté (HPLC/UV 260 nm) : 92 %.

| | | | | |
|---|---|---|---|---|
| **9bl** | 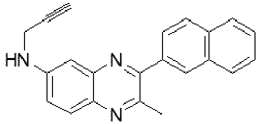 | C₂₁H₁₇N₃ | 323,4 g.mol⁻¹ | Solide jaune |
| 2-methyl-3-(naphthalen-2-yl)-*N*-(prop-2-ynyl)quinoxalin-6-amine | | | | |

Méthode d'obtention similaire à celle utilisée pour la synthèse du composé **9bc.**
Rendement : 28 % (80,5 mg). MS (ESI) m/z : 324.2 ([M+H]⁺, 100). Pureté (HPLC/UV 220 nm) : 90 %.

| | | | | |
|---|---|---|---|---|
| **9bm** | 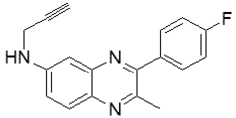 | C₁₈H₁₄FN₃ | 291,3 g.mol⁻¹ | Solide jaune |
| 3-(4-fluorophenyl)-2-methyl-*N*-(prop-2-ynyl)quinoxalin-6-amine | | | | |

Méthode d'obtention similaire à celle utilisée pour la synthèse du composé **9bc.**
Rendement : 28 % (74 mg). RMN ¹H (300 MHz, CDCl₃) δ ppm : 2.26 (t, *J* = 2,4 Hz, 1H), 2.69 (s, 3H), 4.06 (brs, 2H), 4,38 (brs, 1H, NH), 7.12-7.15 (m, 2H), 7.20 (t, *J* = 8,8 Hz, 2H), 7.61-7.65 (m, 2H), 7.84 (d, *J* = 8,8 Hz, 1H). RMN ¹³C (100 MHz, CDCl₃) δ ppm: 23.8, 33.5, 71.9, 79.9, 105.3, 115.4, 115.6, 121.8, 129.1, 130.8, 130.9, 136.5, 139.4, 142.9, 147.4, 147.9, 153.7, 164.3. MS (ESI) m/z : 292.2 ([M+H]⁺, 100). Pureté (HPLC/UV 260 nm) : 94 %.

| | | | | |
|---|---|---|---|---|
| **9bn** | 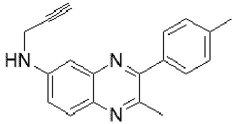 | C₁₉H₁₇N₃ | 287,4 g.mol⁻¹ | Solide jaune |
| | 2-methyl-*N*-(prop-2-ynyl)-3-*p*-tolylquinoxalin-6-amine | | | |

Méthode d'obtention similaire à celle utilisée pour la synthèse du composé **9bc.**
Rendement : 40 % (207,0 mg). MS (ESI) m/z : 288.2 ([M+H]⁺, 100).

| | | | | |
|---|---|---|---|---|
| **9bo** | 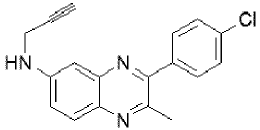 | C₁₈H₁₄ClN₃ | 307,8 g.mol⁻¹ | Solide jaune |
| | 3-(4-chlorophenyl)-2-methyl-*N*-(prop-2-ynyl)quinoxalin-6-amine | | | |

Méthode d'obtention similaire à celle utilisée pour la synthèse du composé **9bc.**
Rendement : 34 % (160 mg). MS (ESI) m/z : 308.2 ([M+H]⁺, 25) et 310.1 ([M+H]⁺, 7,5). Pureté (HPLC/UV 274 nm) : 95 %.

| | | | | |
|---|---|---|---|---|
| **9bp** | 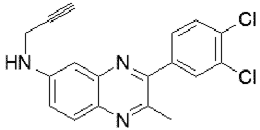 | C₁₈H₁₃Cl₂N₃ | 342,2 g.mol⁻¹ | Solide jaune |
| | 3-(3,4-dichlorophenyl)-2-methyl-*N*-(prop-2-ynyl)quinoxalin-6-amine | | | |

Méthode d'obtention similaire à celle utilisée pour la synthèse du composé **9bc.**
Rendement : 50 % (196,0 mg). MS (ESI) m/z : 242.1 ([M+H]⁺, 100) et 344.1 ([M+H]⁺, 50). Pureté (HPLC/UV 264 nm) : 94 %.

| | | | | |
|---|---|---|---|---|
| **9ba** | 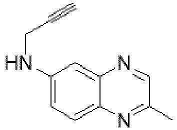 | C₁₂H₁₁N₃ | 197,2 g.mol⁻¹ | Solide jaune |
| | 2-methyl-*N*-(prop-2-ynyl)quinoxalin-6-amine | | | |

Méthode d'obtention similaire à celle utilisée pour la synthèse du composé **9bc.**
Rendement : 41 % (2,570 g). MS (ESI) m/z : 198.2 ([M+H]⁺, 100). Pureté (HPLC/UV 220 nm) : 94 %.

### II. Evaluation biologique

Les amino-quinoxalines ont ensuite été évaluées pour leurs propriétés neuroprotectrices et neurodifférentiatrices (ou neuritogéniques).

### II.1 Effet des composés sur la neuroprotection et la différenciation des neurones dopaminergiques en culture primaire dans un modèle de dégénérescence spontanée.

### II.1.1 Relation structure-activité et détermination de la concentration efficace (EC50)

- Les dérivés quinoxalines ont été testés sur un modèle de dégénérescence spontanée des neurones dopaminergiques en culture selon le protocole décrit dans l'article de F. Schmidt et al. PLoS ONE 2009, 4(7) e2615 (« Chemicals Possessing a Neurotrophin-Like Activity on Dopaminergic Neurons in Primary Culture »). Ce modèle consiste en la mise en culture de mésencéphale ventral d'embryon de rat. Cette partie du cerveau en culture contient une proportion en neurones dopaminergiques de 5 % des neurones totaux, les autres neurones étant essentiellement GABAergiques. Ces cultures sont également composées de cellules gliales à savoir les astrocytes, oligodendrocytes et la microglie. Ces cultures sont caractérisées par la mort progressive des neurones. L'effet neuroprotecteur des composés selon l'invention a été évalué par comptages des neurones dopaminergiques (TH⁺) marqués par immunohistochimie de la tyrosine hydroxylase (TH) après 8 jours de culture. Ainsi, par exemple les composés **4aa, 4ba, 4ad, 4bd, 4bc** et **4ed** ont été évalués à 1nM, 10nM, 100nM et 1µM et comparés à l'activité de la dibutyryl-AMP cyclique (dbc-AMP) à 200 µM.

**Tableau 1. Activité de certains dérivés d'aminoquinoxaline à 100nM sur la survie des neurones dopaminergiques foetaux en culture.**

| | | | | |
|---|---|---|---|---|
| 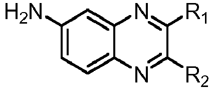 | | | | |

| Composé (100nM) | R₁ | R₂ | Neurones TH⁺ en % du contrôle +/- SEM^{a} | Diff en % du contrôle +/- SEM |
|---|---|---|---|---|
| contrôle | | | 100 +/- 1,9% | 100 +/- 5,6% |
| dbcAMP | | | 149,9 +/- 3,6 % | 333 +/- 8,7% |
| **4ad** | *n*-Bu | H | 101,0 +/- 4,1 % | 118,4 +/- 9,0% |
| **4bd** | *n*-Bu | CH₃ | 120,6 +/- 5,4 % | 102,5 +/- 4,6% |
| **4bc** | Ph | CH₃ | 140,4 +/- 4,1 % | 142,1 +/- 3,3% |
| **4ed** | *n*-Bu | *n*-Hex | 114,6 +/- 5,9 % | 104,3 +/- 9,5% |

| | | | | |
|---|---|---|---|---|
| ^{a} Erreur Standard à la Moyenne. Les données sont exprimées en pourcentage de la valeur du contrôle négatif (moyenne ± SEM). | | | | |

Les résultats montrent une activité particulièrement importante du composé **4bc** avec un pourcentage de survie quasiment égal à celui induit par la dbc-AMP. On observe en outre une activité particulièrement intéressante des composés substitués en position 2 du cycle quinoxaline par un motif aromatique.
- Les dérivés quinoxalines ont également été testés sur un modèle de dégénérescence spontanée des neurones dopaminergiques en culture selon un protocole légérement différent de celui décrit précédement. Ce second protocole est décrit dans l'article de S. Mourlevat, P. P. Michel et al. Molecular Pharmacology 2003, 64:578-586 (« Prévention of Dopaminergic Neuronal Death by Cyclic AMP in Mixed Neuronal/Glial Mesencephalic Cultures Requires the Repression of Presumptive Astrocytes»). L'effet neuroprotecteur des composés selon l'invention a été évalué par comptage des neurones dopaminergiques (TH⁺) marqués par immunohistochimie de la tyrosine hydroxylase (TH) après 10 jours de culture. Les composés selon l'invention ont été évalués à 50 µM, sauf le composé **4bc** qui a été évalué à 100 µM. Ils ont été comparés à l'activité de la dibutyryl-AMP cyclique (dbc-AMP) à 1 mM.

**Tableau 2. Activité de certains dérivés d'aminoquinoxaline à 50 ou 100 µM sur la survie des neurones dopaminergiques foetaux en culture.**

| | | | | | |
|---|---|---|---|---|---|
| 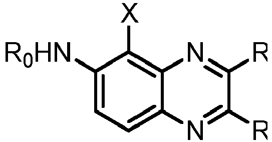 | | | | | |

| Composé (50 µM) | R₁ | R₂ | R₀ | X | Neurones TH⁺ en % du contrôle +/- Std. Err.^{a} |
|---|---|---|---|---|---|
| contrôle | | | | | 100 ± 4 % |
| dbAMPc | Ph 4-OMe-Ph | | | | 241 ± 9 % |
| **4bc*** | | CH₃ | H | H | 139 ± 11 % |
| **4ch** | | Ph | H | H | 130 ± 10 % |
| **4ac** | Ph | H | H | H | 189 ± 11 % |
| **9bc** | Ph | CH₃ | CH₂C≡CH | H | 174 ± 15 % |
| **9bm** | 4-F-Ph | CH₃ | CH₂C≡CH | H | 166 ± 15 % |
| **7bc** | Ph | CH₃ | H | Br | 153 ± 12 % |
| **8bc** | Ph | CH₃ | H | Cl | 129 ± 8 % |

| | | | | | |
|---|---|---|---|---|---|
| ^{a} Erreur Standard. Les données sont exprimées en pourcentage de la valeur du contrôle négatif (moyenne d'une expérience réalisée en triplicate ± Std. Err.). * Effet à 100 µM. | | | | | |

### II.1.2 Effet neuritogénique des composés selon l'invention

Afin d'évaluer l'activité neuritogénique des composés selon l'invention, la pousse neuritique par cellule a été quantifiée à l'aide du logiciel Neurite Outgrowth développé par Explora Nova. 60 neurones par condition au minimum ont été photographiés et étudiés. Les résultats obtenus avec le composé **4bc** sont présentés dans la figure 1.

Les images de la figure 1 (prises au grossissement x20 et inversées à l'aide d'un logiciel de retraitement d'images) montrent des cultures soit non traitées (contrôle, B), soit traitées avec le composé **4bc** à 100 nM (A) ou avec la dbc-AMP à 200µM (C), dont les neurones dopaminergiques sont immunomarqués par reconnaissance de la tyrosine hydroxylase. On observe une augmentation significative par rapport au contrôle négatif de la différenciation des neurones TH⁺.

Ce composé **4bc** actif sur la survie des neurones dopaminergiques montre ainsi également une activité sur leur différenciation avec des neurones possédant des prolongements neuritiques (c'est-à-dire ensemble axones et dendrites) plus longs et plus nombreux.

### II.2. Etude du passage de la barrière hémato-encéphalique du composé 4bc

### II.2.1. Matériels et méthodes

### Animaux :

Des souris mâles RjOrl:SWISS, âgées approximativement de 5 semaines et pesant entre 20 et 24 g à leur arrivée (Centre d'élevage R Janvier, France) sont maintenues dans l'animalerie, à température constante (22 ± 1°C) et sous hygrométrie contrôlée (55 ± 20%), avec un cycle lumière/obscurité de 12 heures (8h00-20h00). Durant la période d'acclimatation et de l'étude, les souris ont libre accès à la nourriture et à la boisson.

Toutes les expérimentations se sont déroulées en accord avec les conditions du décret n°2001-464 du 29 mai 2001 relatif aux expériences pratiquées sur les animaux.

### Traitements :

Deux modes de traitement ont été utilisés : une administration par voie intra-péritonéale (IP) pendant une durée de 1 ou 2 jours ou une administration par voie orale ou *per os* d'une durée de 5 jours.

### • Traitement par voie intra-péritonéale (IP)

La solution de composé **4bc** à injecter à la concentration de 7,0 g/L est préparée dans le véhicule contenant 10 % Tween20 + 20 % de DMSO + 70 % de NaCl à 0,9 % dans l'eau.

Trois groupes d'animaux ont été constitués :
- des souris qui ont reçu le véhicule par voie IP, à 10 mL/kg (Groupe V, n=2),
- des souris traitées avec le composé **4bc** par voie IP à 70 mg/kg et à 10 mL/kg (Groupe T, n=6),
- une souris non-traitée qui sera utilisée comme contrôle positif lors de l'analyse.

Les souris traitées T2 et T3 ont reçu les traitements comme suit :

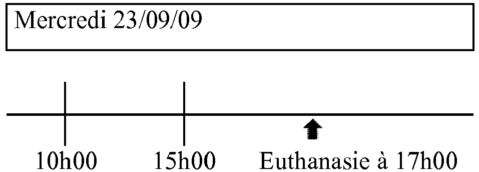

Les souris traitées T1, T4, T5 et T6 ainsi que les souris ayant reçu le véhicule V1 et V2 ont reçu les traitements / véhicules comme suit :

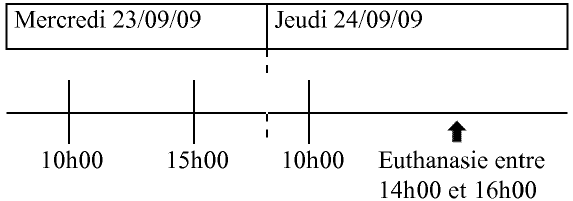

### • Traitement par voie orale (per os)

La solution de composé **4bc** à la concentration de 15,0 g/L à administrer est préparée dans le véhicule suivant : 0,5 % Tween80 + 99,5 % carboxy-methyl cellulose à 1 %, dans NaCl 0,9 % dans l'eau.

Trois groupes ont été constitués :
- des souris qui ont reçu le véhicule par voie orale, à 10 mL/kg (Groupe V, n=4),
- des souris traitées avec le composé **4bc** par voie orale à 150 mg/kg et à 10 mL/kg (Groupe T, n=6),
- des souris contrôles qui n'ont reçu aucun traitement (n=2).

Toutes les souris sont traitées ou reçoivent le véhicule à raison de 2 fois par jour pendant 4 jours. Le 5^{ème} jour un dernier traitement est administré et les animaux sont euthanasiées entre 4h00 et 6h40 après ce dernier traitement selon le schéma suivant :

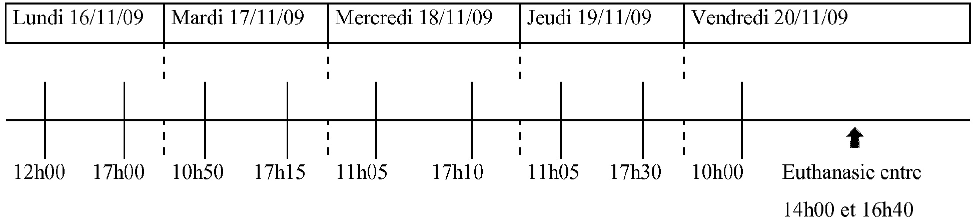

### Euthanasie et dissection :

Les souris sont anesthésiées par une injection de 0,1 mL d'une solution de pentobarbital sodique à 6 % puis les souris sont perfusées, de façon à éliminer le composé présent au niveau plasmatique, avec une solution de NaCl 0,9 % qui contient de l'héparine (200 µL d'héparine choay à 5000 UI/mL dans un litre de NaCl 0,9 %). Chaque animal est perfusé avec au minimum 50 mL de cette solution. La souris est ensuite décérébrée puis le cervelet, le tronc cérébral et les bulbes olfactifs sont éliminés par dissection.

Pour l'étude IP le cerveau est disséqué en 2 parties (hémisphère droit et hémisphère gauche).

Pour l'étude *per os* le cerveau est disséqué en 3 parties selon le schéma présenté sur la figure 2.

Les différentes parties du cerveau sont congelées dans l'isopentane à -30°C pendant une minute, puis conservées séparément à -80°C.

### Méthode d'extraction :

Chaque échantillon de cerveau de souris traitée par le composé **4bc** est pesé, placé dans le MeOH (100 µl de MeOH pour 10 mg de tissu cérébral) puis mixé par sonication. Les suspensions ainsi obtenues sont centrifugées à 14 000 g pendant 7 minutes, à 4°C pour la voie IP ou à 27 000 g pendant 10 minutes, à 4°C pour la voie orale. Les surnageants, dilués au demi dans le MeOH, sont transférés dans des vials HPLC pour l'analyse de la présence du composé **4bc** par HPLC-MS/MS.

Il est à noter que, afin de conserver des échantillons pour de futures analyses, seules les parties 1 des cerveaux de souris traitées *per os* ont été extraites et analysées. Les parties de cerveaux 1 et 2 des souris T2 et T4 ont néanmoins été extraites afin de vérifier que le composé **4bc** ne se distribue pas préférentiellement dans l'une de ces deux parties.

Tous les échantillons sont stockés à -4°C avant et pendant la durée des analyses.

Les extraits sont analysés dans les mêmes conditions, cependant les extraits IP et *per os* ont été analysés à des jours différents.

### Contrôles positifs et négatifs :

Le cerveau d'une souris ayant reçu seulement le véhicule, utilisée dans l'étude IP, est utilisé comme contrôle négatif. Le cerveau d'une souris n'ayant pas été traitée, utilisée dans l'étude IP, est utilisé comme contrôle positif.

Chaque échantillon est pesé, placé dans le MeOH (100 µl de MeOH pour 10 mg de tissu cérébral) et 10 ou 20 µL de la solution de composé **4bc** à 7 g/L sont ajoutés à l'hémisphère gauche ou à l'hémisphère droit de la souris contrôle. L'ensemble est vortexé puis mixé par sonication. Les suspensions ainsi obtenues sont centrifugées à 14 000 g pendant 7 minutes, à 4°C. Les surnageants, dilués au cinquième dans le MeOH, sont transférés dans des vials HPLC pour l'analyse du composé **4bc** par HPLC-MS/MS.

### Préparation de la gamme de composé 4bc :

Une solution mère de composé **4bc** est préparée extemporanément dans le MeOH à une concentration de 1,0 mg/mL. Toutes les autres solutions sont préparées par dilution de cette solution mère dans le MeOH puis stockées à - 4°C.

### Instrumentation et conditions pour l'analyse du composé 4bc :

La détection et la quantification, basée sur l'aire des pics, sont réalisées par HPLC-MS/MS, en mode MRM.

Le système HPLC est composé d'une pompe Dionex Ultimate 3000 équipée d'un injecteur automatique d'échantillon Dionex WPS-3000PL. Le spectromètre de masse utilisé est un Water-Micromass Quattro Ultima équipé d'une source d'ionisation à electrospray et d'un analyseur triple quadrupole. L'acquisition des données ainsi que les analyses sont effectuées *via* Masslynx 3.5.

La chromatographie liquide est réalisée en mode isocratique avec une colonne Nucleodur™ (Macherey-Nagel) C18, 125 × 2,1 mm, possédant une taille de particule de 5,0 µm. La phase mobile est composée d'un mélange de deux phases A et B, en proportions 30/70. La phase A est composée de 1 % d'acide acétique dans l'eau (v/v). La phase B est composée de 1 % d'acide acétique dans le MeOH (v/v). Le débit est fixé à 0,2 mL/min ; le volume d'injection est de 5 µL.

Le spectromètre de masse est relié au système HPLC en utilisant une source d'ionisation à electrospray. Une vanne de commutation est programmée avant injection de l'échantillon pour le rinçage et l'équilibration du capillaire, de façon à empêcher une éventuelle accumulation de sel au niveau du capillaire de la source. Le voltage du capillaire est réglé à 4000 V et le voltage du cône à 80 V ; la température de la source est réglée à 120°C ; le gaz de désolvatation (N₂) est réglé à un débit de 529 L/h et la température à 350°C. Les spectres de masses sont obtenus en mode positif. Les paramètres sont optimisés pour obtenir le maximum d'ion moléculaire ([M+H]⁺) à m/z 236,41, correspondant au composé **4bc.** La collision est effectuée par l'argon et l'énergie est fixé à 30eV en mode MS/MS pour détecter les transitions de masse : m/z 236,41→ 117,08 ; 236,41→ 131,10 et 236,41→ 158,27.

La droite d'étalonnage est réalisée à partir d'une gamme de standard comportant 9 concentrations (100 µg/mL ; 10 µg/mL ; 1 µg/mL ; 0,5 µg/mL; 0,1 µg/mL; 20 ng/mL ; 4 ng/mL ; 0,8 ng/mL et 0,16 ng/mL).

### II.2.2. Résultats

### Toxicité du composé 4bc :

Les animaux sont pesés chaque jour, tout au long de l'étude sub-chronique afin de contrôler leur état général. Le poids des animaux étant considéré comme un bon indice de santé générale, une toxicité du composé se caractériserait par une baisse notoire du poids corporel au cours du temps.

La figure 3 représente ainsi la moyenne des poids des souris d'un même groupe en fonction du temps durant le traitement *per os* (souris non traitées, ayant reçu le véhicule ou ayant reçu le composé **4bc** à raison de 300 mg/kg/jour).

### • Étude per os :

L'augmentation du poids des animaux est linéaire au cours de l'expérience reflètant un bon état général des animaux et une absence de toxicité importante du composé.

### • Étude IP :

Le suivi du poids des souris au cours de l'étude IP est sans valeur compte tenu de la courte durée de l'expérience (1 ou 2 jours).

### Validation de la méthode HPLC-MS/MS :

### • Voie IP :

L'aire des pics HPLC est fonction de la concentration de composés **4bc** dans la gamme de 20 ng/mL à 100,00 µg/mL avec un coéfficient de corrélation R² égal à 0,9999. La limite de détection est de 4 ng/mL avec un rapport S/B (signal sur bruit) de 3 : 1. Le temps de rétention du composé **4bc** est de 3,3 minutes ; la période d'acquisition est de 8 minutes.

### • Voie per os :

L'aire des pics HPLC est fonction de la concentration de composé **4bc** dans la gamme de 4 à 1000,00 ng/mL avec un un coéfficient de corrélation R² égal à 0,9999. La limite de détection est de 0,8 ng/mL avec un rapport S/B (signal/bruit) de 3 : 1. Le temps de rétention du composé **4bc** est de 3,7 minutes ; la période d'acquisition est de 8 minutes.

### Concentration du composé 4bc dans les extraits de cerveaux et dans le plasma :

### • Voie IP :

Le composé **4bc** n'est pas détectable dans l'extrait de cerveaux de souris ayant reçu le véhicule (contrôle négatif) et est quantifié à 0,18 µg/mL et 0, 42 µg/mL dans les contrôles positifs (souris contrôle + 10 ou 20 µL de composé **4bc** à 7 mg/mL). Le composé **4bc** est quantifié dans les extraits de cerveaux de souris traitées par IP et euthanasiées 2 h après le dernier traitement, à la concentration moyenne de 8,83 µg/mL ± 0,92 SEM (n=3).

Les chromatogrammes obtenus sont présentés sur la figure 4.

Il est à noter que les extraits ont été conservés à 4°C plusieurs jours correspondant à la durée totale des analyses. Afin d'éviter toute erreur liée à la dégradation du composé, le dernier échantillon d'une série analysée au cours d'une journée a été réanalysé au jour suivant et utilisé comme standard.

### • Voie IP, analyse ultérieure des extraits :

Le composé **4bc** est quantifiable dans les anciens extraits de cerveaux de souris traitées par voie IP et euthanasiées 2 h après le dernier traitement, à la concentration moyenne de 1,39 µg/mL ± 0,09 SEM (n=4). Ces extraits de cerveaux ont été conservés pendant 40 jours à 4°C. Le composé **4bc** est détectable dans les anciens extraits de cerveaux de souris traitées IP et euthanasiées entre 4 et 6h après le dernier traitement (n=8).

Les chromatogrammes obtenus sont présentés sur la figure 5 pour les extraits de cerveaux de souris euthanasiées 2 h après le dernier traitement et sur la figure 6 pour les extraits de cerveaux de souris euthanasiées entre 4 et 6h après le dernier traitement, les extraits ayant été conservés 40 jours à 4°C avant analyse dans les deux cas.

### • Voie per os :

Le composé **4bc** est indétectable dans tous les échantillons de cerveaux de souris traitées *per os* comme le montre les chromatogrammes obtenus présentés sur la figure 7. Toutefois, il semble que ce résultat résulte du fait que l'euthanasie des souris a eu lieu trop tardivement après le dernier traitement. Une nouvelle étude décrite ci-dessous a donc été menée de manière à examiner l'influence du temps avant euthanasie sur la concentration du composé **4bc** dans les extraits de cerveaux.

### • Etude de l'influence du temps avant euthanasie sur la concentration en composé 4bc :

Cette étude a été effectuée sur des souris C57BL\6J (au lieu de RjOrl:SWISS) traitées avec le composé **4bc** par voie IP ou *per os.*

### - Voie IP

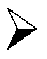 Les souris traitées par voie IP ont reçu un traitement de 70 mg/kg à 10 ml/kg d'une solution de composé **4bc** sur 2 jours comprenant 2 injections IP le 1er jour et 1 injection IP le jour suivant. Les résultats obtenus sont présentés dans le tableau suivant.

| **Temps de l'euthanasie après le dernier traitement** | **Concentration des extraits de cerveaux** | **Concentration plasmatique** |
|---|---|---|
| environ 1 h | 1 µg/ml | 1,2 µg/ml |
| environ 1 h 30 | 0,74 µg/ml | 3,1 µg/ml |
| environ 1 h 55 | 0,1 µg/ml | 1,7 µg/ml |
| environ 2 h 20 | 0,09 µg/ml | non mesuré |

La cinétique du composé **4bc** après administration par voie IP dans le cerveau et le plasma de souris est présenté en outre sur la figure 8.
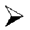 Une autre étude a été effectuée sur des rats traités par voie IP. Ces rats ont reçu un traitement de 20 ou 45 mg/kg à 10 ml/kg d'une solution de composé **4bc** sur 2 jours comprenant 2 injections IP le 1er jour et 1 injection IP le jour suivant. Les résultats obtenus sont présentés dans le tableau suivant.

| **Traitement** | **Temps de l'euthanasie après le dernier traitement** | **Concentration des extraits de cerveaux** | **Concentration plasmatique** |
|---|---|---|---|
| 45 mg/kg à 10 ml/kg de composé **4bc** | environ 1h | 5,1 µg/ml | non mesuré |
| 20 mg/kg à 10 ml/kg de composé **4bc** | environ 3h | 0,1 µg/ml | 0,4 µg/ml |

### - Voie per os

Les souris traitées par voie *per os* ont reçu un traitement de 150 mg/kg à 10 ml/kg d'une solution de composé **4bc** sur 5 jours comprenant 2 administrations par jour pendant 4 jours et 1 administration le dernier jour. Les résultats obtenus sont présentés dans le tableau suivant.

| **Temps de l'euthanasie après le dernier traitement** | **Concentration des extraits de cerveaux** | **Concentration plasmatique** |
|---|---|---|
| environ 1 h | 2,2 µg/ml | 11,6 µg/ml |
| environ 1 h 30 | 0,2 µg/ml | 0,7 µg/ml |
| environ 2 h | 0,1 µg/ml | 0,04 µg/ml |
| environ 2 h 25 | 0,05 µg/ml | 0,4 µg/ml |

### - Conclusion

Le composé **4bc** est donc bien détectable et quantifiable dans tous les échantillons (cerveau et plasma) que ce soit par voie IP ou *per os.*

### II.2.3. Discussion et conclusion

Le composé **4bc** est capable de traverser *in vivo* la barrière hémato-encéphalique après administration par voie intrapéritonéale. Les extraits de cerveaux d'animaux traités par voie IP et euthanasiés entre 4 et 6 h sont 2000 fois moins concentrés en composé **4bc** que les extraits de cerveaux d'animaux traités par voie IP et euthanasiés après 2 h post-traitement. Les extraits analysés ultérieurement, de cerveaux d'animaux traités par voie IP et euthanasiés à 2 h, possèdent une valeur moyenne environ 6 fois moins élevée que celle précédemment calculée (rapport des moyennes des concentrations des mêmes extraits analysés à 10 semaines d'intervalle). Cette variation étant négligeable comparée au rapport de 2000 observé entre des extraits de cerveaux d'animaux traités par voie IP et euthanasiés à des temps différents, on peut en conclure que le composé **4bc** est rapidement éliminé du cerveau au cours du temps.

Le composé **4bc** est indétectable dans les cerveaux d'animaux traités par administration orale et euthanasiés au minimum 6 heures après le dernier traitement. Cependant, compte tenu de la clairance rapide du composé **4bc** après traitement par voie IP, ce résultat est probablement du à un délai d'attente trop long entre le dernier traitement et l'euthanasie. Une deuxième étude par voie orale a permis de confirmer cette hypothèse. En effet lorsque l'euthanasie est effectuée 1 h après la dernière administration, le composé est detecté en quantités comparables que l'administration soit IP ou *per os* (1 µg/ml vs 2,2 µg/ml).

Le composé **4bc** est capable de traverser *in vivo* la barrière hémato-encéphalique de souris ou de rats après administration par voie orale et sans effets indésirables à une dose de
300 mg/kg/jour sur un traitement de 5 jours.

### II.3. Étude de l'efficacité du composé 4bc sur un modèle animal de la maladie de Parkinson

Le but de cette étude est de tester par une étude *in vivo* l'activité neuroprotectrice du composé **4bc** administré par voie orale à une souris après intoxication de celle-ci au MPTP par voie intra-nasale. La prise alimentaire et la prise de poids des animaux seront également contrôlés par rapport à des groupes témoins. L'analyse densitomètrique des fibres striatales et le comptage des corps cellulaires dopaminergiques de substance noire, permettront après marquage immunohistochimmique de la tyrosine hydroxylase de quantifier l'effet neuroprotecteur de la molécule.

### II.3.1. Matériels et méthodes

### Traitement :

30 souris mâles C57BL\6J âgées de 3 mois (fournisseur : Elevage janvier) ont été utilisées au cours de cette étude.

Le composé **4bc** a été administré par voie orale à la dose de 150 mg/kg à 10 ml/kg, deux fois par jour pendant deux jours, puis à 75 mg/kg, une fois par jour. Le véhicule est composé de 0,5 % de Tween 80 + 99,5 % de carboxyméthylcellulose (à 1 % dans l'eau). Une sonde rigide courbe couplée à des seringues de 1 ml Terumo ont été utilisées pour l'administration.

Le MPTP, toxine spécifique des neurones dopaminergiques, a été administré par voie intra-nasale. 1 mg (10 µL) de MPTP/HCl (sigma) dissous dans une solution de NaCl à 0,9 % est délivré par narine avec un intervalle de 3 minutes entre les deux narines. Les deux narines sont soumises à injection à raison d'une fois par jour pendant 4 jours.

L'étude a ainsi été réalisée selon le protocole suivant :
- Co-traitement au MPTP et avec le composé **4bc :** J1 à J4
- Post-traitement avec le composé **4bc** (1 fois par jour pendant 6 jours) : J5 à J10
- Euthanasie : J11

Quatre groupes de souris ont été constitués :
(**T** = traitée avec le composé **4bc,** NT = non traitée, **L** = lésée au MPTP, **NL** = non lésée au MPTP),
   - des souris ayant reçu le véhicule par voie orale, à 10mL/kg (Groupe **NT/NL,** n=6),
   - des souris traitées avec le composé **4bc** par voie orale à 150 mg/kg et à 10 mL/kg 2 fois par jour pendant 2 jours puis à 75 mg/kg et à 10 mL/kg 1 fois par jour pendant 7 jours (Groupe **T/NL,** n=6),
   - des souris ayant reçu du MPTP par voie IN (intra-nasale) (Groupe **NT/L,** n=9),
   - des souris ayant reçu du MPTP par voie IN (intra-nasale) et qui ont été traitées avec le composé **4bc** par voie orale à 150 mg/kg et à 10 mL/kg 2 fois par jour pendant 2 jours puis à 75 mg/kg et à 10 mL/kg 1 fois par jour pendant 7 jours (Groupe **NT/L,** n=9).

### Immunohistochimie :

Le 11^{ème} jour, les animaux sont anesthésiés, perfusés (NaCl 0,9 % puis paraformaldéhyde 4 %) puis euthanasiés. Les cerveaux sont ensuite extraits et post-fixés pendant 2 h dans une solution de formaldéhyde à 4 % puis cryoprotégés dans le sucrose à 30 % pendant 1 jour. Enfin, ces cerveaux sont congelés et des coupes fines sont réalisées au microtome à congélation.

Pour quantifier le pouvoir neuroprotecteur du composé **4bc,** une étude immunohistochimique est réalisée sur coupes flottantes de cerveaux. Deux structures anatomiques du cerveau sont analysées : le striatum (coupes de 20 µm, 10 niveaux) et la *substantia nigra* (coupes de 20 µm, 7 niveaux).

La tyrosine hydroxylase (TH) a été choisie comme marqueur biologique pour évaluer la densité optique des fibres dopaminergiques dans le striatum et le nombre de neurones dopaminergiques dans la *substantia nigra* de chaque animal.

### II.3.2. Résultats

Les résultats obtenus sont présentés sur les figures 9 et 10.

Les abréviations suivantes ont été utilisées sur ces deux figures :
- ST.R : striatum droit,
- ST.L : striatum gauche,
- ** : différence significative (p<0,01),
- * : différence significative (p<0,05), et
- NS : non significatif (p≥0,05),
les valeurs statistiques étant obtenues à l'aide d'un test non paramétrique de comparaison des moyennes de 2 groupes différents (test de Mann-Whitney).Ces résultats montrent que le composé **4bc** possède de façon significative une activité neuroprotectrice des fibres dopaminergiques *in vivo.* La légère toxicité observée chez les animaux ayant reçu uniquement le traitement par le composé **4bc** n'est pas significative.

## Revendications

1. Composé de formule (I) suivante : 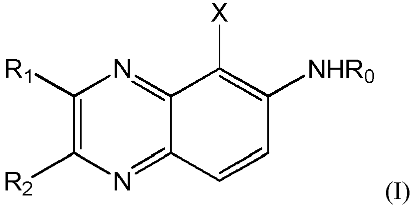 ainsi que ses sels pharmaceutiquement acceptables, ses stéréoisomères ou mélanges de stéréoisomères en toutes proportions, en particulier un mélange d'énantiomères, et notamment un mélange racémique,
dans laquelle :
- X représente un atome d'hydrogène, un atome d'halogène tel qu'un brome ou un chlore, un groupe NO₂ ou NH₂,
- R₀ représente H ou -CH₂-C≡CH, et
- R₁ et R₂ représentent chacun, indépendamment l'un de l'autre, un atome d'hydrogène ; une chaîne hydrocarbonée saturée ou insaturée, linéaire ou ramifiée, comportant de 1 à 10, de préférence 1 à 6 atomes de carbone ; ou un groupe aryle éventuellement substitué,
pour son utilisation en tant que médicament destiné au traitement ou à la prévention de la maladie de Parkinson.

2. Composé selon la revendication 1, **caractérisé en ce que** X représente un atome d'hydrogène, un atome de brome, un groupe NH₂ ou NO₂.

3. Composé selon la revendication 2, **caractérisé en ce que** X représente un atome d'hydrogène.

4. Composé selon la revendication 1, **caractérisé en ce que** R₀ représente un atome d'hydrogène.

5. Composé selon la revendication 1, **caractérisé en ce que** R₁ et R₂ représentent chacun, indépendamment l'un de l'autre, un atome d'hydrogène ; un groupe (C₁-C₆)alkyle ; ou un groupe aryle éventuellement substitué.

6. Composé selon la revendication 5, **caractérisé en ce que** R₁ et R₂ représentent chacun, indépendamment l'un de l'autre, un atome d'hydrogène ; un groupe (C₁-C₆)alkyle ; ou un groupe aryle éventuellement substitué par un ou plusieurs groupes choisis parmi un atome d'halogène, un groupe (C₁-C₆)alkyle, (C₁-C₆)alcoxy, aryle, N₃, NO₂, NH₂, et -NH-((C₁-C₆)alkyle).

7. Composé selon la revendication 6, **caractérisé en ce que** R₁ et R₂ représentent chacun, indépendamment l'un de l'autre, un atome d'hydrogène ; un groupe (C₁-C₆)alkyle ; ou un groupe aryle éventuellement substitué par un ou plusieurs groupes choisis parmi un atome d'halogène, un groupe (C₁-C₆)alkyle, (C₁-C₆)alcoxy, et aryle.

8. Composé selon la revendication 1, **caractérisé en ce que** R₁ et R₂ représentent chacun, indépendamment l'un de l'autre, un atome d'hydrogène ; un groupe (C₁-C₆)alkyle ; ou un groupe phényle éventuellement substitué.

9. Composé selon la revendication 8, **caractérisé en ce que** R₁ et R₂ représentent chacun, indépendamment l'un de l'autre, un atome d'hydrogène ; un groupe (C₁-C₆)alkyle ; ou un groupe phényle éventuellement substitué par un ou plusieurs groupes choisis parmi un atome d'halogène, un groupe (C₁-C₆)alkyle, (C₁-C₆)alcoxy, aryle, N₃, NO₂, NH₂, et -NH-((C₁-C₆)alkyle).

10. Composé selon la revendication 9, **caractérisé en ce que** R₁ et R₂ représentent chacun, indépendamment l'un de l'autre, un atome d'hydrogène ; un groupe (C₁-C₆)alkyle ; ou un groupe phényle éventuellement substitué par un ou plusieurs groupes choisis parmi un atome d'halogène, un groupe (C₁-C₆)alkyle, (C₁-C₆)alcoxy, et aryle.

11. Composé selon la revendication 1, **caractérisé en ce que** R₁ représente un groupe aryle éventuellement substitué.

12. Composé selon la revendication 11, **caractérisé en ce que** R₁ représente un groupe aryle éventuellement substitué par un ou plusieurs groupes choisis parmi un atome d'halogène, un groupe (C₁-C₆)alkyle, (C₁-C₆)alcoxy, aryle, N₃, NO₂, NH₂, et-NH-((C₁-C₆)alkyle).

13. Composé selon la revendication 12, **caractérisé en ce que** R₁ représente un groupe aryle éventuellement substitué par un ou plusieurs groupes choisis parmi un atome d'halogène, un groupe (C₁-C₆)alkyle, (C₁-C₆)alcoxy, et aryle.

14. Composé selon l'une quelconque des revendications 11 à 13, **caractérisé en ce que** le groupe aryle est un groupe phényle.

15. Composé selon la revendication 1, **caractérisé en ce qu'**il est choisi parmi les composés suivants :
| | | | |
|---|---|---|---|
| **4ba** | 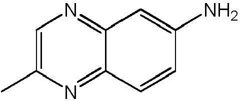 | **4bd** | 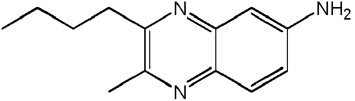 |
| **4be** | 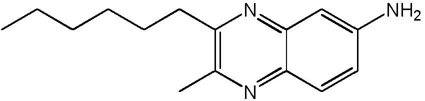 | **4bf** | 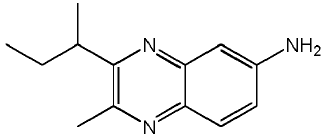 |
| **4bg** | 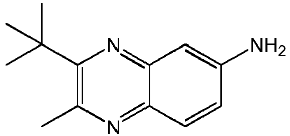 | **4bc** | 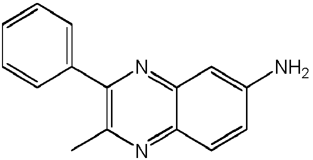 |
| **4bb** | 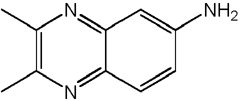 | **4aa** | 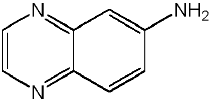 |
| **4ad** | 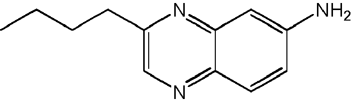 | **4dd** | 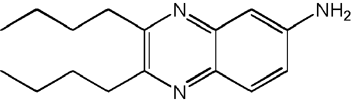 |
| **4ae** | 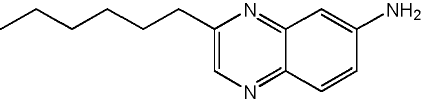 | **4af** | 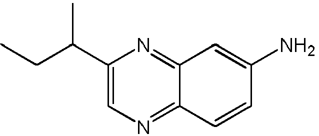 |
| **4ag** | 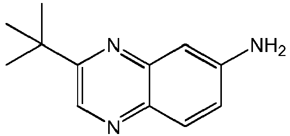 | **4ac** | 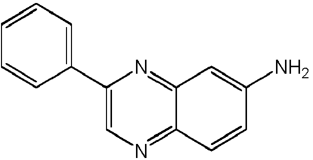 |
| **4ed** | 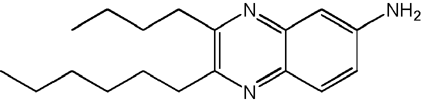 | **4de** | 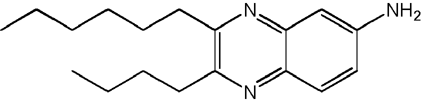 |
| **4cd** | 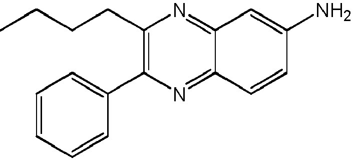 | **4bh** | 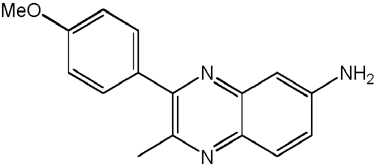 |
| **4bi** | 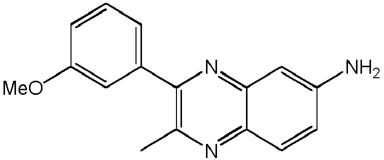 | **4bj** | 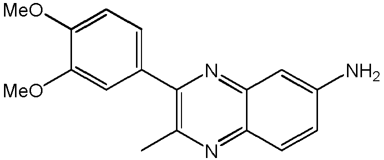 |
| **4bk** | 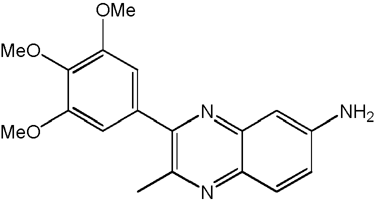 | **4bl** | 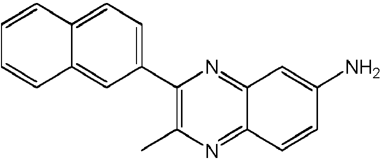 |
| **4bm** | 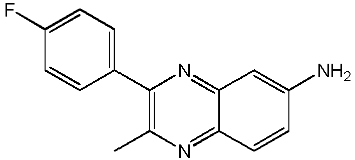 | **4bo** | 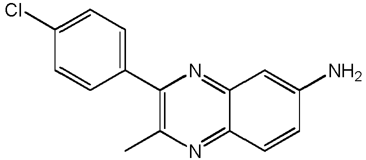 |
| **4bp** | 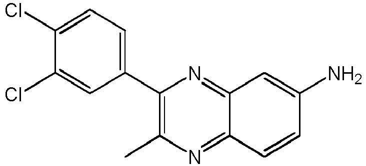 | **4bq** | 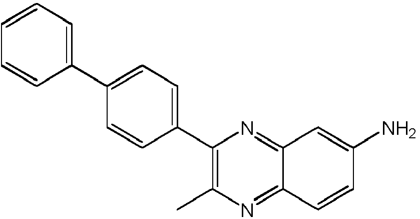 |
| **4bn** | 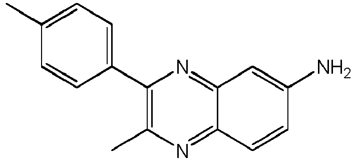 | **4ca** | 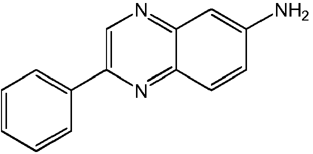 |
| **4ch** | 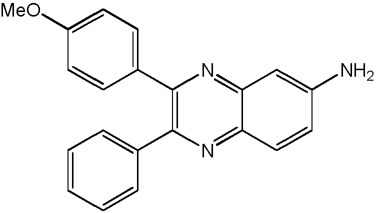 | **4cb** | 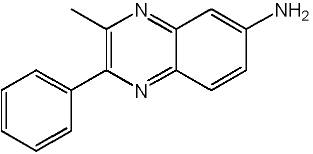 |
| **5aa** | 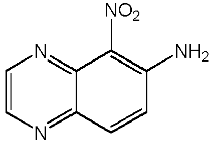 | **5bb** | 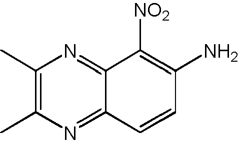 |
| **5ba** | 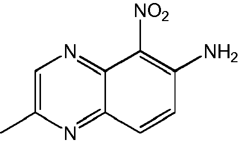 | **5bc** | 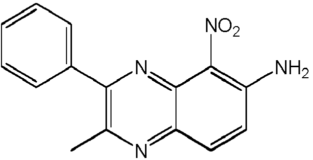 |
| **6aa** | 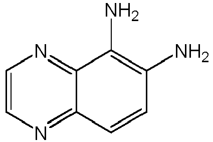 | **6bb** | 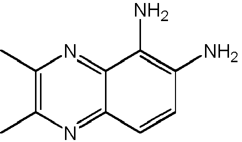 |
| **6ba** | 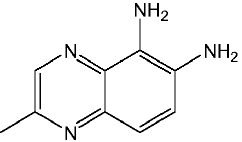 | **6bc** | 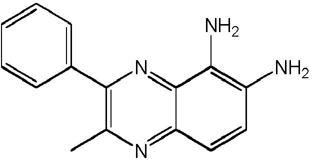 |
| **7aa** | 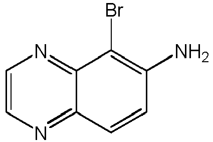 | **7bc** | 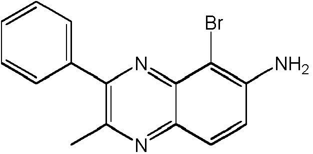 |
| **8bc** | 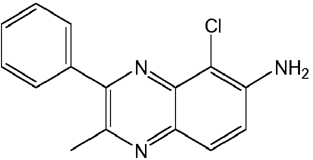 | **9bc** | 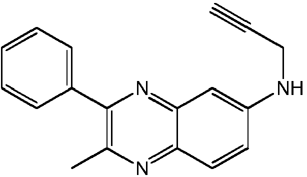 |
| **9bh** | 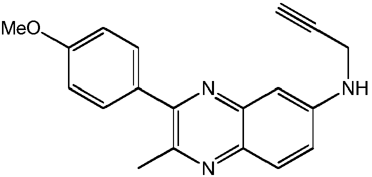 | **9bj** | 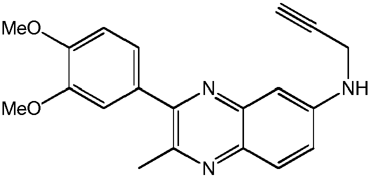 |
| **9bk** | 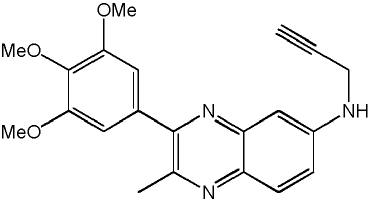 | **9bl** | 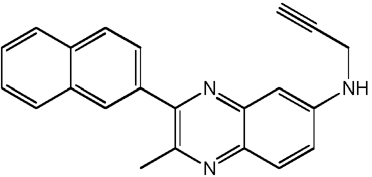 |
| **9bm** | 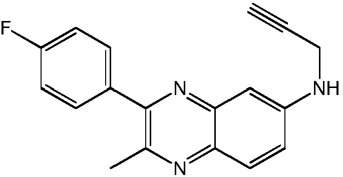 | **9bn** | 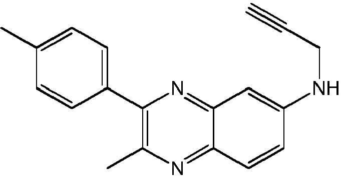 |
| **9bo** | 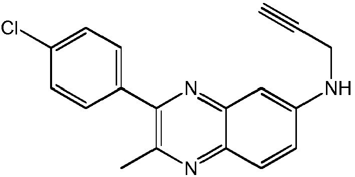 | **9bp** | 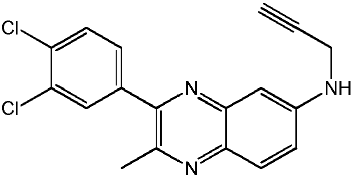 |
| **9ba** | 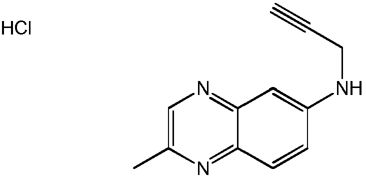 | | |

## Patentansprüche

1. Verbindung der folgenden Formel (I): 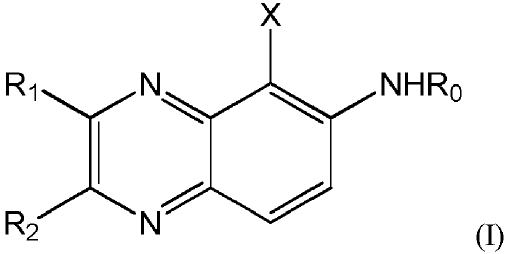 sowie ihre pharmazeutisch verträglichen Salze, ihre Stereoisomere oder Mischungen von Stereoisomeren in beliebigen Verhältnissen, insbesondere eine Mischung aus Enantiomeren und insbesondere eine racemische Mischung,
wobei :
- X ein Wasserstoffatom, ein Halogenatom, wie zum Beispiel Brom oder Chlor, eine Gruppe NO₂ oder NH₂ repräsentiert,
- R₀ H oder -CH₂-C≡CH repräsentiert, und
- R₁ und R₂ jeweils unabhängig voneinander ein Wasserstoffatom, eine gesättigte oder ungesättigte Kohlenwasserstoffkette, die einkettig oder verzweigt ist, bestehend aus 1 bis 10, vorzugsweise 1 bis 6 Kohlenstoffatomen, oder eine gegebenenfalls substituierte Arylgruppe repräsentieren,
zu ihrer Verwendung als Medikament, vorgesehen zur Behandlung oder zur Vorsorge gegen die Parkinsonkrankheit.

2. Verbindung gemäß Anspruch 1, **dadurch gekennzeichnet, dass** X ein Wasserstoffatom, ein Bromatom, eine Gruppe NH₂ oder NO₂ repräsentiert.

3. Verbindung gemäß Anspruch 2, **dadurch gekennzeichnet, dass** X ein Wasserstoffatom repräsentiert.

4. Verbindung gemäß Anspruch 1, **dadurch gekennzeichnet, dass** R₀ ein Wasserstoffatom repräsentiert.

5. Verbindung gemäß Anspruch 1, **dadurch gekennzeichnet, dass** R₁ und R₂ jeweils unabhängig voneinander ein Wasserstoffatom, eine (C₁-C₆)-Alkylgruppe, oder eine gegebenenfalls substituierte Arylgruppe repräsentieren.

6. Verbindung gemäß Anspruch 5, **dadurch gekennzeichnet, dass** R₁ und R₂ jeweils unabhängig voneinander ein Wasserstoffatom, eine (C₁-C₆)-Alkylgruppe, oder eine Arylgruppe, die gegebenenfalls durch eine oder mehrere Gruppen ausgewählt aus einem Halogenatom, einer Gruppe (C₁-C₆)-Alkyl, (C₁-C₆)-Alkoxy, Aryl, N₃, NO₂, NH₂ und -NH-((C₁-C₆)-Alkyl) substituiert ist, repräsentieren.

7. Verbindung gemäß Anspruch 6, **dadurch gekennzeichnet, dass** R₁ und R₂ jeweils unabhängig voneinander ein Wasserstoffatom, eine (C₁-C₆)-Alkylgruppe, oder eine Arylgruppe, die gegebenenfalls durch eine oder mehrere Gruppen ausgewählt aus einem Halogenatom, einer Gruppe (C₁-C₆)-Alkyl, (C₁-C₆)-Alkoxy, und Aryl substituiert ist, repräsentieren.

8. Verbindung gemäß Anspruch 1, **dadurch gekennzeichnet, dass** R₁ und R₂ jeweils unabhängig voneinander ein Wasserstoffatom, eine (C₁-C₆)-Alkylgruppe, oder eine gegebenenfalls substituierte Phenylgruppe repräsentieren.

9. Verbindung gemäß Anspruch 8, **dadurch gekennzeichnet, dass** R₁ und R₂ jeweils unabhängig voneinander ein Wasserstoffatom, eine (C₁-C₆)-Alkylgruppe, oder eine Phenylgruppe, die gegebenenfalls durch eine oder mehrere Gruppen ausgewählt aus einem Halogenatom, einer Gruppe (C₁-C₆)-Alkyl, (C₁-C₆)-Alkoxy, Aryl, N₃, NO₂, NH₂ und -NH-((C₁-C₆)-Alkyl) substituiert ist, repräsentieren.

10. Verbindung gemäß Anspruch 9, **dadurch gekennzeichnet, dass** R₁ und R₂ jeweils unabhängig voneinander ein Wasserstoffatom, eine (C₁-C₆)-Alkylgruppe, oder eine Phenylgruppe, die gegebenenfalls durch eine oder mehrere Gruppen ausgewählt aus einem Halogenatom, einer Gruppe (C₁-C₆)-Alkyl, (C₁-C₆)-Alkoxy und Aryl substituiert ist, repräsentieren.

11. Verbindung gemäß Anspruch 1, **dadurch gekennzeichnet, dass** R₁ eine gegebenenfalls substituierte Arylgruppe repräsentiert.

12. Verbindung gemäß Anspruch 11, **dadurch gekennzeichnet, dass** R₁ eine Arylgruppe, die gegebenenfalls durch eine oder mehrere Gruppen ausgewählt aus einem Halogenatom, einer Gruppe (C₁-C₆)-Alkyl, (C₁-C₆)-Alkoxy, Aryl, N₃, NO₂, NH₂ und -NH-((C₁-C₆)-Alkyl) substituiert ist, repräsentiert.

13. Verbindung gemäß Anspruch 12, **dadurch gekennzeichnet, dass** R₁ eine Arylgruppe, die gegebenenfalls durch eine oder mehrere Gruppen ausgewählt aus einem Halogenatom, einer Gruppe (C₁-C₆)-Alkyl, (C₁-C₆)-Alkoxy und Aryl substituiert ist, repräsentiert.

14. Verbindung gemäß einem der Ansprüche 11 bis 13, **dadurch gekennzeichnet, dass** die Arylgruppe eine Phenylgruppe ist.

15. Verbindung gemäß Anspruch 1, **dadurch gekennzeichnet, dass** sie aus den folgenden Verbindungen ausgewählt ist:
| | | | |
|---|---|---|---|
| **4ba** | 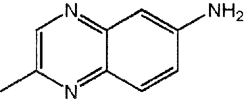 | **4bd** | 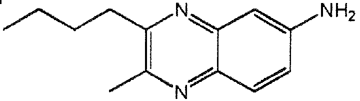 |
| **4be** | 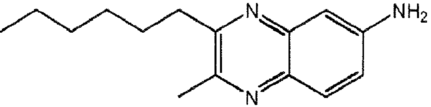 | **4bf** | 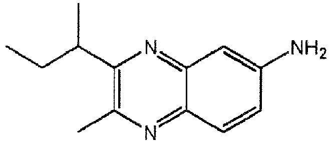 |
| **4bg** | 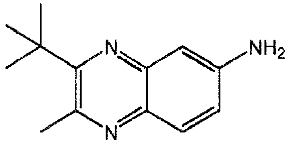 | **4bc** | 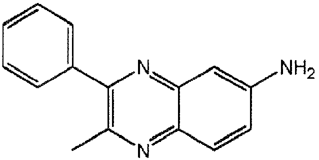 |
| **4bb** | 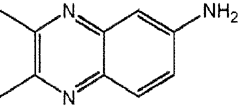 | **4aa** | 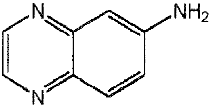 |
| **4ad** | 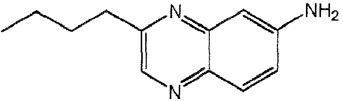 | **4dd** | 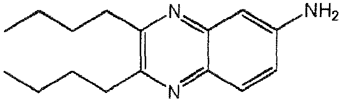 |
| **4ae** | 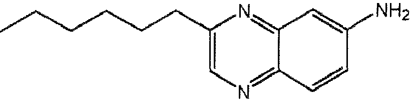 | **4af** | 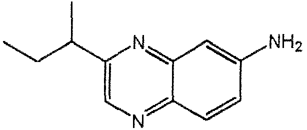 |
| **4ag** | 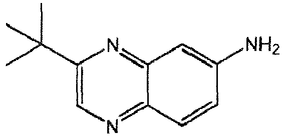 | **4ac** | 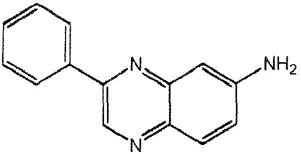 |
| **4ed** | 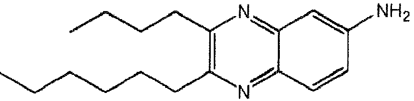 | **4de** | 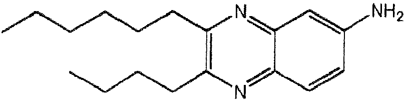 |
| **4cd** | 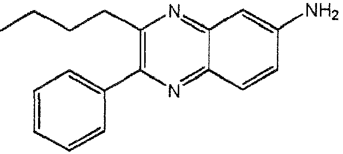 | **4bh** | 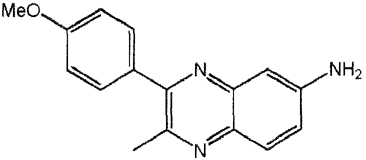 |
| **4bi** | 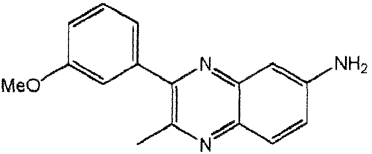 | **4bj** | 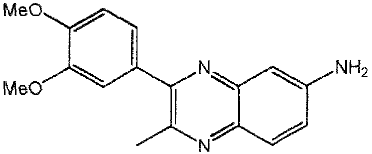 |
| **4bk** | 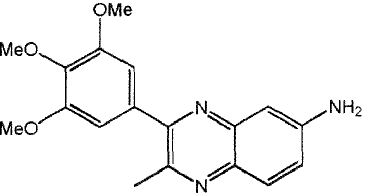 | **4bl** | 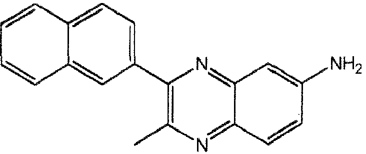 |
| **4bm** | 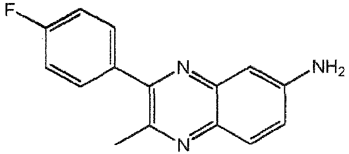 | **4bo** | 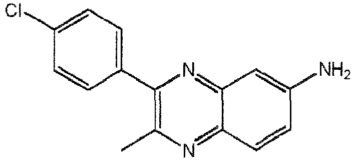 |
| **4bp** | 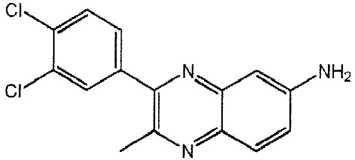 | **4bq** | 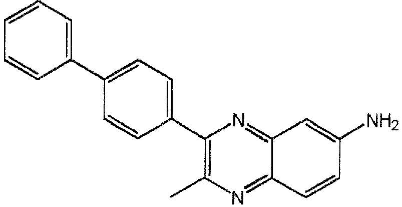 |
| **4bn** | 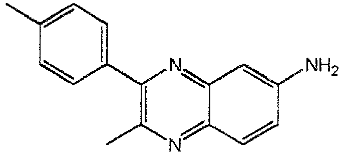 | **4ca** | 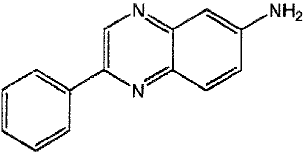 |
| **4ch** | 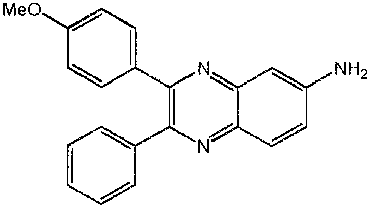 | **4cb** | 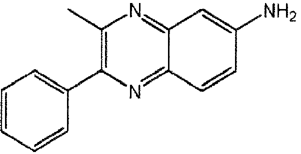 |
| **5aa** | 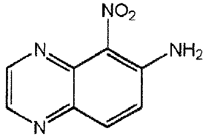 | **5bb** | 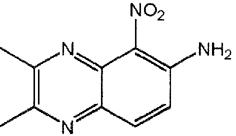 |
| **5ba** | 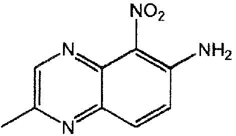 | **5bc** | 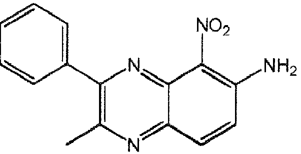 |
| **6aa** | 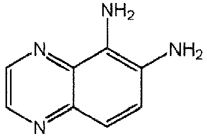 | **6bb** | 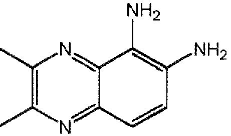 |
| **6ba** | 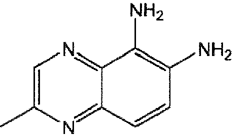 | **6bc** | 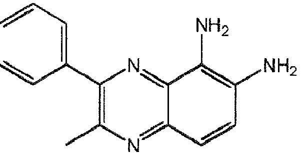 |
| **7aa** | 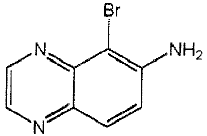 | **7bc** | 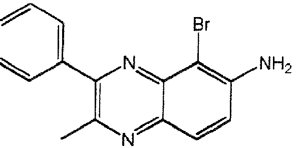 |
| **8bc** | 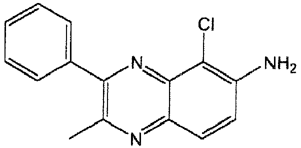 | **9bc** | 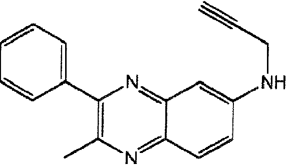 |
| **9bh** | 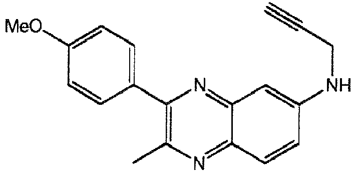 | **9bj** | 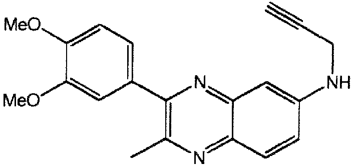 |
| **9bk** | 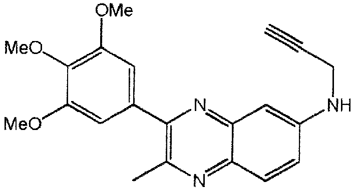 | **9bl** | 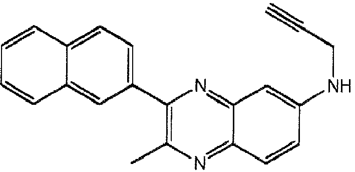 |
| **9bm** | 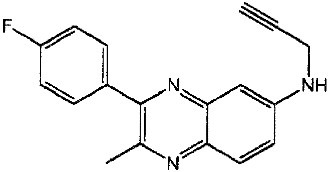 | **9bn** | 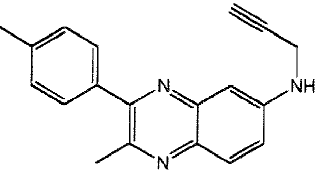 |
| **9bo** | 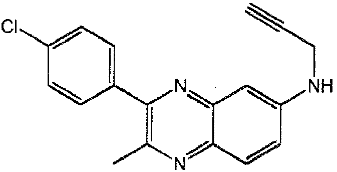 | **9bp** | 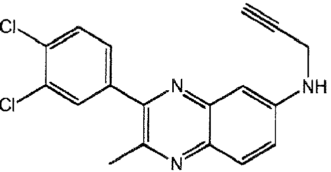 |
| **9ba** | 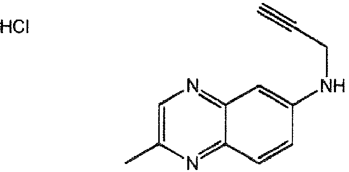 | | |

## Claims

1. Compound of following formula (I): 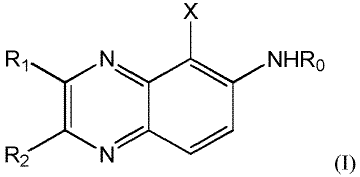 the pharmaceutically acceptable salts and the stereoisomers thereof, or mixtures of stereoisomers in any proportion, in particular a mixture of enantiomers, and particularly a racemic mixture,
where:
- X is a hydrogen atom, a halogen atom such as bromine or chlorine, an NO₂ or NH₂ group;
- R₀ is H or -CH₂-C≡CH; and
- R₁, and R₂ are each independently a hydrogen atom; a saturated or unsaturated, straight-chain or branched hydrocarbon chain having 1 to 10, preferably 1 to 6 carbon atoms; or an optionally substituted aryl group,
for use thereof as medicinal product, intended for the treatment or prevention of Parkinson's disease.

2. The compound according to claim 1, **characterized in that** X is a hydrogen atom, a bromine atom, an NH₂ or NO₂ group.

3. The compound according to claim 2, **characterized in that** X is a hydrogen atom.

4. The compound according to claim 1, **characterized in that** R₀ is a hydrogen atom.

5. The compound according to claim 1, **characterized in that** R₁ and R₂ are each independently a hydrogen atom; (C₁-C₆) alkyl group; or an optionally substituted aryl group.

6. The compound according to claim 5, **characterized in that** R₁ and R₂ are each independently a hydrogen atom; (C₁-C₆) alkyl group; or an aryl group optionally substituted by one or more groups selected from among a halogen atom, (C₁-C₆)alkyl, (C₁-C₆)alkoxy, aryl, N₃, NO₂, NH₂ and -NH-((C₁-C₆)alkyl) groups.

7. The compound according to claim 6, **characterized in that** R₁ and R₂ are each independently a hydrogen atom; (C₁-C₆) alkyl group; or an aryl group optionally substituted by one or more groups selected from among a halogen atom, (C₁-C₆)alkyl, (C₁-C₆)alkoxy and aryl groups.

8. The compound according to claim 1, **characterized in that** R₁ and R₂ are each independently a hydrogen atom; (C₁-C₆) alkyl group; or an optionally substituted phenyl group.

9. The compound according to claim 8, **characterized in that** R₁ and R₂ are each independently a hydrogen atom; (C₁-C₆) alkyl group; or a phenyl group optionally substituted by one or more groups selected from among a halogen atom, (C₁-C₆)alkyl, (C₁-C₆)alkoxy, aryl N₃, NO₂, NH₂ and -NH-((C₁-C₆)alkyl) groups.

10. The compound according to claim 9, **characterized in that** R₁ and R₂ are each independently a hydrogen atom; (C₁-C₆)alkyl group; or a phenyl group optionally substituted by one or more groups selected from among a halogen atom, (C₁-C₆)alkyl, (C₁-C₆)alkoxy and aryl groups.

11. The compound according to claim 1, **characterized in that** R₁ is an optionally substituted aryl group.

12. The compound according to claim 11, **characterized in that** R₁ is an aryl group, optionally substituted by one or more groups selected from among a halogen atom, (C₁-C₆)alkyl, (C₁-C₆)alkoxy, aryl, N₃, NO₂, NH₂ and -NH-((C₁-C₆)alkyl) groups.

13. The compound according to claim 12, **characterized in that** R₁ is an aryl group optionally substituted by one or more groups selected from among a halogen atom, (C₁-C₆)alkyl, (C₁-C₆)alkoxy and aryl groups.

14. The compound according to any of claims 11 to 13, **characterized in that** the aryl group is a phenyl group.

15. The compound according to claim 1, **characterized in that** it is selected from among the following compounds:
| | | | |
|---|---|---|---|
| **4ba** | 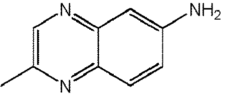 | **4bd** | 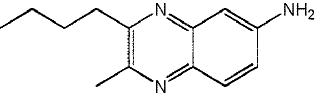 |
| **4be** | 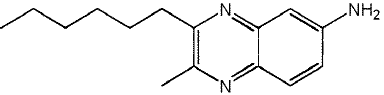 | **4bf** | 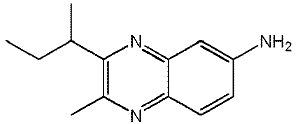 |
| **4bg** | 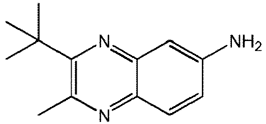 | **4bc** | 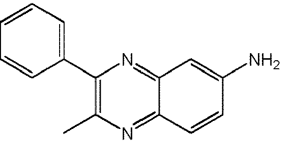 |
| **4bb** | 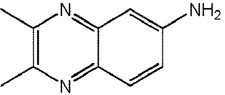 | **4aa** | 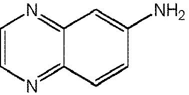 |
| **4ad** | 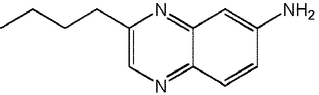 | **4dd** | 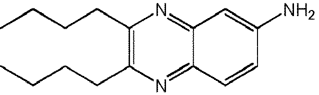 |
| **4ae** | 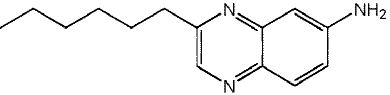 | **4af** | 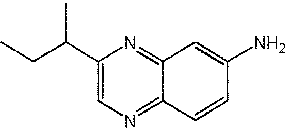 |
| **4ag** | 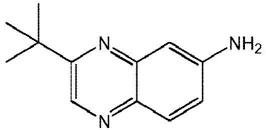 | **4ac** | 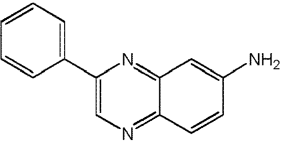 |
| **4ed** | 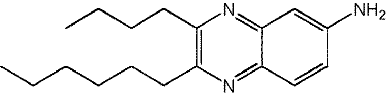 | **4de** | 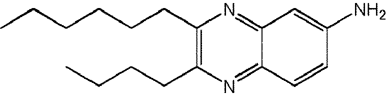 |
| **4cd** | 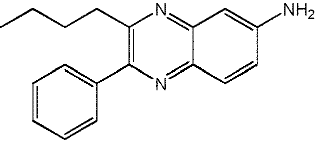 | **4bh** | 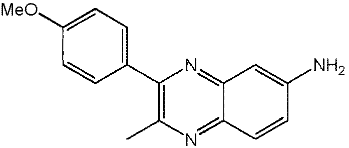 |
| **4bi** | 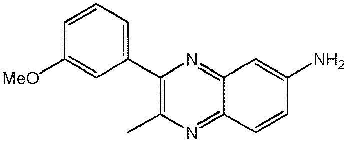 | **4bj** | 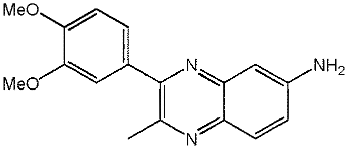 |
| **4bk** | 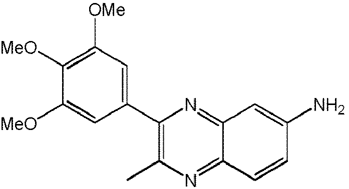 | **4bl** | 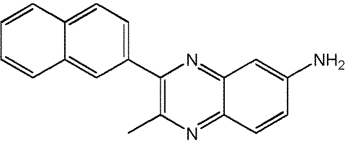 |
| **4bm** | 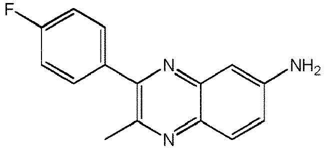 | **4bo** | 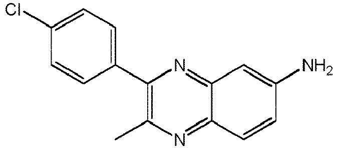 |
| **4bp** | 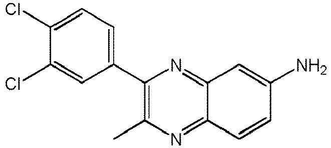 | **4bq** | 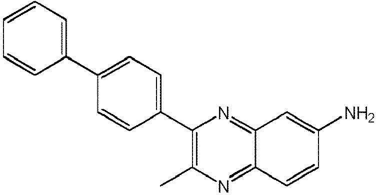 |
| **4bn** | 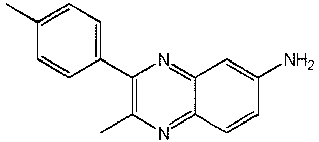 | **4ca** | 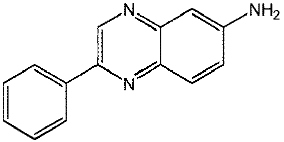 |
| **4ch** | 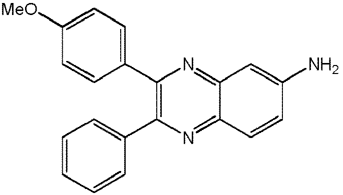 | **4cb** | 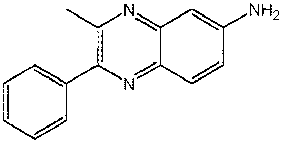 |
| **5aa** | 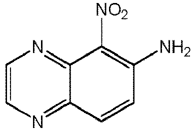 | **5bb** | 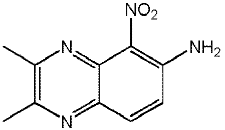 |
| **5ba** | 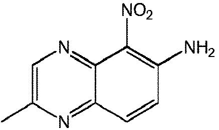 | **5bc** | 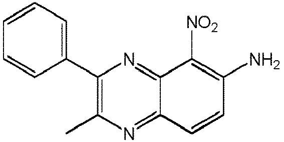 |
| **6aa** | 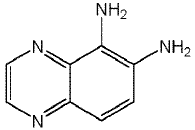 | **6bb** | 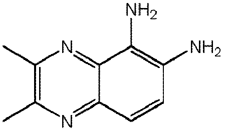 |
| **6ba** | 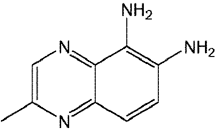 | **6bc** | 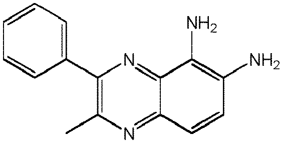 |
| **7aa** | 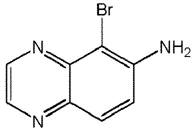 | **7bc** | 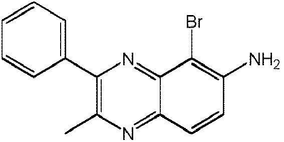 |
| **8bc** | 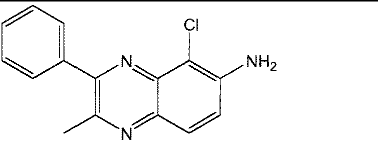 | **9bc** | 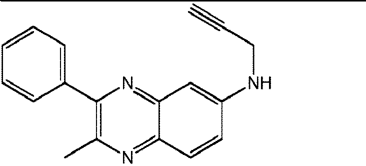 |
| **9bh** | 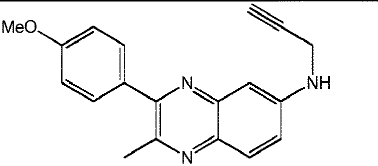 | **9bj** | 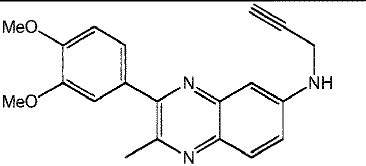 |
| **9bk** | 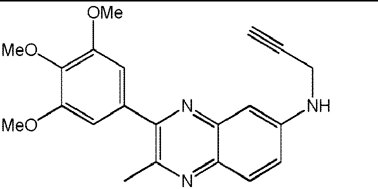 | **9bl** | 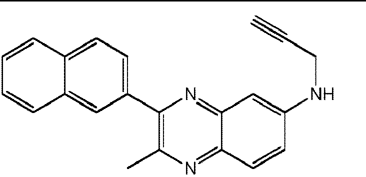 |
| **9bm** | 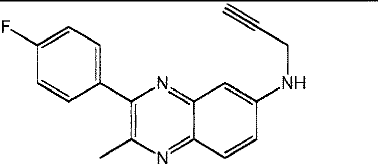 | **9bn** | 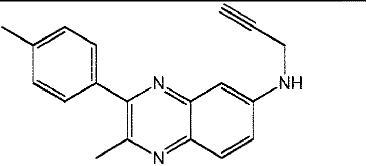 |
| **9bo** | 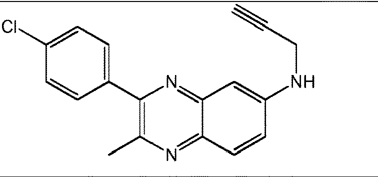 | **9bp** | 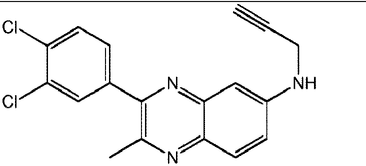 |
| **9ba** | 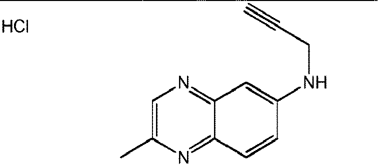 | | |
